# EUROPEAN PATENT SPECIFICATION

(11) **EP 2 812 030 B1**
(45) Date of publication and mention of the grant of the patent: **03.10.2018**
(21) Application number: 12868029.5
(22) Date of filing: 06.08.2012
(51) Int. Cl.: A61K 48/00, A61K 39/155, C12N 15/45, C07K 14/135, A61P 31/14

(54) **COMBINED ANTIGEN AND DNA VACCINE FOR PREVENTING AND TREATING RSV INFECTION**
KOMBINIERTER ANTIGEN- UND DNA-IMPFSTOFF ZUR VORBEUGUNG UND BEHANDLUNG VON RSV-INFEKTIONEN
VACCIN ASSOCIANT UN ANTIGÈNE ET UN ADN POUR LA PRÉVENTION ET LE TRAITEMENT D'UNE INFECTION PAR UN VRS

(30) Priority: 10.02.2012 CN 201210030325
(43) Date of publication of application: 17.12.2014
(73) Proprietor: Beijing Advaccine Biotechnology Co. Ltd., Beijing 100085 (CN)
(72) Inventor: WANG, Bin, Beijing 100085 (CN); CHEN, Xuan, Beijing 100193 (CN); YU, Qingling, Beijing 100085 (CN)
(74) Representative: Leonard, Thomas Charles
(86) International application number: PCT/CN2012/001046
(87) International publication number: WO 2013/116965

(56) References cited:
- WO-A1-2010/076618
- WO-A2-2009/105152
- WO-A2-2010/039224
- CN-A- 1 556 857
- CN-A- 102 307 591
- SOL KIM ET AL: "Baculovirus-based Vaccine Displaying Respiratory Syncytial Virus Glycoprotein Induces Protective Immunity against RSV Infection without Vaccine-Enhanced Disease", IMMUNE NETWORK, vol. 12, no. 1, 1 January 2012 (2012-01-01), page 8, XP055210505, ISSN: 1598-2629, DOI: 10.4110/in.2012.12.1.8
- KANG ET AL: "Co-inoculation of DNA and protein vaccines induces antigen-specific T cell suppression", BIOCHEMICAL AND BIOPHYSICAL RESEARCH COMMUNICATIONS, ACADEMIC PRESS INC. ORLANDO, FL, US, vol. 353, no. 4, 17 January 2007 (2007-01-17), pages 1034-1039, XP005771098, ISSN: 0006-291X, DOI: 10.1016/J.BBRC.2006.12.124
- HUALI JIN ET AL: "Protein/DNA vaccine-induced antigen-specific Treg confer protection against asthma", EUROPEAN JOURNAL OF IMMUNOLOGY, vol. 38, no. 9, 1 September 2008 (2008-09-01), pages 2451-2463, XP055013095, ISSN: 0014-2980, DOI: 10.1002/eji.200737899
- DENNIS M. LINDELL ET AL: "A Novel Inactivated Intranasal Respiratory Syncytial Virus Vaccine Promotes Viral Clearance without Th2 Associated Vaccine-Enhanced Disease", PLOS ONE, vol. 6, no. 7, 15 July 2011 (2011-07-15), page e21823, XP055038673, DOI: 10.1371/journal.pone.0021823
- XU, JUN ET AL.: 'Advances in respiratory syncytial virus vaccines.' LETTERS IN BIOTECHNOLOGY vol. 18, no. 2, 30 March 2007, pages 310 - 312, XP055166230
- CHENG, MING ET AL.: 'Antibody responses induced by mucosal DNA vaccine encoding the codon-optimized F protein of human respiratory syncytial virus (RSV) delivered with attenuated Salmonella typhimurium.' ACTA MICROBIOLOGICA SINICA vol. 51, no. 7, 04 July 2011, pages 965 - 971, XP008174637
- HANSSON, MARIANNE ET AL.: 'Design and production of recombinant subunit vaccines.' BIOTECHNOLOGY AND APPLIED BIOCHEMISTRY vol. 32, no. 2, 31 October 2000, pages 95 - 107, XP002276365

### SEQUENCE LISTING

<110> INOVIO PHARMACEUTICALS, INC.
<120> COMBINED ANTIGEN AND DNA VACCINE FOR PREVENTING AND TREATING RSV INFECTION
<130> 030276-9016-WO00
<140> New International Application
   <141> 2012-08-02
<160> 54
<170> PatentIn version 3.5
<210> 1
   <211> 574
   <212> PRT
   <213> Human respiratory syncytial virus
<400> 1
   Met Glu Leu Pro Ile Leu Lys Ala Asn Ala Ile Thr Thr Ile Leu Ala
   1               5                   10                  15
   Ala Val Thr Phe Cys Phe Ala Ser Ser Gln Asn Ile Thr Glu Glu Phe
               20                  25                  30
   Tyr Gln Ser Thr Cys Ser Ala Val Ser Lys Gly Tyr Leu Ser Ala Leu
           35                  40                  45
   Arg Thr Gly Trp Tyr Thr Ser Val Ile Thr Ile Glu Leu Ser Asn Ile
       50                  55                  60
   Lys Glu Asn Lys Cys Asn Gly Thr Asp Ala Lys Val Lys Leu Ile Asn
   65                  70                  75                  80
   Gln Glu Leu Asp Lys Tyr Lys Asn Ala Val Thr Glu Leu Gln Leu Leu
                   85                  90                  95
   Met Gln Ser Thr Thr Ala Ala Asn Asn Arg Ala Arg Arg Glu Leu Pro
               100                 105                 110
   Arg Phe Met Asn Tyr Thr Leu Asn Asn Thr Lys Lys Thr Asn Val Thr
           115                 120                 125
   Leu Ser Lys Lys Arg Lys Arg Arg Phe Leu Gly Phe Leu Leu Gly Val
       130                 135                 140
   Gly Ser Ala Ile Ala Ser Gly Ile Ala Val Ser Lys Val Leu His Leu
   145                 150                 155                 160
   Glu Gly Glu Val Asn Lys Ile Lys Ser Ala Leu Leu Ser Thr Asn Lys
                   165                 170                 175
   Ala Val Val Ser Leu Ser Asn Gly Val Ser Val Leu Thr Ser Lys Val
   180 185 190
   Leu Asp Leu Lys Asn Tyr Ile Asp Lys Gln Leu Leu Pro Ile Val Asn
   195 200 205
   Lys Gln Ser Cys Arg Ile Ser Asn Ile Glu Thr Val Ile Glu Phe Gln
   210 215 220
   Gln Lys Asn Asn Arg Leu Leu Glu Ile Thr Arg Glu Phe Ser Val Asn
   225 230 235 240
   Ala Gly Val Thr Thr Pro Val Ser Thr Tyr Met Leu Thr Asn Ser Glu
   245 250 255
   Leu Leu Ser Leu Ile Asn Asp Met Pro Ile Thr Asn Asp Gln Lys Lys
   260 265 270
   Leu Met Ser Asn Asn Val Gln Ile Val Arg Gln Gln Ser Tyr Ser Ile
   275 280 285
   Met Ser Ile Ile Lys Glu Glu Val Leu Ala Tyr Val Val Gln Leu Pro
   290 295 300
   Leu Tyr Gly Val Ile Asp Thr Pro Cys Trp Lys Leu His Thr Ser Pro
   305 310 315 320
   Leu Cys Thr Thr Asn Thr Lys Glu Gly Ser Asn Ile Cys Leu Thr Arg
   325 330 335
   Thr Asp Arg Gly Trp Tyr Cys Asp Asn Ala Gly Ser Val Ser Phe Phe
   340 345 350
   Pro Gln Ala Glu Thr Cys Lys Val Gln Ser Asn Arg Val Phe Cys Asp
   355 360 365
   Thr Met Asn Ser Leu Thr Leu Pro Ser Glu Val Asn Leu Cys Asn Val
   370 375 380
   Asp Ile Phe Asn Pro Lys Tyr Asp Cys Lys Ile Met Thr Ser Lys Thr
   385 390 395 400
   Asp Val Ser Ser Ser Val Ile Thr Ser Leu Gly Ala Ile Val Ser Cys
   405 410 415
   Tyr Gly Lys Thr Lys Cys Thr Ala Ser Asn Lys Asn Arg Gly Ile Ile
   420 425 430
   Lys Thr Phe Ser Asn Gly Cys Asp Tyr Val Ser Asn Lys Gly Val Asp
   435 440 445
   Thr Val Ser Val Gly Asn Thr Leu Tyr Tyr Val Asn Lys Gln Glu Gly
   450 455 460
   Lys Ser Leu Tyr Val Lys Gly Glu Pro Ile Ile Asn Phe Tyr Asp Pro
   465 470 475 480
   Leu Val Phe Pro Ser Asp Glu Phe Asp Ala Ser Ile Ser Gln Val Asn
   485 490 495
   Glu Lys Ile Asn Gln Ser Leu Ala Phe Ile Arg Lys Ser Asp Glu Leu
   500 505 510
   Leu His His Val Asn Ala Gly Lys Ser Thr Thr Asn Ile Met Ile Thr
   515 520 525
   Thr Ile Ile Ile Val Ile Ile Val Ile Leu Leu Ser Leu Ile Ala Val
   530 535 540
   Gly Leu Leu Leu Tyr Cys Lys Ala Arg Ser Thr Pro Val Thr Leu Ser
   545 550 555 560
   Lys Asp Gln Leu Ser Gly Ile Asn Asn Ile Ala Phe Ser Asn
   565 570
<210> 2
   <211> 298
   <212> PRT
   <213> Human respiratory syncytial virus
<400> 2
   Met Ser Lys Asn Lys Asp Gln Arg Thr Ala Lys Thr Leu Glu Lys Thr
   1               5                   10                  15
   Trp Asp Thr Leu Asn His Leu Leu Phe Ile Ser Ser Gly Leu Tyr Lys
               20                  25                  30
   Leu Asn Leu Lys Ser Ile Ala Gln Ile Thr Leu Ser Ile Leu Ala Met
           35                  40                  45
   Ile Ile Ser Thr Ser Leu Ile Ile Thr Ala Ile Ile Phe Ile Ala Ser
       50                  55                  60
   Ala Asn His Lys Val Thr Leu Thr Thr Ala Ile Ile Gln Asp Ala Thr
   65                  70                  75                  80
   Ser Gln Ile Lys Asn Thr Thr Pro Thr Tyr Leu Thr Gln Asp Pro Gln
                   85                  90                  95
   Leu Gly Ile Ser Phe Ser Asn Leu Ser Glu Ile Thr Ser Gln Thr Thr
               100                 105                 110
   Thr Ile Leu Ala Ser Thr Thr Pro Gly Val Lys Ser Asn Leu Gln Pro
           115                 120                 125
   Thr Thr Val Lys Thr Lys Asn Thr Thr Thr Thr Gln Thr Gln Pro Ser
   130 135 140
   Lys Pro Thr Thr Lys Gln Arg Gln Asn Lys Pro Pro Asn Lys Pro Asn
   145 150 155 160
   Asn Asp Phe His Phe Glu Val Phe Asn Phe Val Pro Cys Ser Ile Cys
   165 170 175
   Ser Asn Asn Pro Thr Cys Trp Ala Ile Cys Lys Arg Ile Pro Asn Lys
   180 185 190
   Lys Pro Gly Lys Lys Thr Thr Thr Lys Pro Thr Lys Lys Pro Thr Phe
   195 200 205
   Lys Thr Thr Lys Lys Asp Leu Lys Pro Gln Thr Thr Lys Pro Lys Glu
   210 215 220
   Val Pro Thr Thr Lys Pro Thr Glu Glu Pro Thr Ile Asn Thr Thr Lys
   225 230 235 240
   Thr Asn Ile Thr Thr Thr Leu Leu Thr Asn Asn Thr Thr Gly Asn Pro
   245 250 255
   Lys Leu Thr Ser Gln Met Glu Thr Phe His Ser Thr Ser Ser Glu Gly
   260 265 270
   Asn Leu Ser Pro Ser Gln Val Ser Thr Thr Ser Glu His Pro Ser Gln
   275 280 285
   Pro Ser Ser Pro Pro Asn Thr Thr Arg Gln
   290 295
<210> 3
   <211> 714
   <212> DNA
   <213> Artificial Sequence
<220>
   <223> Synthetic Oligonucleotide
<400> 3
   gaattcatgc ataaggtgac tcctacaacg gctatcattc aggacgccac ctcccaaatc 60
   aaaaacacta cacccactta tctgacacag aacccccaac tgggcatcag cccttccaac 120
   ccttctgaaa tcacttccca gatcaccact atcttggctt ctactacccc tggggtcaag 180
   tccactctgc agtctaccac agtcaaaaca aagaatacaa ccactaccca gactcagcca 240
   agcaagccaa caacaaagca gcgacaaaat aaacccccta gtaagccaaa taacgacttc 300
   cactttgagg tgtttaattt tgttccttgc agtatctgct ctaacaatcc cacctgttgg 360
   gcgatatgta aacgcatccc gaataagaag ccaggtaaga agacaaccac aaagcccaca 420
   aagaaaccca ccctgaaaac aaccaagaaa gatccaaagc cccagacgac caaaagcaaa 480
   gaggtgccta cgacaaagcc gacagaagag cctacaatca ataccaccaa gaccaacatt 540
   attaccaccc ttcttacttc taacactacc ggaaatcctg agttgacaag tcagatggag 600
   acattccatt caacgtcctc agaaggcaac ccaagcccct cccaggtatc aaccacctct 660
   gaatacccga gccagccctc cagtccccca aacaccccac ggcagtaatc taga 714
<210> 4
   <211> 233
   <212> PRT
   <213> Artificial Sequence
<220>
   <223> Synthetic Peptide
<400> 4
   Met His Lys Val Thr Pro Thr Thr Ala Ile Ile Gln Asp Ala Thr Ser
   1               5                   10                  15
   Gln Ile Lys Asn Thr Thr Pro Thr Tyr Leu Thr Gln Asn Pro Gln Leu
               20                  25                  30
   Gly Ile Ser Pro Ser Asn Pro Ser Glu Ile Thr Ser Gln Ile Thr Thr
           35                  40                  45
   Ile Leu Ala Ser Thr Thr Pro Gly Val Lys Ser Thr Leu Gln Ser Thr
       50                  55                  60
   Thr Val Lys Thr Lys Asn Thr Thr Thr Thr Gln Thr Gln Pro Ser Lys
   65                  70                  75                  80
   Pro Thr Thr Lys Gln Arg Gln Asn Lys Pro Pro Ser Lys Pro Asn Asn
                   85                  90                  95
   Asp Phe His Phe Glu Val Phe Asn Phe Val Pro Cys Ser Ile Cys Ser
               100                 105                 110
   Asn Asn Pro Thr Cys Trp Ala Ile Cys Lys Arg Ile Pro Asn Lys Lys
           115                 120                 125
   Pro Gly Lys Lys Thr Thr Thr Lys Pro Thr Lys Lys Pro Thr Leu Lys
       130                 135                 140
   Thr Thr Lys Lys Asp Pro Lys Pro Gln Thr Thr Lys Ser Lys Glu Val
   145                 150                 155                 160
   Pro Thr Thr Lys Pro Thr Glu Glu Pro Thr Ile Asn Thr Thr Lys Thr
                   165                 170                 175
   Asn Ile Ile Thr Thr Leu Leu Thr Ser Asn Thr Thr Gly Asn Pro Glu
               180                 185                 190
   Leu Thr Ser Gln Met Glu Thr Phe His Ser Thr Ser Ser Glu Gly Asn
           195                 200                 205
   Pro Ser Pro Ser Gln Val Ser Thr Thr Ser Glu Tyr Pro Ser Gln Pro
       210                 215                 220
   Ser Ser Pro Pro Asn Thr Pro Arg Gln
   225                 230
<210> 5
   <211> 714
   <212> DNA
   <213> Artificial Sequence
<220>
   <223> Synthetic Oligonucleotide
<400> 5
   gaattcatgc ataaagtaac cccgaccacc gctatcatcc aggacgctac cagccagatc      60
   aaaaacacta cgcctaccta tctgactcag aacccgcaac tgggcatctc cccgtccaat     120
   ccgtctgaaa ttacctccca gatcactacc atcctggcat ccactactcc gggtgtgaaa     180
   tctaccctgc agtccactac cgtaaaaacg aaaaacacca ccactaccca gactcagcct     240
   tccaaaccta ctacgaaaca gcgtcagaac aaaccgccga gcaaaccgaa caacgacttc     300
   cactttgaag ttttcaactt cgtcccatgc agcatttgta gcaacaatcc gacctgctgg     360
   gcaatttgca aacgcatccc aaacaaaaag ccgggcaaaa agacgaccac taaaccaacc     420
   aagaaaccta ccctgaaaac taccaaaaaa gacccgaaac cgcagaccac caaatctaaa     480
   gaagttccga cgaccaaacc gaccgaggaa ccgacgatca acaccacgaa aacgaacatc     540
   atcaccaccc tgctgacctc taacactacc ggtaatccgg agctgactag ccagatggaa     600
   acctttcaca gcacttcttc tgaaggtaac ccatctccga gccaggtgtc caccacttct     660
   gaatacccga gccaaccgtc ctccccgcct aatacgccgc gtcaataact cgag           714
<210> 6
   <211> 15226
   <212> DNA
   <213> Human respiratory syncytial virus
<400> 6
   acgcgaaaaa atgcgtacaa caaacttgcg taaaccaaaa aaatggggca aataagaatt      60
   tgataagtac cacttaaatt taactccctt ggttagagat gggcagcaat tcgttgagta     120
   tgataaaagt tagattacaa aatttgtttg acaatgatga agtagcattg ttaaaaataa     180
   catgctatac tgacaaatta atacatttaa ctaatgcttt ggctaaggca gtgatacata     240
   caatcaaatt gaatggcatt gtgtttgtgc atgttattac aagtagtgat atttgcccta     300
   ataataatat tgtagtaaaa tccaatttca caacaatgcc agtgctacaa aatggaggtt     360
   atatatggga aatgatggaa ttaacacatt gctctcaacc taatggtcta atagatgaca     420
   attgtgaaat taaattctcc aaaaaactaa gtgattcaac aatgaccaat tatatgaatc     480
   aattatctga attacttgga tttgatctta atccataaat tataattaat atcaactagc     540
   aaatcaatgt cactagcacc attagttaat ataaaactta acagaagaca aaaatggggc     600
   aaataaatca actcagccaa cccaaccatg gacacaaccc acaatgatac cacaccacaa     660
   agactgatga tcacagacat gagaccgttg tcacttgaga ctacaataac atcactaacc     720
   agagacatca taacacacag atttatatac ttaataaatc atgaatgcat agtgagaaaa     780
   cttgatgaaa gacaggccac atttacattc ctggtcaact atgaaatgaa actattgcac     840
   aaagtaggaa gcactaaata taaaaaatat actgaataca acacaaaata tggcactttc     900
   cctatgccga tattcatcaa tcatgatggg ttcttagaat gcattggcat taagcctaca     960
   aagcatactc ccataatata caagtatgat ctcaatccat gaatttcaac acaagattca    1020
   cacaatccaa aacaacaact ttatgcataa ctacactcca tagtccaaat ggagcctgaa    1080
   aattatagta atttaaaatt aaggagagac ataagataga agatggggca aatacaaaga    1140
   tggctcttag caaagtcaag ttgaatgata cactcaacaa agatcaactt ctgtcatcta    1200
   gcaaatacac catccaacgg agcacaggag atagtattga tactcctaat tatgatgtgc    1260
   agaaacacat caataagtta tgtggcatgt tattaatcac agaagatgct aatcataaat    1320
   tcactgggtt aataggtatg ttatatgcta tgtctaggtt aggaagagaa gacaccataa    1380
   aaatactcag agatgcggga tatcatgtaa aagcaaatgg agtagatgta acaacacatc    1440
   gtcaagacat caatgggaaa gaaatgaaat ttgaagtgtt aacattggca agcttaacaa    1500
   ctgaaattca aatcaacatt gagatagaat ctagaaaatc ctacaaaaaa atgctaaaag    1560
   aaatgggaga ggtagctcca gaatacaggc atgattctcc tgattgtggg atgataatat    1620
   tatgtatagc agcattagta ataaccaaat tggcagcagg ggatagatct ggtcttacag    1680
   ccgtgattag gagagctaat aatgtcctaa aaaatgaaat gaaacgttac aaaggcttac    1740
   tacccaagga tatagccaac agcttctatg aagtgtttga aaaacatccc cactttatag    1800
   atgtttttgt tcattttggt atagcacaat cttccaccag aggtggcagt agagttgaag    1860
   ggatttttgc aggattgttt atgaatgcct atggtgcagg gcaagtaatg ctacggtggg    1920
   gagtcttagc aaaatcagtt aaaaatatta tgttaggaca tgctagtgtg caagcagaaa    1980
   tggaacaagt tgttgaggtt tatgaatatg cccaaaaatt gggtggagaa gcaggattct    2040
   accatatatt gaacaaccca aaagcatcat tattatcttt gactcaattt cctcactttt    2100
   ccagtgtagt attaggcaat gctgctggcc taggcataat gggagagtac agaggtacac    2160
   cgaggaatca agatctatat gatgcagcaa aggcatatgc tgaacaactc aaagaaaatg    2220
   gtgtgattaa ctacagtgta ttagacttga cagcagaaga actagaggct atcaaacatc    2280
   agcttaatcc aaaagataat gatgtagagc tttgagttaa taaaaaatgg ggcaaataaa    2340
   tcatcatgga aaagtttgct cctgaattcc atggagaaga tgcaaacaac agggctacta    2400
   aattcctaga atcaataaag ggcaaattca catcacctaa agatcccaag aaaaaagata    2460
   gtatcatatc tgtcaactca atagatatag aagtaaccaa agaaagccct ataacatcaa 2520
   attcaaccat tattaaccca acaaatgaga cagatgataa tgcagggaac aagcccaatt 2580
   atcaaagaaa acctctagta agtttcaaag aagaccctat accaagtgat aatccctttt 2640
   caaaactata caaagaaacc atagagacat ttgataacaa tgaagaagaa tctagctatt 2700
   catatgaaga aataaatgat cagacgaacg ataatataac tgcaagatta gataggattg 2760
   atgaaaaatt aagtgaaata ctaggaatgc ttcacacatt agtagtagca agtgcaggac 2820
   ctacatctgc tagggatggt ataagagatg ccatggttgg tttaagagaa gaaatgatag 2880
   aaaaaatcag aactgaagca ttaatgacca atgacagatt agaagctatg gcaagactca 2940
   ggaatgagga aagtgaaaag atggcaaaag acacatcaga tgaagtgtct ctcaatccaa 3000
   catcagagaa attgaacaac ctgttggaag ggaatgatag tgacaatgat ctatcacttg 3060
   aagatttctg attagttaca aatctgcact tcaacacaca acaccaacag aagaccaaca 3120
   aacaaaccaa cccactcatc caaccaaaca tccatccgcc aatcagccaa acagccaaca 3180
   aaacaaccag ccaatccaaa accagccacc tggaaaaaat cgacaatata gttacaaaaa 3240
   aagaaaaggg tggggcaaat atggaaacat acgtgaacaa gcttcacgaa ggctccacat 3300
   acacagctgc tgttcaatac aatgtcctag aaaaagacga tgaccctgca tcacttacaa 3360
   tatgggtgcc catgttccaa tcatctatgc cagcagattt acttataaaa gaactagcta 3420
   atgtcaacat actagtgaaa caaatatcca cacccaaggg accttcacta agagtcatga 3480
   taaactcaag aagtgcattg ctagcacaaa tgcccagcaa atttaccata tgtgctaatg 3540
   tgtccttgga tgaaagaagc aaactggcat atgatgtaac cacaccctgt gaaatcaagg 3600
   catgtagtct aacatgccta aaatcaaaaa atatgttaac tacagttaaa gatctcacta 3660
   tgaagacact caaccccaca catgatatta ttgctttatg tgaatttgaa aacatagtaa 3720
   catcaaaaaa agtcataata ccaacatacc taagatccat cagtgtcaga aataaagatc 3780
   tgaacacact tgaaaatata acaaccactg aattcaaaaa tgccatcaca aatgcaaaaa 3840
   tcatccctta ctcaggatta ctattagtca tcacagtgac tgacaacaaa ggagcattca 3900
   aatacataaa gccgcaaagt caattcatag tagatcttgg agcttaccta gaaaaagaaa 3960
   gtatatatta tgttaccaca aattggaagc acacagctac acgatttgca atcaaaccca 4020
   tggaagatta acctttttcc tccacatcag tgagtcaatt catacaaact ttctacctac 4080
   attcttcact tcaccattac aatcacaaac actctgtggt tcaaccaatc aaacaaaact 4140
   tatctgaagt ctcagatcat cccaagtcat tgttcatcag atctagtaat caaataagtt 4200
   aataaaaata tacacatggg gcaaataatc atcggaggaa atccaactaa tcacaatatc 4260
   tgttaacata gacaagtcaa cacaccagac agaatcaacc aatggaaaat acatccataa 4320
   caatagaatt ctcaagcaaa ttctggcctt actttacact aatacacatg atcacaacaa 4380
   taatctcttt gctaatcata atctccatca tgactgcaat actaaacaaa ctttgtgaat 4440
   ataacgtatt ccataacaaa acctttgagt taccaagagc tcgagtcaac acatagcatt 4500
   catcaatcta atagctcaaa atagtaacct tgcatttaaa agtgaacaac ccccacctct 4560
   ttacaacacc tcattaacat cccaccatgc aaaccaccat ccatactata aagtagttaa 4620
   ttaaaaatag tcataacaat gaactaggat atcaagacta acaataacgt tggggcaaat 4680
   gcaaacatgt ccaaaaacaa ggaccaacgc accgctaaga cactagaaaa gacctgggac 4740
   actctcaatc atttattatt catatcatcg ggcttatata agttaaatct taaatctata 4800
   gcacaaatca cattatccat tctggcaatg ataatctcaa cttcacttat aattacagcc 4860
   atcatattca tagcctcggc aaaccacaaa gtcacactaa caactgcaat catacaagat 4920
   gcaacaagcc agatcaagaa cacaacccca acatacctca ctcaggatcc tcagcttgga 4980
   atcagcttct ccaatctgtc tgaaattaca tcacaaacca ccaccatact agcttcaaca 5040
   acaccaggag tcaagtcaaa cctgcaaccc acaacagtca agactaaaaa cacaacaaca 5100
   acccaaacac aacccagcaa gcccactaca aaacaacgcc aaaacaaacc accaaacaaa 5160
   cccaataatg attttcactt cgaagtgttt aactttgtac cctgcagcat atgcagcaac 5220
   aatccaacct gctgggctat ctgcaaaaga ataccaaaca aaaaaccagg aaagaaaacc 5280
   accaccaagc ctacaaaaaa accaaccttc aagacaacca aaaaagatct caaacctcaa 5340
   accactaaac caaaggaagt acccaccacc aagcccacag aagagccaac catcaacacc 5400
   accaaaacaa acatcacaac tacactgctc accaacaaca ccacaggaaa tccaaaactc 5460
   acaagtcaaa tggaaacctt ccactcaacc tcctccgaag gcaatctaag cccttctcaa 5520
   gtctccacaa catccgagca cccatcacaa ccctcatctc cacccaacac aacacgccag 5580
   tagttattaa aaaacatatt atcacaaaag gccatgacca actcaaacag aatcaaaata 5640
   aactctgggg caaataacaa tggagttgcc aatcctcaaa gcaaatgcaa ttaccacaat 5700
   cctcgctgca gtcacatttt gctttgcttc tagtcaaaac atcactgaag aattttatca 5760
   atcaacatgc agtgcagtta gcaaaggcta tcttagtgct ctaagaactg gttggtatac 5820
   tagtgttata actatagaat taagtaatat caaggaaaat aagtgtaatg gaacagatgc 5880
   taaggtaaaa ttgataaacc aagaattaga taaatataaa aatgctgtaa cagaattgca 5940
   gttgctcatg caaagcacaa cagcagcaaa caatcgagcc agaagagaac taccaaggtt 6000
   tatgaattat acactcaaca ataccaaaaa aaccaatgta acattaagca agaaaaggaa 6060
   aagaagattt cttggttttt tgttaggtgt tggatctgca atcgccagtg gcattgctgt 6120
   atctaaggtc ctgcacttag aaggagaagt gaacaagatc aaaagtgctc tactatccac 6180
   aaacaaggcc gtagtcagct tatcaaatgg agttagtgtc ttaaccagca aagtgttaga 6240
   cctcaaaaac tatatagata aacaattgtt acctattgtg aataagcaaa gctgcagaat 6300
   atcaaatata gaaactgtga tagagttcca acaaaagaac aacagactac tagagattac 6360
   cagggaattt agtgttaatg caggtgtaac tacacctgta agcacttaca tgttaactaa 6420
   tagtgaatta ttgtcattaa tcaatgatat gcctataaca aatgatcaga aaaagttaat 6480
   gtccaacaat gttcaaatag ttagacagca aagttactct atcatgtcca taataaaaga 6540
   ggaagtctta gcatatgtag tacaattacc actatatggt gtgatagata caccttgttg 6600
   gaaattacac acatcccctc tatgtacaac caacacaaaa gaagggtcaa acatctgttt 6660
   aacaagaact gacagaggat ggtactgtga caatgcagga tcagtatctt tcttcccaca 6720
   agctgaaaca tgtaaagttc aatcgaatcg agtattttgt gacacaatga acagtttaac 6780
   attaccaagt gaagtaaatc tctgcaatgt tgacatattc aatcccaaat atgattgtaa 6840
   aattatgact tcaaaaacag atgtaagcag ctccgttatc acatctctag gagccattgt 6900
   gtcatgctat ggcaaaacta aatgtacagc atccaataaa aatcgtggaa tcataaagac 6960
   attttctaac gggtgtgatt atgtatcaaa taaaggggtg gacactgtgt ctgtaggtaa 7020
   cacattatat tatgtaaata agcaagaagg caaaagtctc tatgtaaaag gtgaaccaat 7080
   aataaatttc tatgacccat tagtattccc ctctgatgaa tttgatgcat caatatctca 7140
   agtcaatgag aagattaacc agagtttagc atttattcgt aaatccgatg aattattaca 7200
   tcatgtaaat gctggtaaat caaccacaaa tatcatgata actactataa ttatagtgat 7260
   tatagtaata ttgttatcat taattgctgt tggactgctc ctatactgta aggccagaag 7320
   cacaccagtc acactaagca aggatcaact gagtggtata aataatattg catttagtaa 7380
   ctgaataaaa atagcaccta atcatgttct tacaatggtt tactatctgc tcatagacaa 7440
   cccatctatc attggatttt cttaaaatct gaacttcatc gaaactctta tctataaacc 7500
   atctcactta cactatttaa gtagattcct agtttatagt tatataaaac acaattgaat 7560
   accagattaa cttactatct gtaaaaatga gaactggggc aaatatgtca cgaaggaatc 7620
   cttgcaaatt tgaaattcga ggtcattgct tgaatggtaa gagatgtcat tttagtcata 7680
   attattttga atggccaccc catgcactgc tcgtaagaca aaactttatg ttaaacagaa 7740
   tacttaagtc tatggataaa agtatagata ccttatcaga aataagtgga gctgcagagt 7800
   tggacagaac agaagagtat gctcttggtg tagttggagt gctagagagt tatataggat 7860
   caataaataa tataactaaa caatcagcat gtgttgccat gagcaaactc ctcactgaac 7920
   tcaatagtga tgatatcaaa aaactgagag acaatgaaga gctaaattca cccaagataa 7980
   gagtgtacaa tactgtcata tcatatattg aaagcaacag gaaaaacaat aaacaaacta 8040
   tccatctgtt aaaaagattg ccagcagacg tattgaagaa aaccatcaaa aacacattgg 8100
   atatccacaa gagcataacc atcaacaacc caaaagaatt aactgttagt gatacaaatg 8160
   accatgccaa aaataatgat actacctgac aaatatcctt gtagtataac ttccatacta 8220
   ataacaagta gatgtagagt cactatgtat aatcgaaaga acacactata tttcaatcaa 8280
   aacaacccaa ataaccatat gtactcaccg aatcaaacat tcaatgaaat ccattggacc 8340
   tcacaagact tgattgacac aattcaaaat tttctacagc atctaggtgt tattgaggat 8400
   atatatacaa tatatatatt agtgtcataa cactcaatcc taatactgac catatcgttg 8460
   aattattaat tcaaataatt caagctgtgg gacaaaatgg atcccattat taatggaaat 8520
   tctgctaatg tttatctaac cgatagttat ttaaaaggtg ttatctcttt ctcagagtgt 8580
   aatgctttag gaagttacat attcaatggt ccttatctca aaaatgatta taccaactta 8640
   attagtagac aaaatccatt aatagaacac atgaatctaa agaaactaaa tataacacag 8700
   tccttaatat ctaagtatca taaaggtgaa ataaaattag aagagcctac ttattttcag 8760
   tcattactta tgacatacaa gagtatgacc tcgttggaac agattgctac cactaattta 8820
   cttaaaaaga taataagaag agctatagaa ataagtgatg tcaaagtcta tgctatattg 8880
   aataaactag ggcttaaaga aaaggacaag attaaatcca acaatggaca ggatgaagac 8940
   aactcagtta ttacgaccat aatcaaagat gatatacttt cagctgttaa ggataatcaa 9000
   tctcatctta aagcagacaa aaatcactct acaaaacaaa aagacacaat caaaacaaca 9060
   ctcttgaaga aattaatgtg ttcaatgcag catcctccat catggttaat acattggttt 9120
   aatttataca caaaattaaa caacatatta acacagtatc gatcaaatga ggttaaaaac 9180
   catgggttta tattgataga taatcaaact cttagtggat ttcaatttat tttgaatcaa 9240
   tatggttgta tagtttatca taaggaactc aaaagaatta ctgtgacaac ctataatcaa 9300
   ttcttgacat ggaaagatat tagccttagt agattaaatg tttgtttaat tacatggatt 9360
   agtaactgct tgaacacatt aaataaaagc ttaggcttaa gatgcggatt caataatgtt 9420
   atcttgacac aactattcct ttatggtgat tgtatactaa agctatttca caatgagggg 9480
   ttctacataa taaaagaggt agaggatttt attatgtctc taattttaaa tataacagaa 9540
   gaagatcaat tcagaaaacg attttataat agtatgctca acaacatcac agatgctgct 9600
   aataaagctc agaaaaatct gctatcaaga gtatgtcata cattattaga taagacagta 9660
   tccgataata taataaatgg cagatggata attctattaa gtaagttcct taaattaatt 9720
   aagcttgcag gtgacaataa ccttaacaat ctgagtgaac tatatttttt gttcagaata 9780
   tttggacacc caatggtaga tgaaagacaa gccatggatg ctgttaaagt taattgcaat 9840
   gagaccaaat tttacttgtt aagcagtttg agtatgttaa gaggtgcctt tatatataga 9900
   attataaaag ggtttgtaaa taattacaac agatggccta ctttaagaaa tgctattgtt 9960
   ttacccttaa gatggttaac ttactataaa ctaaacactt atccttcttt gttggaactt 10020
   acagaaagag atttgattgt gttatcagga ctacgtttct atcgtgagtt tcggttgcct 10080
   aaaaaagtgg atcttgaaat gattataaat gataaagcta tatcaccccc taaaaatttg 10140
   atatggacta gtttccctag aaattatatg ccgtcacaca tacaaaacta tatagaacat 10200
   gaaaaattaa aattttccga gagtgataaa tcaagaagag tattagagta ttatttaaga 10260
   gataacaaat tcaatgaatg tgatttatac aactgtgtag ttaatcaaag ttatctcaac 10320
   aaccctaatc atgtggtatc attgacaggc aaagaaagag aactcagtgt aggtagaatg 10380
   tttgcaatgc aaccgggaat gttcagacag gttcaaatat tggcagagaa aatgatagct 10440
   gaaaacattt tacaattctt tcctgaaagt cttacaagat atggtgatct agaactacaa 10500
   aaaatattag aattgaaagc aggaataagt aacaaatcaa atcgctacaa tgataattac 10560
   aacaattaca ttagtaagtg ctctatcatc acagatctca gcaaattcaa tcaagcattt 10620
   cgatatgaaa cgtcatgtat ttgtagtgat gtgctggatg aactgcatgg tgtacaatct 10680
   ctattttcct ggttacattt aactattcct catgtcacaa taatatgcac atataggcat 10740
   gcacccccct atataagaga tcatattgta gatcttaaca atgtagatga acaaagtgga 10800
   ttatatagat atcacatggg tggtattgaa gggtggtgtc aaaaactatg gaccatagaa 10860
   gctatatcac tattggatct aatatctctc aaagggaaat tctcaattac tgctttaatt 10920
   aatggtgaca atcaatcaat agatataagc aaaccagtca gactcatgga aggtcaaact 10980
   catgctcaag cagattattt gctagcatta aatagcctta aattactgta taaagagtat 11040
   gcaggcatag gtcacaaatt aaaaggaact gagacttata tatcacgaga tatgcaattt 11100
   atgagtaaaa caattcaaca taacggtgta tattaccctg ctagtataaa gaaagtccta 11160
   agagtgggac cgtggataaa cactatactt gatgatttca aagtgagtct agaatctata 11220
   ggtagtttga cacaagaatt agaatataga ggtgaaagtc tattatgcag tttaatattt 11280
   agaaatgtat ggttatataa tcaaattgct ctacaattaa aaaatcatgc gttatgtaac 11340
   aataaattat atttggacat attaaaggtt ctgaaacact taaaaacctt ttttaatctt 11400
   gataatattg atacagcatt aacattgtat atgaatttac ccatgttatt tggtggtggt 11460
   gatcccaact tgttatatcg aagtttctat agaagaactc ctgatttcct cacagaggct 11520
   atagttcact ctgtgttcat acttagttat tatacaaacc atgacttaaa agataaactt 11580
   caagatttgt cagatgatag attgaataag ttcttaacat gcataatcac gtttgacaaa 11640
   aaccctaatg ctgaattcgt aacattgatg agagatcctc aagctttagg gtctgagaga 11700
   caagctaaaa ttactagtga aatcaataga ctggcagtta cagaggtttt gagtacagct 11760
   ccaaacaaaa tattctccaa aagtgcacaa cattatacca ctacagagat agatctaaat 11820
   gatattatgc aaaatataga acctacatat cctcacgggc taagagttgt ttatgaaagt 11880
   ttaccctttt ataaagcaga gaaaatagta aatcttatat caggtacaaa atctataact 11940
   aacatactgg aaaagacttc tgccatagac ttaacagata ttgatagagc cactgagatg 12000
   atgaggaaaa acataacttt gcttataagg atacttccat tggattgtaa cagagataaa 12060
   agagaaatat tgagtatgga aaacctaagt attactgaat taagcaaata tgttagggaa 12120
   agatcttggt ctttatccaa tatagttggt gttacatcac ccagtatcat gtatacaatg 12180
   gacatcaaat atacaacaag cactatagct agtggcataa ttatagagaa atataatgtt 12240
   aacagtttaa cacgtggtga gagaggacca actaaaccat gggttggttc atctacacaa 12300
   gagaaaaaaa caatgccagt ttataataga caagttttaa ccaaaaaaca aagagatcaa 12360
   atagatctat tagcaaaatt ggattgggtg tatgcatcta tagataacaa ggatgaattc 12420
   atggaagaac tcagcatagg aacccttggg ttaacatatg aaaaggccaa aaaattattt 12480
   ccacaatatt taagtgtcaa ctatttgcat cgccttacag tcagtagtag accatgtgaa 12540
   ttccctgcat caataccagc ttatagaaca acaaattatc actttgacac tagccctatt 12600
   aatcgcatat taacagaaaa gtatggtgat gaagatattg acatagtatt ccaaaactgt 12660
   ataagctttg gccttagctt aatgtcagta gtagaacaat ttactaatgt atgtcctaac 12720
   agaattattc tcatacctaa gcttaatgag atacatttga tgaaacctcc catattcaca 12780
   ggtgatgttg atattcacaa gttaaaacaa gtgatacaaa aacagcatat gtttttacca 12840
   gacaaaataa gtttgactca atatgtggaa ttattcttaa gtaacaaaac actcaaatct 12900
   ggatctcatg ttaattctaa tttaatattg gcacataaaa tatctgacta ttttcataat 12960
   acttacattt taagtactaa tttagctgga cattggattc taattataca acttatgaaa 13020
   gattctaaag gtatttttga aaaagattgg ggagagggat atataactga tcatatgttt 13080
   attaatttga aagttttctt caatgcttat aagacctatc tcttgtgttt tcataaaggt 13140
   tatggcaaag caaaactgga gtgtgatatg aacacttcag atcttctatg tgtattggaa 13200
   ttaatagaca gtagttattg gaagtctatg tctaaggtat ttttagaaca aaaagttatc 13260
   aaatacattc ttagccaaga tgcaagttta catagagtaa aaggatgtca tagcttcaaa 13320
   ttatggtttc ttaaacgtct taatgtagca gaatttacag tttgcccttg ggttgttaac 13380
   atagattatc atccaacaca tatgaaagca atattaactt atatagatct tgttagaatg 13440
   ggattgataa atatagatag aatacacatt aaaaataaac acaaattcaa tgatgaattt 13500
   tatacttcta atctctttta cattaattat aacttctcag ataatactca tctattaact 13560
   aaacatataa ggattgctaa ttcagaatta gaaaataatt acaacaaatt atatcatcct 13620
   acaccagaaa ccctagagaa tatactagcc aatccgatta aaagtaatga caaaaagaca 13680
   ctgaacgact attgtatagg taaaaatgtt gactcaataa tgttaccatt gttatctaat 13740
   aagaagcttg ttaaatcgtc tgcaatgatt agaaccaatt acagcaaaca agacctgtac 13800
   aatctattcc ctacggttgt gatcgataga attatagatc attcaggtaa tacagccaaa 13860
   tccaaccaac tttacactac tacttcccat caaatatctt tagtgcacaa tagcacatca 13920
   ctttattgca tgcttccttg gcatcatatt aatagattca attttgtatt tagttctaca 13980
   ggttgtaaaa ttagtataga gtatatttta aaagacctta aaattaaaga tcctaattgt 14040
   atagcattca taggtgaagg agcagggaat ttattattgc gtacagtggt ggaacttcat 14100
   cctgacataa gatatattta cagaagtctg aaagattgca atgatcatag tttacctatt 14160
   gagtttttaa ggctatacaa tggacatatc aacattgatt atggtgaaaa tttgaccatt 14220
   cctgctacag atgcaaccaa caacattcat tggtcttatt tacatataaa gtttgctgaa 14280
   cctatcagtc tttttgtatg tgatgccgaa ttgcctgtaa cagtcaactg gagtaaaatt 14340
   ataatagaat ggagcaagca tgtaagaaaa tgcaagtact gttcctcagt taataaatgt 14400
   acgttaatag taaaatatca tgctcaagat gatattgatt tcaaattaga caatataact 14460
   atattaaaaa cttatgtatg cttaggcagt aagttaaagg gatcggaggt ttacttagtc 14520
   cttacaatag gtcctgcaaa tatatttcca gtatttaatg tagtacaaaa tgctaaattg 14580
   atactatcaa gaaccaaaaa tttcatcatg cctaagaaag ctgataaaga gtctattgat 14640
   gcaaatatta aaagtttgat accctttctt tgttacccta taacaaaaaa aggaattaat 14700
   actgcattgt caaaactaaa gagtgttgtt agtggagata tactatcata ttctatagct     14760
   ggacggaatg aagttttcag caataaactt ataaatcata agcatatgaa catcttaaag     14820
   tggttcaatc atgttttaaa tttcagatca acagaactaa actataacca tttatatatg     14880
   gtagaatcta catatcctta cctaagtgaa ttgttaaaca gcttgacaac taatgaactt     14940
   aaaaaactga ttaaaatcac aggtagtctg ttatacaact ttcataatga ataatgaata     15000
   aagatcttat aataaaaatt cctatagcta tacactagca ctgtattcaa ttatagttat     15060
   taaaaaatta aaaatcatat aattttttat aaaaataact tttagtgaac taatcctaaa     15120
   gttatcattt tgatctagga ggaataaatt taaatcccaa tctaattggt ttatatgtgt     15180
   attaactaaa ctacgagata ttagtttttg acactttttt tctcgt                    15226
<210> 7
   <211> 18
   <212> DNA
   <213> Artificial Sequence
<220>
   <223> Synthetic Oligonucleotide
<400> 7
   atgcataagg tgactcct 18
<210> 8
   <211> 18
   <212> DNA
   <213> Artificial Sequence
<220>
   <223> Synthetic Oligonucleotide
<400> 8
   ttactgccgt ggggtgtt 18
<210> 9
   <211> 19
   <212> DNA
   <213> Artificial Sequence
<220>
   <223> Synthetic Oligonucleotide
<400> 9
   acaggagaag ggacgccat 19
<210> 10
   <211> 21
   <212> DNA
   <213> Artificial Sequence
<220>
   <223> Synthetic Oligonucleotide
<400> 10
   gaagccctac agacgagctc a 21
<210> 11
   <211> 23
   <212> DNA
   <213> Artificial Sequence
<220>
   <223> Synthetic Oligonucleotide
<400> 11
   agcacagtgg tgaaagagac ctt 23
<210> 12
   <211> 22
   <212> DNA
   <213> Artificial Sequence
<220>
   <223> Synthetic Oligonucleotide
<400> 12
   tccaatgcat agctggtgat tt 22
<210> 13
   <211> 21
   <212> DNA
   <213> Artificial Sequence
<220>
   <223> Synthetic Oligonucleotide
<400> 13
   ggagctgagc aacatcacac a 21
<210> 14
   <211> 21
   <212> DNA
   <213> Artificial Sequence
<220>
   <223> Synthetic Oligonucleotide
<400> 14
   ggtcctgtag atggcattgc a 21
<210> 15
   <211> 24
   <212> DNA
   <213> Artificial Sequence
<220>
   <223> Synthetic Oligonucleotide
<400> 15
   tcaagtggca tagatgtgga agaa 24
<210> 16
   <211> 21
   <212> DNA
   <213> Artificial Sequence
<220>
   <223> Synthetic Oligonucleotide
<400> 16
   tggctctgca ggattttcat g 21
<210> 17
   <211> 20
   <212> DNA
   <213> Artificial Sequence
<220>
   <223> Synthetic Oligonucleotide
<400> 17
   ctggtgaaaa ggacctctcg 20
<210> 18
   <211> 26
   <212> DNA
   <213> Artificial Sequence
<220>
   <223> Synthetic Oligonucleotide
<400> 18
   tgaagtactc attatagtca agggca 26
<210> 19
   <211> 897
   <212> DNA
   <213> Human respiratory syncytial virus
<400> 19
   atgtccaaaa acaaggacca acgcaccgct aagacactag aaaagacctg ggacactctc 60
   aatcatttat tattcatatc atcgggctta tataagttaa atcttaaatc tatagcacaa 120
   atcacattat ccattctggc aatgataatc tcaacttcac ttataattac agccatcata 180
   ttcatagcct cggcaaacca caaagtcaca ctaacaactg caatcataca agatgcaaca 240
   agccagatca agaacacaac cccaacatac ctcactcagg atcctcagct tggaatcagc 300
   ttctccaatc tgtctgaaat tacatcacaa accaccacca tactagcttc aacaacacca 360
   ggagtcaagt caaacctgca acccacaaca gtcaagacta aaaacacaac aacaacccaa 420
   acacaaccca gcaagcccac tacaaaacaa cgccaaaaca aaccaccaaa caaacccaat 480
   aatgattttc acttcgaagt gtttaacttt gtaccctgca gcatatgcag caacaatcca 540
   acctgctggg ctatctgcaa aagaatacca aacaaaaaac caggaaagaa aaccaccacc 600
   aagcctacaa aaaaaccaac cttcaagaca accaaaaaag atctcaaacc tcaaaccact 660
   aaaccaaagg aagtacccac caccaagccc acagaagagc caaccatcaa caccaccaaa 720
   acaaacatca caactacact gctcaccaac aacaccacag gaaatccaaa actcacaagt 780
   caaatggaaa ccttccactc aacctcctcc gaaggcaatc taagcccttc tcaagtctcc 840
   acaacatccg agcacccatc acaaccctca tctccaccca acacaacacg ccagtag 897
<210> 20
   <211> 702
   <212> DNA
   <213> Artificial Sequence
<220>
   <223> Synthetic Oligonucleotide
<400> 20
   atgcacaaag tcacaccaac aactgcaatc atacaagatg caacaagcca gatcaagaac 60
   acaaccccaa catacctcac ccagaatcct cagcttggaa tcagtccctc taatccgtct 120
   gaaattacat cacaaatcac caccatacta gcttcaacaa caccaggagt caagtcaacc 180
   ctgcaatcca caacagtcaa gaccaaaaac acaacaacaa ctcaaacaca acccagcaag 240
   cccaccacaa aacaacgcca aaacaaacca ccaagcaaac ccaataatga ttttcacttt 300
   gaagtgttca actttgtacc ctgcagcata tgcagcaaca atccaacctg ctgggctatc 360
   tgcaaaagaa taccaaacaa aaaaccagga aagaaaacca ctaccaagcc cacaaaaaaa 420
   ccaaccctca agacaaccaa aaaagatccc aaacctcaaa ccactaaatc aaaggaagta 480
   cccaccacca agcccacaga agagccaacc atcaacacca ccaaaacaaa catcataact 540
   acactactca cctccaacac cacaggaaat ccagaactca caagtcaaat ggaaaccttc 600
   cactcaactt cctccgaagg caatccaagc ccttctcaag tctctacaac atccgagtac 660
   ccatcacaac cttcatctcc acccaacaca ccacgccagt ag 702
<210> 21
   <211> 702
   <212> DNA
   <213> Artificial Sequence
<220>
   <223> Synthetic Oligonucleotide
<400> 21
   atgcataaag taaccccgac caccgctatc atccaggacg ctaccagcca gatcaaaaac      60
   actacgccta cctatctgac tcagaacccg caactgggca tctccccgtc caatccgtct     120
   gaaattacct cccagatcac taccatcctg gcatccacta ctccgggtgt gaaatctacc     180
   ctgcagtcca ctaccgtaaa aacgaaaaac accaccacta cccagactca gccttccaaa     240
   cctactacga aacagcgtca gaacaaaccg ccgagcaaac cgaacaacga cttccacttt     300
   gaagttttca acttcgtccc atgcagcatt tgtagcaaca atccgacctg ctgggcaatt     360
   tgcaaacgca tcccaaacaa aaagccgggc aaaaagacga ccactaaacc aaccaagaaa     420
   cctaccctga aaactaccaa aaaagacccg aaaccgcaga ccaccaaatc taaagaagtt     480
   ccgacgacca aaccgaccga ggaaccgacg atcaacacca cgaaaacgaa catcatcacc     540
   accctgctga cctctaacac taccggtaat ccggagctga ctagccagat ggaaaccttt     600
   cacagcactt cttctgaagg taacccatct ccgagccagg tgtccaccac ttctgaatac     660
   ccgagccaac cgtcctcccc gcctaatacg ccgcgtcaat aa                        702
<210> 22
   <211> 702
   <212> DNA
   <213> Artificial Sequence
<220>
   <223> Synthetic Oligonucleotide
<400> 22
   atgcataagg tgactcctac aacggctatc attcaggacg ccacctccca aatcaaaaac    60
   actacaccca cttatctgac acagaacccc caactgggca tcagcccttc caacccttct   120
   gaaatcactt cccagatcac cactatcttg gcttctacta cccctggggt caagtccact   180
   ctgcagtcta ccacagtcaa aacaaagaat acaaccacta cccagactca gccaagcaag   240
   ccaacaacaa agcagcgaca aaataaaccc cctagtaagc caaataacga cttccacttt   300
   gaggtgttta attttgttcc ttgcagtatc tgctctaaca atcccacctg ttgggcgata   360
   tgtaaacgca tcccgaataa gaagccaggt aagaagacaa ccacaaagcc cacaaagaaa   420
   cccaccctga aaacaaccaa gaaagatcca aagccccaga cgaccaaaag caaagaggtg   480
   cctacgacaa agccgacaga agagcctaca atcaatacca ccaagaccaa cattattacc   540
   acccttctta cttctaacac taccggaaat cctgagttga caagtcagat ggagacattc   600
   cattcaacgt cctcagaagg caacccaagc ccctcccagg tatcaaccac ctctgaatac   660
   ccgagccagc cctccagtcc cccaaacacc ccacggcagt aa                      702
<210> 23
   <211> 699
   <212> DNA
   <213> Artificial Sequence
<220>
   <223> Synthetic Oligonucleotide
<400> 23
<210> 24
   <211> 699
   <212> DNA
   <213> Artificial Sequence
<220>
   <223> Synthetic Oligonucleotide
<400> 24
   atggctattg taagctgcta tggtaagact aaatgcactg cgagcaataa aaaccgtggt      60
   attatcaaaa cctttagcaa cggctgtgat tacgtatcca acaaaggcgt tgacactgtt     120
   tctgtgggca acaccctgta ttacgtgaac aagcaggaag gcaaaagcct gtacgtgaaa     180
   ggtgaaccga ttatcaactt ttacgacccg ctggtcttcc cgtctgatga gttcgatgct     240
   tctatcagcc aggttaacga aaagatcaat cagtctctgg ctttcatccg taaaagcgat     300
   gagctgctgc ataacgtcaa cgctggtaaa tctaccacta acggtggtgg cggtggcgct     360
   attgttagct gctacggtaa aacgaaatgc accgctagca acaaaaatcg tggcatcatc     420
   aaaacgttct ctaacggttg cgactatgtt tctaacaaag gtgtagacac tgtgtctgtg     480
   ggtaacactc tgtactacgt taacaaacag gaaggtaagt ctctgtacgt taaaggcgag     540
   ccgatcatca acttctacga cccactggtt tttccatctg acgaatttga cgcatctatt     600
   agccaggtga acgagaaaat caaccagagc ctggcgttca tccgcaaatc cgacgaactg     660
   ctgcacaacg ttaacgctgg caaatccacc acgaactaa                            699
<210> 25
   <211> 232
   <212> PRT
   <213> Artificial Sequence
<220>
   <223> Synthetic Peptide
<400> 25
   Met Ala Ile Val Ser Cys Tyr Gly Lys Thr Lys Cys Thr Ala Ser Asn
   1               5                   10                  15
   Lys Asn Arg Gly Ile Ile Lys Thr Phe Ser Asn Gly Cys Asp Tyr Val
               20                  25                  30
   Ser Asn Lys Gly Val Asp Thr Val Ser Val Gly Asn Thr Leu Tyr Tyr
           35                  40                  45
   Val Asn Lys Gln Glu Gly Lys Ser Leu Tyr Val Lys Gly Glu Pro Ile
       50                  55                  60
   Ile Asn Phe Tyr Asp Pro Leu Val Phe Pro Ser Asp Glu Phe Asp Ala
   65                  70                  75                  80
   Ser Ile Ser Gln Val Asn Glu Lys Ile Asn Gln Ser Leu Ala Phe Ile
                   85                  90                  95
   Arg Lys Ser Asp Glu Leu Leu His Asn Val Asn Ala Gly Lys Ser Thr
               100                 105                 110
   Thr Asn Gly Gly Gly Gly Gly Ala Ile Val Ser Cys Tyr Gly Lys Thr
   115 120 125
   Lys Cys Thr Ala Ser Asn Lys Asn Arg Gly Ile Ile Lys Thr Phe Ser
   130 135 140
   Asn Gly Cys Asp Tyr Val Ser Asn Lys Gly Val Asp Thr Val Ser Val
   145 150 155 160
   Gly Asn Thr Leu Tyr Tyr Val Asn Lys Gln Glu Gly Lys Ser Leu Tyr
   165 170 175
   Val Lys Gly Glu Pro Ile Ile Asn Phe Tyr Asp Pro Leu Val Phe Pro
   180 185 190
   Ser Asp Glu Phe Asp Ala Ser Ile Ser Gln Val Asn Glu Lys Ile Asn
   195 200 205
   Gln Ser Leu Ala Phe Ile Arg Lys Ser Asp Glu Leu Leu His Asn Val
   210 215 220
   Asn Ala Gly Lys Ser Thr Thr Asn
   225 230
<210> 26
   <211> 1725
   <212> DNA
   <213> Human respiratory syncytial virus
<400> 26
   atggagttgc caatcctcaa agcaaatgca attaccacaa tcctcgctgc agtcacattt 60
   tgctttgctt ctagtcaaaa catcactgaa gaattttatc aatcaacatg cagtgcagtt 120
   agcaaaggct atcttagtgc tctaagaact ggttggtata ctagtgttat aactatagaa 180
   ttaagtaata tcaaggaaaa taagtgtaat ggaacagatg ctaaggtaaa attgataaac 240
   caagaattag ataaatataa aaatgctgta acagaattgc agttgctcat gcaaagcaca 300
   acagcagcaa acaatcgagc cagaagagaa ctaccaaggt ttatgaatta tacactcaac 360
   aataccaaaa aaaccaatgt aacattaagc aagaaaagga aaagaagatt tcttggtttt 420
   ttgttaggtg ttggatctgc aatcgccagt ggcattgctg tatctaaggt cctgcactta 480
   gaaggagaag tgaacaagat caaaagtgct ctactatcca caaacaaggc cgtagtcagc 540
   ttatcaaatg gagttagtgt cttaaccagc aaagtgttag acctcaaaaa ctatatagat 600
   aaacaattgt tacctattgt gaataagcaa agctgcagaa tatcaaatat agaaactgtg 660
   atagagttcc aacaaaagaa caacagacta ctagagatta ccagggaatt tagtgttaat 720
   gcaggtgtaa ctacacctgt aagcacttac atgttaacta atagtgaatt attgtcatta 780
   atcaatgata tgcctataac aaatgatcag aaaaagttaa tgtccaacaa tgttcaaata 840
   gttagacagc aaagttactc tatcatgtcc ataataaaag aggaagtctt agcatatgta 900
   gtacaattac cactatatgg tgtgatagat acaccttgtt ggaaattaca cacatcccct 960
   ctatgtacaa ccaacacaaa agaagggtca aacatctgtt taacaagaac tgacagagga 1020
   tggtactgtg acaatgcagg atcagtatct ttcttcccac aagctgaaac atgtaaagtt 1080
   caatcgaatc gagtattttg tgacacaatg aacagtttaa cattaccaag tgaagtaaat 1140
   ctctgcaatg ttgacatatt caatcccaaa tatgattgta aaattatgac ttcaaaaaca 1200
   gatgtaagca gctccgttat cacatctcta ggagccattg tgtcatgcta tggcaaaact 1260
   aaatgtacag catccaataa aaatcgtgga atcataaaga cattttctaa cgggtgtgat 1320
   tatgtatcaa ataaaggggt ggacactgtg tctgtaggta acacattata ttatgtaaat 1380
   aagcaagaag gcaaaagtct ctatgtaaaa ggtgaaccaa taataaattt ctatgaccca 1440
   ttagtattcc cctctgatga atttgatgca tcaatatctc aagtcaatga gaagattaac 1500
   cagagtttag catttattcg taaatccgat gaattattac atcatgtaaa tgctggtaaa 1560
   tcaaccacaa atatcatgat aactactata attatagtga ttatagtaat attgttatca 1620
   ttaattgctg ttggactgct cctatactgt aaggccagaa gcacaccagt cacactaagc 1680
   aaggatcaac tgagtggtat aaataatatt gcatttagta actga 1725
<210> 27
   <211> 420
   <212> DNA
   <213> Human respiratory syncytial virus
<400> 27
   atgggcagca attcgttgag tatgataaaa gttagattac aaaatttgtt tgacaatgat        60
   gaagtagcat tgttaaaaat aacatgctat actgacaaat taatacattt aactaatgct       120
   ttggctaagg cagtgataca tacaatcaaa ttgaatggca ttgtgtttgt gcatgttatt       180
   acaagtagtg atatttgccc taataataat attgtagtaa aatccaattt cacaacaatg       240
   ccagtgctac aaaatggagg ttatatatgg gaaatgatgg aattaacaca ttgctctcaa       300
   cctaatggtc taatagatga caattgtgaa attaaattct ccaaaaaact aagtgattca       360
   acaatgacca attatatgaa tcaattatct gaattacttg gatttgatct taatccataa       420
<210> 28
   <211> 139
   <212> PRT
   <213> Human respiratory syncytial virus
<400> 28
   Met Gly Ser Asn Ser Leu Ser Met Ile Lys Val Arg Leu Gln Asn Leu
   1               5                   10                  15
   Phe Asp Asn Asp Glu Val Ala Leu Leu Lys Ile Thr Cys Tyr Thr Asp
               20                  25                  30
   Lys Leu Ile His Leu Thr Asn Ala Leu Ala Lys Ala Val Ile His Thr
           35                  40                  45
   Ile Lys Leu Asn Gly Ile Val Phe Val His Val Ile Thr Ser Ser Asp
       50                  55                  60
   Ile Cys Pro Asn Asn Asn Ile Val Val Lys Ser Asn Phe Thr Thr Met
   65                  70                  75                  80
   Pro Val Leu Gln Asn Gly Gly Tyr Ile Trp Glu Met Met Glu Leu Thr
                   85                  90                  95
   His Cys Ser Gln Pro Asn Gly Leu Ile Asp Asp Asn Cys Glu Ile Lys
               100                 105                 110
   Phe Ser Lys Lys Leu Ser Asp Ser Thr Met Thr Asn Tyr Met Asn Gln
           115                 120                 125
   Leu Ser Glu Leu Leu Gly Phe Asp Leu Asn Pro
       130                 135
<210> 29
   <211> 375
   <212> DNA
   <213> Human respiratory syncytial virus
<400> 29
   atggacacaa cccacaatga taccacacca caaagactga tgatcacaga catgagaccg      60
   ttgtcacttg agactacaat aacatcacta accagagaca tcataacaca cagatttata     120
   tacttaataa atcatgaatg catagtgaga aaacttgatg aaagacaggc cacatttaca     180
   ttcctggtca actatgaaat gaaactattg cacaaagtag gaagcactaa atataaaaaa     240
   tatactgaat acaacacaaa atatggcact ttccctatgc cgatattcat caatcatgat     300
   gggttcttag aatgcattgg cattaagcct acaaagcata ctcccataat atacaagtat     360
   gatctcaatc catga                                                      375
<210> 30
   <211> 124
   <212> PRT
   <213> Human respiratory syncytial virus
<400> 30
   Met Asp Thr Thr His Asn Asp Thr Thr Pro Gln Arg Leu Met Ile Thr
   1               5                   10                  15
   Asp Met Arg Pro Leu Ser Leu Glu Thr Thr Ile Thr Ser Leu Thr Arg
               20                  25                  30
   Asp Ile Ile Thr His Arg Phe Ile Tyr Leu Ile Asn His Glu Cys Ile
           35                  40                  45
   Val Arg Lys Leu Asp Glu Arg Gln Ala Thr Phe Thr Phe Leu Val Asn
       50                  55                  60
   Tyr Glu Met Lys Leu Leu His Lys Val Gly Ser Thr Lys Tyr Lys Lys
   65                  70                  75                  80
   Tyr Thr Glu Tyr Asn Thr Lys Tyr Gly Thr Phe Pro Met Pro Ile Phe
                   85                  90                  95
   Ile Asn His Asp Gly Phe Leu Glu Cys Ile Gly Ile Lys Pro Thr Lys
               100                 105                 110
   His Thr Pro Ile Ile Tyr Lys Tyr Asp Leu Asn Pro
           115                 120
<210> 31
   <211> 1176
   <212> DNA
   <213> Human respiratory syncytial virus
<400> 31
   atggctctta gcaaagtcaa gttgaatgat acactcaaca aagatcaact tctgtcatct      60
   agcaaataca ccatccaacg gagcacagga gatagtattg atactcctaa ttatgatgtg     120
   cagaaacaca tcaataagtt atgtggcatg ttattaatca cagaagatgc taatcataaa     180
   ttcactgggt taataggtat gttatatgct atgtctaggt taggaagaga agacaccata     240
   aaaatactca gagatgcggg atatcatgta aaagcaaatg gagtagatgt aacaacacat     300
   cgtcaagaca tcaatgggaa agaaatgaaa tttgaagtgt taacattggc aagcttaaca     360
   actgaaattc aaatcaacat tgagatagaa tctagaaaat cctacaaaaa aatgctaaaa     420
   gaaatgggag aggtagctcc agaatacagg catgattctc ctgattgtgg gatgataata     480
   ttatgtatag cagcattagt aataaccaaa ttggcagcag gggatagatc tggtcttaca     540
   gccgtgatta ggagagctaa taatgtccta aaaaatgaaa tgaaacgtta caaaggctta     600
   ctacccaagg atatagccaa cagcttctat gaagtgtttg aaaaacatcc ccactttata     660
   gatgtttttg ttcattttgg tatagcacaa tcttccacca gaggtggcag tagagttgaa     720
   gggatttttg caggattgtt tatgaatgcc tatggtgcag ggcaagtaat gctacggtgg     780
   ggagtcttag caaaatcagt taaaaatatt atgttaggac atgctagtgt gcaagcagaa     840
   atggaacaag ttgttgaggt ttatgaatat gcccaaaaat tgggtggaga agcaggattc     900
   taccatatat tgaacaaccc aaaagcatca ttattatctt tgactcaatt tcctcacttt     960
   tccagtgtag tattaggcaa tgctgctggc ctaggcataa tgggagagta cagaggtaca    1020
   ccgaggaatc aagatctata tgatgcagca aaggcatatg ctgaacaact caaagaaaat    1080
   ggtgtgatta actacagtgt attagacttg acagcagaag aactagaggc tatcaaacat    1140
   cagcttaatc caaaagataa tgatgtagag ctttga                              1176
<210> 32
   <211> 391
   <212> PRT
   <213> Human respiratory syncytial virus
<400> 32
   Met Ala Leu Ser Lys Val Lys Leu Asn Asp Thr Leu Asn Lys Asp Gln
   1               5                   10                  15
   Leu Leu Ser Ser Ser Lys Tyr Thr Ile Gln Arg Ser Thr Gly Asp Ser
               20                  25                  30
   Ile Asp Thr Pro Asn Tyr Asp Val Gln Lys His Ile Asn Lys Leu Cys
           35                  40                  45
   Gly Met Leu Leu Ile Thr Glu Asp Ala Asn His Lys Phe Thr Gly Leu
       50                  55                  60
   Ile Gly Met Leu Tyr Ala Met Ser Arg Leu Gly Arg Glu Asp Thr Ile
   65                  70                  75                  80
   Lys Ile Leu Arg Asp Ala Gly Tyr His Val Lys Ala Asn Gly Val Asp
                   85                  90                  95
   Val Thr Thr His Arg Gln Asp Ile Asn Gly Lys Glu Met Lys Phe Glu
               100                 105                 110
   Val Leu Thr Leu Ala Ser Leu Thr Thr Glu Ile Gln Ile Asn Ile Glu
           115                 120                 125
   Ile Glu Ser Arg Lys Ser Tyr Lys Lys Met Leu Lys Glu Met Gly Glu
       130                 135                 140
   Val Ala Pro Glu Tyr Arg His Asp Ser Pro Asp Cys Gly Met Ile Ile
   145                 150                 155                 160
   Leu Cys Ile Ala Ala Leu Val Ile Thr Lys Leu Ala Ala Gly Asp Arg
                   165                 170                 175
   Ser Gly Leu Thr Ala Val Ile Arg Arg Ala Asn Asn Val Leu Lys Asn
               180                 185                 190
   Glu Met Lys Arg Tyr Lys Gly Leu Leu Pro Lys Asp Ile Ala Asn Ser
           195                 200                 205
   Phe Tyr Glu Val Phe Glu Lys His Pro His Phe Ile Asp Val Phe Val
       210                 215                 220
   His Phe Gly Ile Ala Gln Ser Ser Thr Arg Gly Gly Ser Arg Val Glu
   225                 230                 235                 240
   Gly Ile Phe Ala Gly Leu Phe Met Asn Ala Tyr Gly Ala Gly Gln Val
                   245                 250                 255
   Met Leu Arg Trp Gly Val Leu Ala Lys Ser Val Lys Asn Ile Met Leu
   260 265 270
   Gly His Ala Ser Val Gln Ala Glu Met Glu Gln Val Val Glu Val Tyr
   275 280 285
   Glu Tyr Ala Gln Lys Leu Gly Gly Glu Ala Gly Phe Tyr His Ile Leu
   290 295 300
   Asn Asn Pro Lys Ala Ser Leu Leu Ser Leu Thr Gln Phe Pro His Phe
   305 310 315 320
   Ser Ser Val Val Leu Gly Asn Ala Ala Gly Leu Gly Ile Met Gly Glu
   325 330 335
   Tyr Arg Gly Thr Pro Arg Asn Gln Asp Leu Tyr Asp Ala Ala Lys Ala
   340 345 350
   Tyr Ala Glu Gln Leu Lys Glu Asn Gly Val Ile Asn Tyr Ser Val Leu
   355 360 365
   Asp Leu Thr Ala Glu Glu Leu Glu Ala Ile Lys His Gln Leu Asn Pro
   370 375 380
   Lys Asp Asn Asp Val Glu Leu
   385 390
<210> 33
   <211> 726
   <212> DNA
   <213> Human respiratory syncytial virus
<400> 33
   atggaaaagt ttgctcctga attccatgga gaagatgcaa acaacagggc tactaaattc 60
   ctagaatcaa taaagggcaa attcacatca cctaaagatc ccaagaaaaa agatagtatc 120
   atatctgtca actcaataga tatagaagta accaaagaaa gccctataac atcaaattca 180
   accattatta acccaacaaa tgagacagat gataatgcag ggaacaagcc caattatcaa 240
   agaaaacctc tagtaagttt caaagaagac cctataccaa gtgataatcc cttttcaaaa 300
   ctatacaaag aaaccataga gacatttgat aacaatgaag aagaatctag ctattcatat 360
   gaagaaataa atgatcagac gaacgataat ataactgcaa gattagatag gattgatgaa 420
   aaattaagtg aaatactagg aatgcttcac acattagtag tagcaagtgc aggacctaca 480
   tctgctaggg atggtataag agatgccatg gttggtttaa gagaagaaat gatagaaaaa 540
   atcagaactg aagcattaat gaccaatgac agattagaag ctatggcaag actcaggaat 600
   gaggaaagtg aaaagatggc aaaagacaca tcagatgaag tgtctctcaa tccaacatca 660
   gagaaattga acaacctgtt ggaagggaat gatagtgaca atgatctatc acttgaagat 720
   ttctga 726
<210> 34
   <211> 241
   <212> PRT
   <213> Human respiratory syncytial virus
<400> 34
   Met Glu Lys Phe Ala Pro Glu Phe His Gly Glu Asp Ala Asn Asn Arg
   1               5                   10                  15
   Ala Thr Lys Phe Leu Glu Ser Ile Lys Gly Lys Phe Thr Ser Pro Lys
               20                  25                  30
   Asp Pro Lys Lys Lys Asp Ser Ile Ile Ser Val Asn Ser Ile Asp Ile
           35                  40                  45
   Glu Val Thr Lys Glu Ser Pro Ile Thr Ser Asn Ser Thr Ile Ile Asn
       50                  55                  60
   Pro Thr Asn Glu Thr Asp Asp Asn Ala Gly Asn Lys Pro Asn Tyr Gln
   65                  70                  75                  80
   Arg Lys Pro Leu Val Ser Phe Lys Glu Asp Pro Ile Pro Ser Asp Asn
                   85                  90                  95
   Pro Phe Ser Lys Leu Tyr Lys Glu Thr Ile Glu Thr Phe Asp Asn Asn
               100                 105                 110
   Glu Glu Glu Ser Ser Tyr Ser Tyr Glu Glu Ile Asn Asp Gln Thr Asn
           115                 120                 125
   Asp Asn Ile Thr Ala Arg Leu Asp Arg Ile Asp Glu Lys Leu Ser Glu
       130                 135                 140
   Ile Leu Gly Met Leu His Thr Leu Val Val Ala Ser Ala Gly Pro Thr
   145                 150                 155                 160
   Ser Ala Arg Asp Gly Ile Arg Asp Ala Met Val Gly Leu Arg Glu Glu
                   165                 170                 175
   Met Ile Glu Lys Ile Arg Thr Glu Ala Leu Met Thr Asn Asp Arg Leu
               180                 185                 190
   Glu Ala Met Ala Arg Leu Arg Asn Glu Glu Ser Glu Lys Met Ala Lys
           195                 200                 205
   Asp Thr Ser Asp Glu Val Ser Leu Asn Pro Thr Ser Glu Lys Leu Asn
       210                 215                 220
   Asn Leu Leu Glu Gly Asn Asp Ser Asp Asn Asp Leu Ser Leu Glu Asp
   225                 230                 235                 240
   Phe
<210> 35
   <211> 771
   <212> DNA
   <213> Human respiratory syncytial virus
<400> 35
   atggaaacat acgtgaacaa gcttcacgaa ggctccacat acacagctgc tgttcaatac      60
   aatgtcctag aaaaagacga tgaccctgca tcacttacaa tatgggtgcc catgttccaa     120
   tcatctatgc cagcagattt acttataaaa gaactagcta atgtcaacat actagtgaaa     180
   caaatatcca cacccaaggg accttcacta agagtcatga taaactcaag aagtgcattg     240
   ctagcacaaa tgcccagcaa atttaccata tgtgctaatg tgtccttgga tgaaagaagc     300
   aaactggcat atgatgtaac cacaccctgt gaaatcaagg catgtagtct aacatgccta     360
   aaatcaaaaa atatgttaac tacagttaaa gatctcacta tgaagacact caaccccaca     420
   catgatatta ttgctttatg tgaatttgaa aacatagtaa catcaaaaaa agtcataata     480
   ccaacatacc taagatccat cagtgtcaga aataaagatc tgaacacact tgaaaatata     540
   acaaccactg aattcaaaaa tgccatcaca aatgcaaaaa tcatccctta ctcaggatta     600
   ctattagtca tcacagtgac tgacaacaaa ggagcattca aatacataaa gccgcaaagt     660
   caattcatag tagatcttgg agcttaccta gaaaaagaaa gtatatatta tgttaccaca     720
   aattggaagc acacagctac acgatttgca atcaaaccca tggaagatta a              771
<210> 36
   <211> 256
   <212> PRT
   <213> Human respiratory syncytial virus
<400> 36
   Met Glu Thr Tyr Val Asn Lys Leu His Glu Gly Ser Thr Tyr Thr Ala
   1               5                   10                  15
   Ala Val Gln Tyr Asn Val Leu Glu Lys Asp Asp Asp Pro Ala Ser Leu
               20                  25                  30
   Thr Ile Trp Val Pro Met Phe Gln Ser Ser Met Pro Ala Asp Leu Leu
           35                  40                  45
   Ile Lys Glu Leu Ala Asn Val Asn Ile Leu Val Lys Gln Ile Ser Thr
       50                  55                  60
   Pro Lys Gly Pro Ser Leu Arg Val Met Ile Asn Ser Arg Ser Ala Leu
   65                  70                  75                  80
   Leu Ala Gln Met Pro Ser Lys Phe Thr Ile Cys Ala Asn Val Ser Leu
                   85                  90                  95
   Asp Glu Arg Ser Lys Leu Ala Tyr Asp Val Thr Thr Pro Cys Glu Ile
   100 105 110
   Lys Ala Cys Ser Leu Thr Cys Leu Lys Ser Lys Asn Met Leu Thr Thr
   115 120 125
   Val Lys Asp Leu Thr Met Lys Thr Leu Asn Pro Thr His Asp Ile Ile
   130 135 140
   Ala Leu Cys Glu Phe Glu Asn Ile Val Thr Ser Lys Lys Val Ile Ile
   145 150 155 160
   Pro Thr Tyr Leu Arg Ser Ile Ser Val Arg Asn Lys Asp Leu Asn Thr
   165 170 175
   Leu Glu Asn Ile Thr Thr Thr Glu Phe Lys Asn Ala Ile Thr Asn Ala
   180 185 190
   Lys Ile Ile Pro Tyr Ser Gly Leu Leu Leu Val Ile Thr Val Thr Asp
   195 200 205
   Asn Lys Gly Ala Phe Lys Tyr Ile Lys Pro Gln Ser Gln Phe Ile Val
   210 215 220
   Asp Leu Gly Ala Tyr Leu Glu Lys Glu Ser Ile Tyr Tyr Val Thr Thr
   225 230 235 240
   Asn Trp Lys His Thr Ala Thr Arg Phe Ala Ile Lys Pro Met Glu Asp
   245 250 255
<210> 37
   <211> 195
   <212> DNA
   <213> Human respiratory syncytial virus
<400> 37
   atggaaaata catccataac aatagaattc tcaagcaaat tctggcctta ctttacacta 60
   atacacatga tcacaacaat aatctctttg ctaatcataa tctccatcat gactgcaata 120
   ctaaacaaac tttgtgaata taacgtattc cataacaaaa cctttgagtt accaagagct 180
   cgagtcaaca catag 195
<210> 38
   <211> 64
   <212> PRT
   <213> Human respiratory syncytial virus
<400> 38
   Met Glu Asn Thr Ser Ile Thr Ile Glu Phe Ser Ser Lys Phe Trp Pro
   1               5                   10                  15
   Tyr Phe Thr Leu Ile His Met Ile Thr Thr Ile Ile Ser Leu Leu Ile
               20                  25                  30
   Ile Ile Ser Ile Met Thr Ala Ile Leu Asn Lys Leu Cys Glu Tyr Asn
           35                  40                  45
   Val Phe His Asn Lys Thr Phe Glu Leu Pro Arg Ala Arg Val Asn Thr
       50                  55                  60
<210> 39
   <211> 585
   <212> DNA
   <213> Human respiratory syncytial virus
<400> 39
   atgtcacgaa ggaatccttg caaatttgaa attcgaggtc attgcttgaa tggtaagaga 60
   tgtcatttta gtcataatta ttttgaatgg ccaccccatg cactgctcgt aagacaaaac 120
   tttatgttaa acagaatact taagtctatg gataaaagta tagatacctt atcagaaata 180
   agtggagctg cagagttgga cagaacagaa gagtatgctc ttggtgtagt tggagtgcta 240
   gagagttata taggatcaat aaataatata actaaacaat cagcatgtgt tgccatgagc 300
   aaactcctca ctgaactcaa tagtgatgat atcaaaaaac tgagagacaa tgaagagcta 360
   aattcaccca agataagagt gtacaatact gtcatatcat atattgaaag caacaggaaa 420
   aacaataaac aaactatcca tctgttaaaa agattgccag cagacgtatt gaagaaaacc 480
   atcaaaaaca cattggatat ccacaagagc ataaccatca acaacccaaa agaattaact 540
   gttagtgata caaatgacca tgccaaaaat aatgatacta cctga 585
<210> 40
   <211> 194
   <212> PRT
   <213> Human respiratory syncytial virus
<400> 40
   Met Ser Arg Arg Asn Pro Cys Lys Phe Glu Ile Arg Gly His Cys Leu
   1 5 10 15
   Asn Gly Lys Arg Cys His Phe Ser His Asn Tyr Phe Glu Trp Pro Pro
   20 25 30
   His Ala Leu Leu Val Arg Gln Asn Phe Met Leu Asn Arg Ile Leu Lys
   35 40 45
   Ser Met Asp Lys Ser Ile Asp Thr Leu Ser Glu Ile Ser Gly Ala Ala
   50 55 60
   Glu Leu Asp Arg Thr Glu Glu Tyr Ala Leu Gly Val Val Gly Val Leu
   65 70 75 80
   Glu Ser Tyr Ile Gly Ser Ile Asn Asn Ile Thr Lys Gln Ser Ala Cys
   85 90 95
   Val Ala Met Ser Lys Leu Leu Thr Glu Leu Asn Ser Asp Asp Ile Lys
   100 105 110
   Lys Leu Arg Asp Asn Glu Glu Leu Asn Ser Pro Lys Ile Arg Val Tyr
   115 120 125
   Asn Thr Val Ile Ser Tyr Ile Glu Ser Asn Arg Lys Asn Asn Lys Gln
   130 135 140
   Thr Ile His Leu Leu Lys Arg Leu Pro Ala Asp Val Leu Lys Lys Thr
   145 150 155 160
   Ile Lys Asn Thr Leu Asp Ile His Lys Ser Ile Thr Ile Asn Asn Pro
   165 170 175
   Lys Glu Leu Thr Val Ser Asp Thr Asn Asp His Ala Lys Asn Asn Asp
   180 185 190
   Thr Thr
<210> 41
   <211> 273
   <212> DNA
   <213> Human respiratory syncytial virus
<400> 41
   atgaccatgc caaaaataat gatactacct gacaaatatc cttgtagtat aacttccata      60
   ctaataacaa gtagatgtag agtcactatg tataatcgaa agaacacact atatttcaat     120
   caaaacaacc caaataacca tatgtactca ccgaatcaaa cattcaatga aatccattgg     180
   acctcacaag acttgattga cacaattcaa aattttctac agcatctagg tgttattgag     240
   gatatatata caatatatat attagtgtca taa                                  273
<210> 42
   <211> 90
   <212> PRT
   <213> Human respiratory syncytial virus
<400> 42
   Met Thr Met Pro Lys Ile Met Ile Leu Pro Asp Lys Tyr Pro Cys Ser
   1               5                   10                  15
   Ile Thr Ser Ile Leu Ile Thr Ser Arg Cys Arg Val Thr Met Tyr Asn
               20                  25                  30
   Arg Lys Asn Thr Leu Tyr Phe Asn Gln Asn Asn Pro Asn Asn His Met
           35                  40                  45
   Tyr Ser Pro Asn Gln Thr Phe Asn Glu Ile His Trp Thr Ser Gln Asp
       50                  55                  60
   Leu Ile Asp Thr Ile Gln Asn Phe Leu Gln His Leu Gly Val Ile Glu
   65                  70                  75                  80
   Asp Ile Tyr Thr Ile Tyr Ile Leu Val Ser
                   85                  90
<210> 43
   <211> 6498
   <212> DNA
   <213> Human respiratory syncytial virus
<400> 43
   atggatccca ttattaatgg aaattctgct aatgtttatc taaccgatag ttatttaaaa      60
   ggtgttatct ctttctcaga gtgtaatgct ttaggaagtt acatattcaa tggtccttat     120
   ctcaaaaatg attataccaa cttaattagt agacaaaatc cattaataga acacatgaat     180
   ctaaagaaac taaatataac acagtcctta atatctaagt atcataaagg tgaaataaaa     240
   ttagaagagc ctacttattt tcagtcatta cttatgacat acaagagtat gacctcgttg     300
   gaacagattg ctaccactaa tttacttaaa aagataataa gaagagctat agaaataagt     360
   gatgtcaaag tctatgctat attgaataaa ctagggctta aagaaaagga caagattaaa     420
   tccaacaatg gacaggatga agacaactca gttattacga ccataatcaa agatgatata     480
   ctttcagctg ttaaggataa tcaatctcat cttaaagcag acaaaaatca ctctacaaaa     540
   caaaaagaca caatcaaaac aacactcttg aagaaattaa tgtgttcaat gcagcatcct     600
   ccatcatggt taatacattg gtttaattta tacacaaaat taaacaacat attaacacag     660
   tatcgatcaa atgaggttaa aaaccatggg tttatattga tagataatca aactcttagt     720
   ggatttcaat ttattttgaa tcaatatggt tgtatagttt atcataagga actcaaaaga     780
   attactgtga caacctataa tcaattcttg acatggaaag atattagcct tagtagatta     840
   aatgtttgtt taattacatg gattagtaac tgcttgaaca cattaaataa aagcttaggc     900
   ttaagatgcg gattcaataa tgttatcttg acacaactat tcctttatgg tgattgtata     960
   ctaaagctat ttcacaatga ggggttctac ataataaaag aggtagaggg atttattatg    1020
   tctctaattt taaatataac agaagaagat caattcagaa aacgatttta taatagtatg    1080
   ctcaacaaca tcacagatgc tgctaataaa gctcagaaaa atctgctatc aagagtatgt    1140
   catacattat tagataagac agtatccgat aatataataa atggcagatg gataattcta    1200
   ttaagtaagt tccttaaatt aattaagctt gcaggtgaca ataaccttaa caatctgagt    1260
   gaactatatt ttttgttcag aatatttgga cacccaatgg tagatgaaag acaagccatg    1320
   gatgctgtta aagttaattg caatgagacc aaattttact tgttaagcag tttgagtatg    1380
   ttaagaggtg cctttatata tagaattata aaagggtttg taaataatta caacagatgg    1440
   cctactttaa gaaatgctat tgttttaccc ttaagatggt taacttacta taaactaaac    1500
   acttatcctt ctttgttgga acttacagaa agagatttga ttgtgttatc aggactacgt    1560
   ttctatcgtg agtttcggtt gcctaaaaaa gtggatcttg aaatgattat aaatgataaa    1620
   gctatatcac cccctaaaaa tttgatatgg actagtttcc ctagaaatta tatgccgtca    1680
   cacatacaaa actatataga acatgaaaaa ttaaaatttt ccgagagtga taaatcaaga 1740
   agagtattag agtattattt aagagataac aaattcaatg aatgtgattt atacaactgt 1800
   gtagttaatc aaagttatct caacaaccct aatcatgtgg tatcattgac aggcaaagaa 1860
   agagaactca gtgtaggtag aatgtttgca atgcaaccgg gaatgttcag acaggttcaa 1920
   atattggcag agaaaatgat agctgaaaac attttacaat tctttcctga aagtcttaca 1980
   agatatggtg atctagaact acaaaaaata ttagaattga aagcaggaat aagtaacaaa 2040
   tcaaatcgct acaatgataa ttacaacaat tacattagta agtgctctat catcacagat 2100
   ctcagcaaat tcaatcaagc atttcgatat gaaacgtcat gtatttgtag tgatgtgctg 2160
   gatgaactgc atggtgtaca atctctattt tcctggttac atttaactat tcctcatgtc 2220
   acaataatat gcacatatag gcatgcaccc ccctatataa gagatcatat tgtagatctt 2280
   aacaatgtag atgaacaaag tggattatat agatatcaca tgggtggtat tgaagggtgg 2340
   tgtcaaaaac tatggaccat agaagctata tcactattgg atctaatatc tctcaaaggg 2400
   aaattctcaa ttactgcttt aattaatggt gacaatcaat caatagatat aagcaaacca 2460
   gtcagactca tggaaggtca aactcatgct caagcagatt atttgctagc attaaatagc 2520
   cttaaattac tgtataaaga gtatgcaggc ataggtcaca aattaaaagg aactgagact 2580
   tatatatcac gagatatgca atttatgagt aaaacaattc aacataacgg tgtatattac 2640
   cctgctagta taaagaaagt cctaagagtg ggaccgtgga taaacactat acttgatgat 2700
   ttcaaagtga gtctagaatc tataggtagt ttgacacaag aattagaata tagaggtgaa 2760
   agtctattat gcagtttaat atttagaaat gtatggttat ataatcaaat tgctctacaa 2820
   ttaaaaaatc atgcgttatg taacaataaa ttatatttgg acatattaaa ggttctgaaa 2880
   cacttaaaaa ccttttttaa tcttgataat attgatacag cattaacatt gtatatgaat 2940
   ttacccatgt tatttggtgg tggtgatccc aacttgttat atcgaagttt ctatagaaga 3000
   actcctgatt tcctcacaga ggctatagtt cactctgtgt tcatacttag ttattataca 3060
   aaccatgact taaaagataa acttcaagat ttgtcagatg atagattgaa taagttctta 3120
   acatgcataa tcacgtttga caaaaaccct aatgctgaat tcgtaacatt gatgagagat 3180
   cctcaagctt tagggtctga gagacaagct aaaattacta gtgaaatcaa tagactggca 3240
   gttacagagg ttttgagtac agctccaaac aaaatattct ccaaaagtgc acaacattat 3300
   accactacag agatagatct aaatgatatt atgcaaaata tagaacctac atatcctcac 3360
   gggctaagag ttgtttatga aagtttaccc ttttataaag cagagaaaat agtaaatctt 3420
   atatcaggta caaaatctat aactaacata ctggaaaaga cttctgccat agacttaaca 3480
   gatattgata gagccactga gatgatgagg aaaaacataa ctttgcttat aaggatactt 3540
   ccattggatt gtaacagaga taaaagagaa atattgagta tggaaaacct aagtattact 3600
   gaattaagca aatatgttag ggaaagatct tggtctttat ccaatatagt tggtgttaca 3660
   tcacccagta tcatgtatac aatggacatc aaatatacaa caagcactat agctagtggc 3720
   ataattatag agaaatataa tgttaacagt ttaacacgtg gtgagagagg accaactaaa 3780
   ccatgggttg gttcatctac acaagagaaa aaaacaatgc cagtttataa tagacaagtt 3840
   ttaaccaaaa aacaaagaga tcaaatagat ctattagcaa aattggattg ggtgtatgca 3900
   tctatagata acaaggatga attcatggaa gaactcagca taggaaccct tgggttaaca 3960
   tatgaaaagg ccaaaaaatt atttccacaa tatttaagtg tcaactattt gcatcgcctt 4020
   acagtcagta gtagaccatg tgaattccct gcatcaatac cagcttatag aacaacaaat 4080
   tatcactttg acactagccc tattaatcgc atattaacag aaaagtatgg tgatgaagat 4140
   attgacatag tattccaaaa ctgtataagc tttggcctta gcttaatgtc agtagtagaa 4200
   caatttacta atgtatgtcc taacagaatt attctcatac ctaagcttaa tgagatacat 4260
   ttgatgaaac ctcccatatt cacaggtgat gttgatattc acaagttaaa acaagtgata 4320
   caaaaacagc atatgttttt accagacaaa ataagtttga ctcaatatgt ggaattattc 4380
   ttaagtaaca aaacactcaa atctggatct catgttaatt ctaatttaat attggcacat 4440
   aaaatatctg actattttca taatacttac attttaagta ctaatttagc tggacattgg 4500
   attctaatta tacaacttat gaaagattct aaaggtattt ttgaaaaaga ttggggagag 4560
   ggatatataa ctgatcatat gtttattaat ttgaaagttt tcttcaatgc ttataagacc 4620
   tatctcttgt gttttcataa aggttatggc aaagcaaaac tggagtgtga tatgaacact 4680
   tcagatcttc tatgtgtatt ggaattaata gacagtagtt attggaagtc tatgtctaag 4740
   gtatttttag aacaaaaagt tatcaaatac attcttagcc aagatgcaag tttacataga 4800
   gtaaaaggat gtcatagctt caaattatgg tttcttaaac gtcttaatgt agcagaattt 4860
   acagtttgcc cttgggttgt taacatagat tatcatccaa cacatatgaa agcaatatta 4920
   acttatatag atcttgttag aatgggattg ataaatatag atagaataca cattaaaaat 4980
   aaacacaaat tcaatgatga attttatact tctaatctct tttacattaa ttataacttc 5040
   tcagataata ctcatctatt aactaaacat ataaggattg ctaattcaga attagaaaat 5100
   aattacaaca aattatatca tcctacacca gaaaccctag agaatatact agccaatccg 5160
   attaaaagta atgacaaaaa gacactgaac gactattgta taggtaaaaa tgttgactca 5220
   ataatgttac cattgttatc taataagaag cttgttaaat cgtctgcaat gattagaacc 5280
   aattacagca aacaagacct gtacaatcta ttccctacgg ttgtgatcga tagaattata 5340
   gatcattcag gtaatacagc caaatccaac caactttaca ctactacttc ccatcaaata 5400
   tctttagtgc acaatagcac atcactttat tgcatgcttc cttggcatca tattaataga 5460
   ttcaattttg tatttagttc tacaggttgt aaaattagta tagagtatat tttaaaagac 5520
   cttaaaatta aagatcctaa ttgtatagca ttcataggtg aaggagcagg gaatttatta 5580
   ttgcgtacag tggtggaact tcatcctgac ataagatata tttacagaag tctgaaagat 5640
   tgcaatgatc atagtttacc tattgagttt ttaaggctat acaatggaca tatcaacatt 5700
   gattatggtg aaaatttgac cattcctgct acagatgcaa ccaacaacat tcattggtct 5760
   tatttacata taaagtttgc tgaacctatc agtctttttg tatgtgatgc cgaattgcct   5820
   gtaacagtca actggagtaa aattataata gaatggagca agcatgtaag aaaatgcaag   5880
   tactgttcct cagttaataa atgtacgtta atagtaaaat atcatgctca agatgatatt   5940
   gatttcaaat tagacaatat aactatatta aaaacttatg tatgcttagg cagtaagtta   6000
   aagggatcgg aggtttactt agtccttaca ataggtcctg caaatatatt tccagtattt   6060
   aatgtagtac aaaatgctaa attgatacta tcaagaacca aaaatttcat catgcctaag   6120
   aaagctgata aagagtctat tgatgcaaat attaaaagtt tgataccctt tctttgttac   6180
   cctataacaa aaaaaggaat taatactgca ttgtcaaaac taaagagtgt tgttagtgga   6240
   gatatactat catattctat agctggacgg aatgaagttt tcagcaataa acttataaat   6300
   cataagcata tgaacatctt aaagtggttc aatcatgttt taaatttcag atcaacagaa   6360
   ctaaactata accatttata tatggtagaa tctacatatc cttacctaag tgaattgtta   6420
   aacagcttga caactaatga acttaaaaaa ctgattaaaa tcacaggtag tctgttatac   6480
   aactttcata atgaataa                                                 6498
<210> 44
   <211> 2165
   <212> PRT
   <213> Human respiratory syncytial virus
<400> 44
   Met Asp Pro Ile Ile Asn Gly Asn Ser Ala Asn Val Tyr Leu Thr Asp
   1               5                   10                  15
   Ser Tyr Leu Lys Gly Val Ile Ser Phe Ser Glu Cys Asn Ala Leu Gly
               20                  25                  30
   Ser Tyr Ile Phe Asn Gly Pro Tyr Leu Lys Asn Asp Tyr Thr Asn Leu
           35                  40                  45
   Ile Ser Arg Gln Asn Pro Leu Ile Glu His Met Asn Leu Lys Lys Leu
       50                  55                  60
   Asn Ile Thr Gln Ser Leu Ile Ser Lys Tyr His Lys Gly Glu Ile Lys
   65                  70                  75                  80
   Leu Glu Glu Pro Thr Tyr Phe Gln Ser Leu Leu Met Thr Tyr Lys Ser
                   85                  90                  95
   Met Thr Ser Leu Glu Gln Ile Ala Thr Thr Asn Leu Leu Lys Lys Ile
               100                 105                 110
   Ile Arg Arg Ala Ile Glu Ile Ser Asp Val Lys Val Tyr Ala Ile Leu
           115                 120                 125
   Asn Lys Leu Gly Leu Lys Glu Lys Asp Lys Ile Lys Ser Asn Asn Gly
   130 135 140
   Gln Asp Glu Asp Asn Ser Val Ile Thr Thr Ile Ile Lys Asp Asp Ile
   145 150 155 160
   Leu Ser Ala Val Lys Asp Asn Gln Ser His Leu Lys Ala Asp Lys Asn
   165 170 175
   His Ser Thr Lys Gln Lys Asp Thr Ile Lys Thr Thr Leu Leu Lys Lys
   180 185 190
   Leu Met Cys Ser Met Gln His Pro Pro Ser Trp Leu Ile His Trp Phe
   195 200 205
   Asn Leu Tyr Thr Lys Leu Asn Asn Ile Leu Thr Gln Tyr Arg Ser Asn
   210 215 220
   Glu Val Lys Asn His Gly Phe Ile Leu Ile Asp Asn Gln Thr Leu Ser
   225 230 235 240
   Gly Phe Gln Phe Ile Leu Asn Gln Tyr Gly Cys Ile Val Tyr His Lys
   245 250 255
   Glu Leu Lys Arg Ile Thr Val Thr Thr Tyr Asn Gln Phe Leu Thr Trp
   260 265 270
   Lys Asp Ile Ser Leu Ser Arg Leu Asn Val Cys Leu Ile Thr Trp Ile
   275 280 285
   Ser Asn Cys Leu Asn Thr Leu Asn Lys Ser Leu Gly Leu Arg Cys Gly
   290 295 300
   Phe Asn Asn Val Ile Leu Thr Gln Leu Phe Leu Tyr Gly Asp Cys Ile
   305 310 315 320
   Leu Lys Leu Phe His Asn Glu Gly Phe Tyr Ile Ile Lys Glu Val Glu
   325 330 335
   Gly Phe Ile Met Ser Leu Ile Leu Asn Ile Thr Glu Glu Asp Gln Phe
   340 345 350
   Arg Lys Arg Phe Tyr Asn Ser Met Leu Asn Asn Ile Thr Asp Ala Ala
   355 360 365
   Asn Lys Ala Gln Lys Asn Leu Leu Ser Arg Val Cys His Thr Leu Leu
   370 375 380
   Asp Lys Thr Val Ser Asp Asn Ile Ile Asn Gly Arg Trp Ile Ile Leu
   385 390 395 400
   Leu Ser Lys Phe Leu Lys Leu Ile Lys Leu Ala Gly Asp Asn Asn Leu
   405 410 415
   Asn Asn Leu Ser Glu Leu Tyr Phe Leu Phe Arg Ile Phe Gly His Pro
   420 425 430
   Met Val Asp Glu Arg Gln Ala Met Asp Ala Val Lys Val Asn Cys Asn
   435 440 445
   Glu Thr Lys Phe Tyr Leu Leu Ser Ser Leu Ser Met Leu Arg Gly Ala
   450 455 460
   Phe Ile Tyr Arg Ile Ile Lys Gly Phe Val Asn Asn Tyr Asn Arg Trp
   465 470 475 480
   Pro Thr Leu Arg Asn Ala Ile Val Leu Pro Leu Arg Trp Leu Thr Tyr
   485 490 495
   Tyr Lys Leu Asn Thr Tyr Pro Ser Leu Leu Glu Leu Thr Glu Arg Asp
   500 505 510
   Leu Ile Val Leu Ser Gly Leu Arg Phe Tyr Arg Glu Phe Arg Leu Pro
   515 520 525
   Lys Lys Val Asp Leu Glu Met Ile Ile Asn Asp Lys Ala Ile Ser Pro
   530 535 540
   Pro Lys Asn Leu Ile Trp Thr Ser Phe Pro Arg Asn Tyr Met Pro Ser
   545 550 555 560
   His Ile Gln Asn Tyr Ile Glu His Glu Lys Leu Lys Phe Ser Glu Ser
   565 570 575
   Asp Lys Ser Arg Arg Val Leu Glu Tyr Tyr Leu Arg Asp Asn Lys Phe
   580 585 590
   Asn Glu Cys Asp Leu Tyr Asn Cys Val Val Asn Gln Ser Tyr Leu Asn
   595 600 605
   Asn Pro Asn His Val Val Ser Leu Thr Gly Lys Glu Arg Glu Leu Ser
   610 615 620
   Val Gly Arg Met Phe Ala Met Gln Pro Gly Met Phe Arg Gln Val Gln
   625 630 635 640
   Ile Leu Ala Glu Lys Met Ile Ala Glu Asn Ile Leu Gln Phe Phe Pro
   645 650 655
   Glu Ser Leu Thr Arg Tyr Gly Asp Leu Glu Leu Gln Lys Ile Leu Glu
   660 665 670
   Leu Lys Ala Gly Ile Ser Asn Lys Ser Asn Arg Tyr Asn Asp Asn Tyr
   675 680 685
   Asn Asn Tyr Ile Ser Lys Cys Ser Ile Ile Thr Asp Leu Ser Lys Phe
   690 695 700
   Asn Gln Ala Phe Arg Tyr Glu Thr Ser Cys Ile Cys Ser Asp Val Leu
   705 710 715 720
   Asp Glu Leu His Gly Val Gln Ser Leu Phe Ser Trp Leu His Leu Thr
   725 730 735
   Ile Pro His Val Thr Ile Ile Cys Thr Tyr Arg His Ala Pro Pro Tyr
   740 745 750
   Ile Arg Asp His Ile Val Asp Leu Asn Asn Val Asp Glu Gln Ser Gly
   755 760 765
   Leu Tyr Arg Tyr His Met Gly Gly Ile Glu Gly Trp Cys Gln Lys Leu
   770 775 780
   Trp Thr Ile Glu Ala Ile Ser Leu Leu Asp Leu Ile Ser Leu Lys Gly
   785 790 795 800
   Lys Phe Ser Ile Thr Ala Leu Ile Asn Gly Asp Asn Gln Ser Ile Asp
   805 810 815
   Ile Ser Lys Pro Val Arg Leu Met Glu Gly Gln Thr His Ala Gln Ala
   820 825 830
   Asp Tyr Leu Leu Ala Leu Asn Ser Leu Lys Leu Leu Tyr Lys Glu Tyr
   835 840 845
   Ala Gly Ile Gly His Lys Leu Lys Gly Thr Glu Thr Tyr Ile Ser Arg
   850 855 860
   Asp Met Gln Phe Met Ser Lys Thr Ile Gln His Asn Gly Val Tyr Tyr
   865 870 875 880
   Pro Ala Ser Ile Lys Lys Val Leu Arg Val Gly Pro Trp Ile Asn Thr
   885 890 895
   Ile Leu Asp Asp Phe Lys Val Ser Leu Glu Ser Ile Gly Ser Leu Thr
   900 905 910
   Gln Glu Leu Glu Tyr Arg Gly Glu Ser Leu Leu Cys Ser Leu Ile Phe
   915 920 925
   Arg Asn Val Trp Leu Tyr Asn Gln Ile Ala Leu Gln Leu Lys Asn His
   930 935 940
   Ala Leu Cys Asn Asn Lys Leu Tyr Leu Asp Ile Leu Lys Val Leu Lys
   945 950 955 960
   His Leu Lys Thr Phe Phe Asn Leu Asp Asn Ile Asp Thr Ala Leu Thr
   965 970 975
   Leu Tyr Met Asn Leu Pro Met Leu Phe Gly Gly Gly Asp Pro Asn Leu
   980 985 990
   Leu Tyr Arg Ser Phe Tyr Arg Arg Thr Pro Asp Phe Leu Thr Glu Ala
   995 1000 1005
   Ile Val His Ser Val Phe Ile Leu Ser Tyr Tyr Thr Asn His Asp
   1010 1015 1020
   Leu Lys Asp Lys Leu Gln Asp Leu Ser Asp Asp Arg Leu Asn Lys
   1025 1030 1035
   Phe Leu Thr Cys Ile Ile Thr Phe Asp Lys Asn Pro Asn Ala Glu
   1040 1045 1050
   Phe Val Thr Leu Met Arg Asp Pro Gln Ala Leu Gly Ser Glu Arg
   1055 1060 1065
   Gln Ala Lys Ile Thr Ser Glu Ile Asn Arg Leu Ala Val Thr Glu
   1070 1075 1080
   Val Leu Ser Thr Ala Pro Asn Lys Ile Phe Ser Lys Ser Ala Gln
   1085 1090 1095
   His Tyr Thr Thr Thr Glu Ile Asp Leu Asn Asp Ile Met Gln Asn
   1100 1105 1110
   Ile Glu Pro Thr Tyr Pro His Gly Leu Arg Val Val Tyr Glu Ser
   1115 1120 1125
   Leu Pro Phe Tyr Lys Ala Glu Lys Ile Val Asn Leu Ile Ser Gly
   1130 1135 1140
   Thr Lys Ser Ile Thr Asn Ile Leu Glu Lys Thr Ser Ala Ile Asp
   1145 1150 1155
   Leu Thr Asp Ile Asp Arg Ala Thr Glu Met Met Arg Lys Asn Ile
   1160 1165 1170
   Thr Leu Leu Ile Arg Ile Leu Pro Leu Asp Cys Asn Arg Asp Lys
   1175 1180 1185
   Arg Glu Ile Leu Ser Met Glu Asn Leu Ser Ile Thr Glu Leu Ser
   1190 1195 1200
   Lys Tyr Val Arg Glu Arg Ser Trp Ser Leu Ser Asn Ile Val Gly
   1205 1210 1215
   Val Thr Ser Pro Ser Ile Met Tyr Thr Met Asp Ile Lys Tyr Thr
   1220 1225 1230
   Thr Ser Thr Ile Ala Ser Gly Ile Ile Ile Glu Lys Tyr Asn Val
   1235 1240 1245
   Asn Ser Leu Thr Arg Gly Glu Arg Gly Pro Thr Lys Pro Trp Val
   1250 1255 1260
   Gly Ser Ser Thr Gln Glu Lys Lys Thr Met Pro Val Tyr Asn Arg
   1265 1270 1275
   Gln Val Leu Thr Lys Lys Gln Arg Asp Gln Ile Asp Leu Leu Ala
   1280 1285 1290
   Lys Leu Asp Trp Val Tyr Ala Ser Ile Asp Asn Lys Asp Glu Phe
   1295 1300 1305
   Met Glu Glu Leu Ser Ile Gly Thr Leu Gly Leu Thr Tyr Glu Lys
   1310 1315 1320
   Ala Lys Lys Leu Phe Pro Gln Tyr Leu Ser Val Asn Tyr Leu His
   1325 1330 1335
   Arg Leu Thr Val Ser Ser Arg Pro Cys Glu Phe Pro Ala Ser Ile
   1340 1345 1350
   Pro Ala Tyr Arg Thr Thr Asn Tyr His Phe Asp Thr Ser Pro Ile
   1355 1360 1365
   Asn Arg Ile Leu Thr Glu Lys Tyr Gly Asp Glu Asp Ile Asp Ile
   1370 1375 1380
   Val Phe Gln Asn Cys Ile Ser Phe Gly Leu Ser Leu Met Ser Val
   1385 1390 1395
   Val Glu Gln Phe Thr Asn Val Cys Pro Asn Arg Ile Ile Leu Ile
   1400 1405 1410
   Pro Lys Leu Asn Glu Ile His Leu Met Lys Pro Pro Ile Phe Thr
   1415 1420 1425
   Gly Asp Val Asp Ile His Lys Leu Lys Gln Val Ile Gln Lys Gln
   1430 1435 1440
   His Met Phe Leu Pro Asp Lys Ile Ser Leu Thr Gln Tyr Val Glu
   1445 1450 1455
   Leu Phe Leu Ser Asn Lys Thr Leu Lys Ser Gly Ser His Val Asn
   1460 1465 1470
   Ser Asn Leu Ile Leu Ala His Lys Ile Ser Asp Tyr Phe His Asn
   1475 1480 1485
   Thr Tyr Ile Leu Ser Thr Asn Leu Ala Gly His Trp Ile Leu Ile
   1490 1495 1500
   Ile Gln Leu Met Lys Asp Ser Lys Gly Ile Phe Glu Lys Asp Trp
   1505 1510 1515
   Gly Glu Gly Tyr Ile Thr Asp His Met Phe Ile Asn Leu Lys Val
   1520 1525 1530
   Phe Phe Asn Ala Tyr Lys Thr Tyr Leu Leu Cys Phe His Lys Gly
   1535 1540 1545
   Tyr Gly Lys Ala Lys Leu Glu Cys Asp Met Asn Thr Ser Asp Leu
   1550 1555 1560
   Leu Cys Val Leu Glu Leu Ile Asp Ser Ser Tyr Trp Lys Ser Met
   1565 1570 1575
   Ser Lys Val Phe Leu Glu Gln Lys Val Ile Lys Tyr Ile Leu Ser
   1580 1585 1590
   Gln Asp Ala Ser Leu His Arg Val Lys Gly Cys His Ser Phe Lys
   1595 1600 1605
   Leu Trp Phe Leu Lys Arg Leu Asn Val Ala Glu Phe Thr Val Cys
   1610 1615 1620
   Pro Trp Val Val Asn Ile Asp Tyr His Pro Thr His Met Lys Ala
   1625 1630 1635
   Ile Leu Thr Tyr Ile Asp Leu Val Arg Met Gly Leu Ile Asn Ile
   1640 1645 1650
   Asp Arg Ile His Ile Lys Asn Lys His Lys Phe Asn Asp Glu Phe
   1655 1660 1665
   Tyr Thr Ser Asn Leu Phe Tyr Ile Asn Tyr Asn Phe Ser Asp Asn
   1670 1675 1680
   Thr His Leu Leu Thr Lys His Ile Arg Ile Ala Asn Ser Glu Leu
   1685 1690 1695
   Glu Asn Asn Tyr Asn Lys Leu Tyr His Pro Thr Pro Glu Thr Leu
   1700 1705 1710
   Glu Asn Ile Leu Ala Asn Pro Ile Lys Ser Asn Asp Lys Lys Thr
   1715 1720 1725
   Leu Asn Asp Tyr Cys Ile Gly Lys Asn Val Asp Ser Ile Met Leu
   1730 1735 1740
   Pro Leu Leu Ser Asn Lys Lys Leu Val Lys Ser Ser Ala Met Ile
   1745 1750 1755
   Arg Thr Asn Tyr Ser Lys Gln Asp Leu Tyr Asn Leu Phe Pro Thr
   1760 1765 1770
   Val Val Ile Asp Arg Ile Ile Asp His Ser Gly Asn Thr Ala Lys
   1775 1780 1785
   Ser Asn Gln Leu Tyr Thr Thr Thr Ser His Gln Ile Ser Leu Val
   1790 1795 1800
   His Asn Ser Thr Ser Leu Tyr Cys Met Leu Pro Trp His His Ile
   1805 1810 1815
   Asn Arg Phe Asn Phe Val Phe Ser Ser Thr Gly Cys Lys Ile Ser
   1820 1825 1830
   Ile Glu Tyr Ile Leu Lys Asp Leu Lys Ile Lys Asp Pro Asn Cys
   1835 1840 1845
   Ile Ala Phe Ile Gly Glu Gly Ala Gly Asn Leu Leu Leu Arg Thr
   1850 1855 1860
   Val Val Glu Leu His Pro Asp Ile Arg Tyr Ile Tyr Arg Ser Leu
   1865 1870 1875
   Lys Asp Cys Asn Asp His Ser Leu Pro Ile Glu Phe Leu Arg Leu
   1880 1885 1890
   Tyr Asn Gly His Ile Asn Ile Asp Tyr Gly Glu Asn Leu Thr Ile
   1895 1900 1905
   Pro Ala Thr Asp Ala Thr Asn Asn Ile His Trp Ser Tyr Leu His
   1910 1915 1920
   Ile Lys Phe Ala Glu Pro Ile Ser Leu Phe Val Cys Asp Ala Glu
   1925 1930 1935
   Leu Pro Val Thr Val Asn Trp Ser Lys Ile Ile Ile Glu Trp Ser
   1940 1945 1950
   Lys His Val Arg Lys Cys Lys Tyr Cys Ser Ser Val Asn Lys Cys
   1955 1960 1965
   Thr Leu Ile Val Lys Tyr His Ala Gln Asp Asp Ile Asp Phe Lys
   1970 1975 1980
   Leu Asp Asn Ile Thr Ile Leu Lys Thr Tyr Val Cys Leu Gly Ser
   1985 1990 1995
   Lys Leu Lys Gly Ser Glu Val Tyr Leu Val Leu Thr Ile Gly Pro
   2000 2005 2010
   Ala Asn Ile Phe Pro Val Phe Asn Val Val Gln Asn Ala Lys Leu
   2015 2020 2025
   Ile Leu Ser Arg Thr Lys Asn Phe Ile Met Pro Lys Lys Ala Asp
   2030 2035 2040
   Lys Glu Ser Ile Asp Ala Asn Ile Lys Ser Leu Ile Pro Phe Leu
   2045 2050 2055
   Cys Tyr Pro Ile Thr Lys Lys Gly Ile Asn Thr Ala Leu Ser Lys
   2060 2065 2070
   Leu Lys Ser Val Val Ser Gly Asp Ile Leu Ser Tyr Ser Ile Ala
   2075 2080 2085
   Gly Arg Asn Glu Val Phe Ser Asn Lys Leu Ile Asn His Lys His
   2090 2095 2100
   Met Asn Ile Leu Lys Trp Phe Asn His Val Leu Asn Phe Arg Ser
   2105 2110 2115
   Thr Glu Leu Asn Tyr Asn His Leu Tyr Met Val Glu Ser Thr Tyr
   2120 2125 2130
   Pro Tyr Leu Ser Glu Leu Leu Asn Ser Leu Thr Thr Asn Glu Leu
   2135 2140 2145
   Lys Lys Leu Ile Lys Ile Thr Gly Ser Leu Leu Tyr Asn Phe His
   2150 2155 2160
   Asn Glu
   2165
<210> 45
   <211> 909
   <212> DNA
   <213> Human respiratory syncytial virus
<400> 45
   aatgcaacca tgtccaaaca caagaatcaa cgcactgcca ggactctaga aaagacctgg 60
   gatactctta atcatctaat tgtaatatcc tcttgtttat acagattaaa tttaaaatct 120
   atagcacaaa tagcactatc agtattggca atgataatct caacctctct cataattgca 180
   gccataatat tcatcatctc tgccaatcac aaagttacac taacaacggt cacagtttca 240
   acaataaaaa accacactga aaaaaacatc accacttacc ttactcaagt ctcaccagaa 300
   agagttagcc catccaaaca acccacaacc acatcaccaa tccacacaaa ctcagccaca 360
   atatcaccca atacaaaatc agaaacacac catacaacag cgcaaaccaa aggcagaatc 420
   accactccaa cacagaccaa caagccaagc acaaaaccac gtccaaaaat tccaccaaaa 480
   aaagatgatt accattttga agtgttcaac ttcgttccct gtagtatatg tggcaacaat 540
   cgactttgca aatccatctg caaaacaata ccaagcaaca aaccaaagaa aaaaccaacc 600
   atcaaaccta caaacaaacc aactaccaaa accacaaaca aaatagaccc aaaaacacca 660
   gccaaaacac cgaaaaaaga aactaccacc aacccaacaa aaaaaccaac cctcaagatc 720
   acagaaaaag acaccagcac ttcacaatcc actatgctcg acacaaccaa accaaatcac 780
   acaatccaac agcaatacct ccactcaacc acccccgata acacacccaa ctccacacaa 840
   acacccacag catccgagcc ctccacatca aactcaaccc aagaagtcta gtcacatgct 900
   tagttattc 909
<210> 46
   <211> 293
   <212> PRT
   <213> Human respiratory syncytial virus
<400> 46
   Met Ser Lys His Lys Asn Gln Arg Thr Ala Arg Thr Leu Glu Lys Thr
   1               5                   10                  15
   Trp Asp Thr Leu Asn His Leu Ile Val Ile Ser Ser Cys Leu Tyr Arg
               20                  25                  30
   Leu Asn Leu Lys Ser Ile Ala Gln Ile Ala Leu Ser Val Leu Ala Met
           35                  40                  45
   Ile Ile Ser Thr Ser Leu Ile Ile Ala Ala Ile Ile Phe Ile Ile Ser
       50                  55                  60
   Ala Asn His Lys Val Thr Leu Thr Thr Val Thr Val Ser Thr Ile Lys
   65                  70                  75                  80
   Asn His Thr Glu Lys Asn Ile Thr Thr Tyr Leu Thr Gln Val Ser Pro
                   85                  90                  95
   Glu Arg Val Ser Pro Ser Lys Gln Pro Thr Thr Thr Ser Pro Ile His
               100                 105                 110
   Thr Asn Ser Ala Thr Ile Ser Pro Asn Thr Lys Ser Glu Thr His His
           115                 120                 125
   Thr Thr Ala Gln Thr Lys Gly Arg Ile Thr Thr Pro Thr Gln Thr Asn
       130                 135                 140
   Lys Pro Ser Thr Lys Pro Arg Pro Lys Ile Pro Pro Lys Lys Asp Asp
   145 150 155 160
   Tyr His Phe Glu Val Phe Asn Phe Val Pro Cys Ser Ile Cys Gly Asn
   165 170 175
   Asn Arg Leu Cys Lys Ser Ile Cys Lys Thr Ile Pro Ser Asn Lys Pro
   180 185 190
   Lys Lys Lys Pro Thr Ile Lys Pro Thr Asn Lys Pro Thr Thr Lys Thr
   195 200 205
   Thr Asn Lys Ile Asp Pro Lys Thr Pro Ala Lys Thr Pro Lys Lys Glu
   210 215 220
   Thr Thr Thr Asn Pro Thr Lys Lys Pro Thr Leu Lys Ile Thr Glu Lys
   225 230 235 240
   Asp Thr Ser Thr Ser Gln Ser Thr Met Leu Asp Thr Thr Lys Pro Asn
   245 250 255
   His Thr Ile Gln Gln Gln Tyr Leu His Ser Thr Thr Pro Asp Asn Thr
   260 265 270
   Pro Asn Ser Thr Gln Thr Pro Thr Ala Ser Glu Pro Ser Thr Ser Asn
   275 280 285
   Ser Thr Gln Glu Val
   290
<210> 47
   <211> 975
   <212> DNA
   <213> Human respiratory syncytial virus
<400> 47
   aatgcaacca tgtccaaaca caagaatcaa cgcactgcca ggactctaga aaagacctgg 60
   gatactctta atcatctaat tgtaatatcc tcttgtttat acaaattaaa tttaaaatct 120
   atagcacaaa tagcactatc agttttggca atgataatct caacctctct cataattgca 180
   gccataatat tcatcatctc tgccaatcac aaagttacac taacaactgt cacagttcaa 240
   acaataaaaa accacactga gaaaaacatc accacttacc ttactcaagt ctcaccagaa 300
   agggttagcc catccaaaca acccacaacc acaccaccaa tccacacaaa ctcagccaca 360
   atatcaccta atacaagatc agaaacacac catacaacag cacaaaccaa aggcagaacc 420
   accactccga cacagaacaa caagccaagc acaaaaccac gtccaaaaaa tccaccaaaa 480
   aaaccaaaag atgattacca ttttgaagtg ttcaacttcg ttccctgtag tatatgtggc 540
   aacaatcaac tctgcaaatc catttgcaaa acaataccaa gcaataaacc aaagaaaaaa 600
   ccaaccataa aacccacaaa caaaccaccc accaaaacca caaccaaaag agacccaaaa 660
   acactagcca aaacactgaa aaaagaaacc accatcaacc caacaaaaaa accaaccccc 720
   aagaccacag aaagagacac cagtacccca caatccactg tgctcgacac aaccacatca 780
   aaacacacag gaagagacac cagcacctca caatccattg tgctcgacac aaccacatca 840
   aaacacacaa tccaacagca atccctctac tcaaccaccc ccgaaaacac acccaactcc 900
   acacaaacgc ccacagcatc cgagccctcc acatcaaatt ccacccaaaa actctagtca 960
   catgcttagt tattc 975
<210> 48
   <211> 315
   <212> PRT
   <213> Human respiratory syncytial virus
<400> 48
   Met Ser Lys His Lys Asn Gln Arg Thr Ala Arg Thr Leu Glu Lys Thr
   1 5 10 15
   Trp Asp Thr Leu Asn His Leu Ile Val Ile Ser Ser Cys Leu Tyr Lys
   20 25 30
   Leu Asn Leu Lys Ser Ile Ala Gln Ile Ala Leu Ser Val Leu Ala Met
   35 40 45
   Ile Ile Ser Thr Ser Leu Ile Ile Ala Ala Ile Ile Phe Ile Ile Ser
   50 55 60
   Ala Asn His Lys Val Thr Leu Thr Thr Val Thr Val Gln Thr Ile Lys
   65 70 75 80
   Asn His Thr Glu Lys Asn Ile Thr Thr Tyr Leu Thr Gln Val Ser Pro
   85 90 95
   Glu Arg Val Ser Pro Ser Lys Gln Pro Thr Thr Thr Pro Pro Ile His
   100 105 110
   Thr Asn Ser Ala Thr Ile Ser Pro Asn Thr Arg Ser Glu Thr His His
   115 120 125
   Thr Thr Ala Gln Thr Lys Gly Arg Thr Thr Thr Pro Thr Gln Asn Asn
   130 135 140
   Lys Pro Ser Thr Lys Pro Arg Pro Lys Asn Pro Pro Lys Lys Pro Lys
   145 150 155 160
   Asp Asp Tyr His Phe Glu Val Phe Asn Phe Val Pro Cys Ser Ile Cys
   165 170 175
   Gly Asn Asn Gln Leu Cys Lys Ser Ile Cys Lys Thr Ile Pro Ser Asn
   180 185 190
   Lys Pro Lys Lys Lys Pro Thr Ile Lys Pro Thr Asn Lys Pro Pro Thr
   195 200 205
   Lys Thr Thr Thr Lys Arg Asp Pro Lys Thr Leu Ala Lys Thr Leu Lys
   210 215 220
   Lys Glu Thr Thr Ile Asn Pro Thr Lys Lys Pro Thr Pro Lys Thr Thr
   225 230 235 240
   Glu Arg Asp Thr Ser Thr Pro Gln Ser Thr Val Leu Asp Thr Thr Thr
   245 250 255
   Ser Lys His Thr Gly Arg Asp Thr Ser Thr Ser Gln Ser Ile Val Leu
   260 265 270
   Asp Thr Thr Thr Ser Lys His Thr Ile Gln Gln Gln Ser Leu Tyr Ser
   275 280 285
   Thr Thr Pro Glu Asn Thr Pro Asn Ser Thr Gln Thr Pro Thr Ala Ser
   290 295 300
   Glu Pro Ser Thr Ser Asn Ser Thr Gln Lys Leu
   305 310 315
<210> 49
   <211> 909
   <212> DNA
   <213> Human respiratory syncytial virus
<400> 49
   aatgcaacca tgtccaaaca caagaatcaa cgcactgcca ggactctaga aaagacctgg 60
   gatactctta atcatctaat tgtaatatcc tcttgtttat acaggttaaa tttaaaatct 120
   atagcacaaa tagcactatc agtattggca atgataatct caacctctct cataattgca 180
   gccataatat tcatcatctc tgccaatcac aaagttacac taacaacggt cacagtttca 240
   acaataaaaa gccacactga aaaaaacatc accacttacc ttactcaagt ctcaccagaa 300
   agggttagcc catccaaaca acccacaacc acatcaccaa tccacacaaa ctcagccaca 360
   atatcaccca atacaaaatc agaaacacac catacaacag cacaaaccaa aggcagattc 420
   accactccaa cacagaccaa caagccaagc acaaaaccac gtccaaaaat tccaccaaaa 480
   aaagatgatt accattttga agtgttcaac ttcgttccct gtagtatatg tggcaacaat 540
   cgactttgca aatccatctg caaaacaata ccaagcaaca aaccaaagaa aaaaccaacc 600
   atcaaaccta caaacaaacc aaccaccaaa accacaaaca aaatagaccc aaaaacacca 660
   gccaaaacac cggaaaaaga aactaccacc aactcaacaa aaaaaccaac cctcaagatc 720
   acagaaaaag acaccagcac ctcacaatcc actatgctcg acacaaccac accaaatcac 780
   acaatccaac agcaatccct ccactcaacc acccccgata acacacccaa ctccacacaa 840
   acacccacag catccgagcc ctccacatca aactcaaccc aaaaagtcta gtcacatgct 900
   tagttattc 909
<210> 50
   <211> 293
   <212> PRT
   <213> Human respiratory syncytial virus
<400> 50
   Met Ser Lys His Lys Asn Gln Arg Thr Ala Arg Thr Leu Glu Lys Thr
   1 5 10 15
   Trp Asp Thr Leu Asn His Leu Ile Val Ile Ser Ser Cys Leu Tyr Arg
   20 25 30
   Leu Asn Leu Lys Ser Ile Ala Gln Ile Ala Leu Ser Val Leu Ala Met
   35 40 45
   Ile Ile Ser Thr Ser Leu Ile Ile Ala Ala Ile Ile Phe Ile Ile Ser
   50 55 60
   Ala Asn His Lys Val Thr Leu Thr Thr Val Thr Val Ser Thr Ile Lys
   65 70 75 80
   Ser His Thr Glu Lys Asn Ile Thr Thr Tyr Leu Thr Gln Val Ser Pro
   85 90 95
   Glu Arg Val Ser Pro Ser Lys Gln Pro Thr Thr Thr Ser Pro Ile His
   100 105 110
   Thr Asn Ser Ala Thr Ile Ser Pro Asn Thr Lys Ser Glu Thr His His
   115 120 125
   Thr Thr Ala Gln Thr Lys Gly Arg Phe Thr Thr Pro Thr Gln Thr Asn
   130 135 140
   Lys Pro Ser Thr Lys Pro Arg Pro Lys Ile Pro Pro Lys Lys Asp Asp
   145 150 155 160
   Tyr His Phe Glu Val Phe Asn Phe Val Pro Cys Ser Ile Cys Gly Asn
   165 170 175
   Asn Arg Leu Cys Lys Ser Ile Cys Lys Thr Ile Pro Ser Asn Lys Pro
   180 185 190
   Lys Lys Lys Pro Thr Ile Lys Pro Thr Asn Lys Pro Thr Thr Lys Thr
   195 200 205
   Thr Asn Lys Ile Asp Pro Lys Thr Pro Ala Lys Thr Pro Glu Lys Glu
   210 215 220
   Thr Thr Thr Asn Ser Thr Lys Lys Pro Thr Leu Lys Ile Thr Glu Lys
   225 230 235 240
   Asp Thr Ser Thr Ser Gln Ser Thr Met Leu Asp Thr Thr Thr Pro Asn
   245 250 255
   His Thr Ile Gln Gln Gln Ser Leu His Ser Thr Thr Pro Asp Asn Thr
   260 265 270
   Pro Asn Ser Thr Gln Thr Pro Thr Ala Ser Glu Pro Ser Thr Ser Asn
   275 280 285
   Ser Thr Gln Lys Val
   290
<210> 51
   <211> 915
   <212> DNA
   <213> Human respiratory syncytial virus
<400> 51
   aatgcaacca tgtccaaaca caagaatcaa cgcactgcca ggactctaga aaagacctgg 60
   gatactctta atcatctaat tgtaatatcc tcttgtttat acaaattaaa tttaaaatct 120
   atagcacaaa tagcactatc agttttggca atgataatct caacctctct cataattgca 180
   gccataatat tcatcatctc tgccaatcac aaagttacac taacaacggt cacagttcaa 240
   acaataaaaa accacactga aaaaaacatc acctcttacc ttactcaagt ctcaccagaa 300
   agggccagcc catccaaaca acccacaacc acaccaccaa tccacacaaa ctcagccaca 360
   acatcaccca acacaaaatc agaaacacac catacaacag cacaaaccaa aggcagaacc 420
   accactccaa cacagaacaa caagccaagc acaaaaccac gtccaaaaaa tccaccaaaa 480
   aaaccaaaag atgattacca ttttgaagtg ttcaacttcg ttccctgtag tatatgtggc 540
   aacaatcaac tttgcagatc catctgcaaa acaataccaa gcaataaacc aaagaaaaaa 600
   ccaaccatca aacccacaaa caaaccaccc accaaaacca caaacaaaag agacccaaaa 660
   acaccagcaa aaccactgaa aaaagaaacc accaccaacc caacaaaaaa accaaccccc 720
   aagaccacag aaagagactc cagcacttca caatccactg tgctcgacac aaccacatca 780
   aaacacacaa tccaacagca atccctccac tcaaccaccc ccgaaaacac acccaactcc 840
   acacaaacac ccacagcatc cgagccctcc acatcaaatt ccacccaaga accctagtca 900
   catgcttagt tattc 915
<210> 52
   <211> 295
   <212> PRT
   <213> Human respiratory syncytial virus
<400> 52
   Met Ser Lys His Lys Asn Gln Arg Thr Ala Arg Thr Leu Glu Lys Thr
   1               5                   10                  15
   Trp Asp Thr Leu Asn His Leu Ile Val Ile Ser Ser Cys Leu Tyr Lys
   20 25 30
   Leu Asn Leu Lys Ser Ile Ala Gln Ile Ala Leu Ser Val Leu Ala Met
   35 40 45
   Ile Ile Ser Thr Ser Leu Ile Ile Ala Ala Ile Ile Phe Ile Ile Ser
   50 55 60
   Ala Asn His Lys Val Thr Leu Thr Thr Val Thr Val Gln Thr Ile Lys
   65 70 75 80
   Asn His Thr Glu Lys Asn Ile Thr Ser Tyr Leu Thr Gln Val Ser Pro
   85 90 95
   Glu Arg Ala Ser Pro Ser Lys Gln Pro Thr Thr Thr Pro Pro Ile His
   100 105 110
   Thr Asn Ser Ala Thr Thr Ser Pro Asn Thr Lys Ser Glu Thr His His
   115 120 125
   Thr Thr Ala Gln Thr Lys Gly Arg Thr Thr Thr Pro Thr Gln Asn Asn
   130 135 140
   Lys Pro Ser Thr Lys Pro Arg Pro Lys Asn Pro Pro Lys Lys Pro Lys
   145 150 155 160
   Asp Asp Tyr His Phe Glu Val Phe Asn Phe Val Pro Cys Ser Ile Cys
   165 170 175
   Gly Asn Asn Gln Leu Cys Arg Ser Ile Cys Lys Thr Ile Pro Ser Asn
   180 185 190
   Lys Pro Lys Lys Lys Pro Thr Ile Lys Pro Thr Asn Lys Pro Pro Thr
   195 200 205
   Lys Thr Thr Asn Lys Arg Asp Pro Lys Thr Pro Ala Lys Pro Leu Lys
   210 215 220
   Lys Glu Thr Thr Thr Asn Pro Thr Lys Lys Pro Thr Pro Lys Thr Thr
   225 230 235 240
   Glu Arg Asp Ser Ser Thr Ser Gln Ser Thr Val Leu Asp Thr Thr Thr
   245 250 255
   Ser Lys His Thr Ile Gln Gln Gln Ser Leu His Ser Thr Thr Pro Glu
   260 265 270
   Asn Thr Pro Asn Ser Thr Gln Thr Pro Thr Ala Ser Glu Pro Ser Thr
   275 280 285
   Ser Asn Ser Thr Gln Glu Pro
   290 295
<210> 53
   <211> 1725
   <212> DNA
   <213> Human respiratory syncytial virus
<400> 53
   atggagttgc taatcctcaa agcaaatgca attaccacaa tcctcactgc agtcacattt      60
   tgttttgctt ctggtcaaaa catcactgaa gaattttatc aatcaacatg cagtgcagtt     120
   agcaaaggct atcttagtgc tctgagaact ggttggtata ccagtgttat aactatagaa     180
   ttaagtaata tcaaggaaaa taagtgtaat ggaacagatg ctaaggtaaa attgataaaa     240
   caagaattag ataaatataa aaatgctgta acagaattgc agttgctcat gcaaagcaca     300
   ccaccaacaa acaatcgagc cagaagagaa ctaccaaggt ttatgaatta tacactcaac     360
   aatgccaaaa aaaccaatgt aacattaagc aagaaaagga aaagaagatt tcttggtttt     420
   ttgttaggtg ttggatctgc aatcgccagt ggcgttgctg tatctaaggt cctgcaccta     480
   gaaggggaag tgaacaagat caaaagtgct ctactatcca caaacaaggc tgtagtcagc     540
   ttatcaaatg gagttagtgt cttaaccagc aaagtgttag acctcaaaaa ctatatagat     600
   aaacaattgt tacctattgt gaacaagcaa agctgcagca tatcaaatat agaaactgtg     660
   atagagttcc aacaaaagaa caacagacta ctagagatta ccagggaatt tagtgttaat     720
   gcaggtgtaa ctacacctgt aagcacttac atgttaacta atagtgaatt attgtcatta     780
   atcaatgata tgcctataac aaatgatcag aaaaagttaa tgtccaacaa tgttcaaata     840
   gttagacagc aaagttactc tatcatgtcc ataataaaag aggaagtctt agcatatgta     900
   gtacaattac cactatatgg tgttatagat acaccctgtt ggaaactaca cacatcccct     960
   ctatgtacaa ccaacacaaa agaagggtcc aacatctgtt taacaagaac tgacagagga    1020
   tggtactgtg acaatgcagg atcagtatct ttcttcccac aagctgaaac atgtaaagtt    1080
   caatcaaatc gagtattttg tgacacaatg aacagtttaa cattaccaag tgaaataaat    1140
   ctctgcaatg ttgacatatt caaccccaaa tatgattgta aaattatgac ttcaaaaaca    1200
   gatgtaagca gctccgttat cacatctcta ggagccattg tgtcatgcta tggcaaaact    1260
   aaatgtacag catccaataa aaatcgtgga atcataaaga cattttctaa cgggtgcgat    1320
   tatgtatcaa ataaagggat ggacactgtg tctgtaggta acacattata ttatgtaaat    1380
   aagcaagaag gtaaaagtct ctatgtaaaa ggtgaaccaa taataaattt ctatgaccca    1440
   ttagtattcc cctctgatga atttgatgca tcaatatctc aagtcaacga gaagattaac    1500
   cagagcctag catttattcg taaatccgat gaattattac ataatgtaaa tgctggtaaa    1560
   tccaccacaa atatcatgat aactactata attatagtga ttatagtaat attgttatca    1620
   ttaattgctg ttggactgct cttatactgt aaggccagaa gcacaccagt cacactaagc    1680
   aaagatcaac tgagtggtat aaataatatt gcatttagta actaa                    1725
<210> 54
   <211> 574
   <212> PRT
   <213> Human respiratory syncytial virus
<400> 54
   Met Glu Leu Leu Ile Leu Lys Ala Asn Ala Ile Thr Thr Ile Leu Thr
   1               5                   10                  15
   Ala Val Thr Phe Cys Phe Ala Ser Gly Gln Asn Ile Thr Glu Glu Phe
               20                  25                  30
   Tyr Gln Ser Thr Cys Ser Ala Val Ser Lys Gly Tyr Leu Ser Ala Leu
           35                  40                  45
   Arg Thr Gly Trp Tyr Thr Ser Val Ile Thr Ile Glu Leu Ser Asn Ile
       50                  55                  60
   Lys Glu Asn Lys Cys Asn Gly Thr Asp Ala Lys Val Lys Leu Ile Lys
   65                  70                  75                  80
   Gln Glu Leu Asp Lys Tyr Lys Asn Ala Val Thr Glu Leu Gln Leu Leu
                   85                  90                  95
   Met Gln Ser Thr Pro Pro Thr Asn Asn Arg Ala Arg Arg Glu Leu Pro
               100                 105                 110
   Arg Phe Met Asn Tyr Thr Leu Asn Asn Ala Lys Lys Thr Asn Val Thr
           115                 120                 125
   Leu Ser Lys Lys Arg Lys Arg Arg Phe Leu Gly Phe Leu Leu Gly Val
       130                 135                 140
   Gly Ser Ala Ile Ala Ser Gly Val Ala Val Ser Lys Val Leu His Leu
   145                 150                 155                 160
   Glu Gly Glu Val Asn Lys Ile Lys Ser Ala Leu Leu Ser Thr Asn Lys
                   165                 170                 175
   Ala Val Val Ser Leu Ser Asn Gly Val Ser Val Leu Thr Ser Lys Val
               180                 185                 190
   Leu Asp Leu Lys Asn Tyr Ile Asp Lys Gln Leu Leu Pro Ile Val Asn
           195                 200                 205
   Lys Gln Ser Cys Ser Ile Ser Asn Ile Glu Thr Val Ile Glu Phe Gln
       210                 215                 220
   Gln Lys Asn Asn Arg Leu Leu Glu Ile Thr Arg Glu Phe Ser Val Asn
   225                 230                 235                 240
   Ala Gly Val Thr Thr Pro Val Ser Thr Tyr Met Leu Thr Asn Ser Glu
   245 250 255
   Leu Leu Ser Leu Ile Asn Asp Met Pro Ile Thr Asn Asp Gln Lys Lys
   260 265 270
   Leu Met Ser Asn Asn Val Gln Ile Val Arg Gln Gln Ser Tyr Ser Ile
   275 280 285
   Met Ser Ile Ile Lys Glu Glu Val Leu Ala Tyr Val Val Gln Leu Pro
   290 295 300
   Leu Tyr Gly Val Ile Asp Thr Pro Cys Trp Lys Leu His Thr Ser Pro
   305 310 315 320
   Leu Cys Thr Thr Asn Thr Lys Glu Gly Ser Asn Ile Cys Leu Thr Arg
   325 330 335
   Thr Asp Arg Gly Trp Tyr Cys Asp Asn Ala Gly Ser Val Ser Phe Phe
   340 345 350
   Pro Gln Ala Glu Thr Cys Lys Val Gln Ser Asn Arg Val Phe Cys Asp
   355 360 365
   Thr Met Asn Ser Leu Thr Leu Pro Ser Glu Ile Asn Leu Cys Asn Val
   370 375 380
   Asp Ile Phe Asn Pro Lys Tyr Asp Cys Lys Ile Met Thr Ser Lys Thr
   385 390 395 400
   Asp Val Ser Ser Ser Val Ile Thr Ser Leu Gly Ala Ile Val Ser Cys
   405 410 415
   Tyr Gly Lys Thr Lys Cys Thr Ala Ser Asn Lys Asn Arg Gly Ile Ile
   420 425 430
   Lys Thr Phe Ser Asn Gly Cys Asp Tyr Val Ser Asn Lys Gly Met Asp
   435 440 445
   Thr Val Ser Val Gly Asn Thr Leu Tyr Tyr Val Asn Lys Gln Glu Gly
   450 455 460
   Lys Ser Leu Tyr Val Lys Gly Glu Pro Ile Ile Asn Phe Tyr Asp Pro
   465 470 475 480
   Leu Val Phe Pro Ser Asp Glu Phe Asp Ala Ser Ile Ser Gln Val Asn
   485 490 495
   Glu Lys Ile Asn Gln Ser Leu Ala Phe Ile Arg Lys Ser Asp Glu Leu
   500 505 510
   Leu His Asn Val Asn Ala Gly Lys Ser Thr Thr Asn Ile Met Ile Thr
           515                 520                 525
   Thr Ile Ile Ile Val Ile Ile Val Ile Leu Leu Ser Leu Ile Ala Val
       530                 535                 540
   Gly Leu Leu Leu Tyr Cys Lys Ala Arg Ser Thr Pro Val Thr Leu Ser
   545                 550                 555                 560
   Lys Asp Gln Leu Ser Gly Ile Asn Asn Ile Ala Phe Ser Asn
                   565                 570

## Description

### SEQUENCE LISTING

The instant application contains a Sequence Listing which has been submitted in ASCII format. Said ASCII copy, created on August 2, 2012, is named "030276-9016_SequenceListing_ST25.txt" and is 116,697 bytes in size.

### FIELD OF THE INVENTION

The present invention relates to treating and preventing respiratory syncytial virus (RSV) infection using a vaccine containing an RSV antigenic peptide and DNA encoding the RSV antigenic peptide.

### BACKGROUND

Human RSV is a *Pneumovirus* of the family *Paramyxoviridae,* and a major cause of lower respiratory tract infections amongst children and the elderly, but most commonly in infants less than three years of -age. Conservative estimates suggest approximately 3.3 million cases of respiratory tract disease annually in the elderly in the USA today. Like influenza A disease, RSV epidemics occur every winter, and re-infection with RSV is very common, recurring throughout life. Although the development of a vaccine against RSV is a high priority, no safe and effective vaccine against RSV is available due to the associated vaccine-induced disease (VID). VID is caused by the robust pathogenic inflammation reaction in subjects due to an enhanced inflammatory CD4⁺ T cell responsiveness from RSV antigens.

A safer and effective RSV vaccine should be harmless and induce the right immune responses against the virus. However, the first candidate vaccine, a formalin-inactivated RSV (FI-RSV) vaccine developed in the 1960s induced severe disease following subsequent natural exposure to the virus rather than serving as a vaccine against infection. It resulted in the hospitalization of 80% of the vaccinated infants and two deaths. Peripheral blood lymphocytes from these children showed T lymphocyte hyper-responses compared to naive or RSV infected controls.

A similar pathogenesis of VID was revealed in animal models that were FI-RSV immunized and then challenged with live RSV. The enhanced histopathologic changes of VID in mice could be abrogated by depletion of CD4⁺ T cells or IL-4 before RSV challenge, further suggesting that the FI-RSV-induced pathologic changes were T cell mediated in this animal model.

F and G proteins of RSV serve as significant neutralizing and major protective antigens, but also result in VID. In particular, it has been found that mice immunized with respiratory syncytial virus (RSV) G glycoprotein (G) exhibit VID following RSV challenge and experienced atypical pulmonary eosinophilia. The deletion of CD4⁺ T cells resulted in less severe disease, suggesting that sequences within the G antigen might contain epitopes that over-stimulate T cell responses.

Although a RSV vaccine is needed where VID occurs upon RSV challenge, a RSV vaccine has not been generated that avoids over-reactive responses such as VID, which further leads to lung pathology damage. An ideal vaccine against RSV infection should meet two requirements: 1) to induce RSV specific neutralizing antibodies; and 2) to not stimulate the excessive T cell responses that cause VID. Many approaches have been tested, such as the use of DNA vaccine, adenoviral vector, Th1 type of adjuvant, and oral delivery, but none have been successful so far. Accordingly, there is a need in the art to develop a vaccine that will induce a neutralizing antibody against RSV infection, but suppress inflammatory CD4⁺ T cells.

Lindell et al., 2011 discloses RSV vaccines that do not trigger vaccine induced disease. WO2009/105152 discloses recombinant virus like particles, including a virus like particle comprising a RSV ectodomain. WO2010/039224 discloses RSV G proteins for use in a vaccine.

### SUMMARY OF THE INVENTION

The subject matter for which protection is sought is as defined in the claims.

In particular, the invention provides a matched antigenic peptide and DNA vaccine against respiratory syncytial virus (RSV) infection comprising an RSV antigenic peptide and a nucleic acid encoding the RSV antigenic peptide, wherein the nucleic acid is DNA and the DNA encodes for the RSV antigenic peptide of SEQ ID NO:4, and wherein the vaccine stimulates iTreg cells (CD4⁺, CD25⁻, FoxP3⁺, IL-10).

The present invention is directed to a vaccine against respiratory syncytial virus (RSV) infection comprising an RSV antigenic peptide and a nucleic acid encoding the RSV antigenic peptide wherein the vaccine stimulates iTreg cells (CD4⁺, CD25⁻, FoxP3⁺, IL-10⁺). The RSV antigenic peptide of the vaccine is SEQ ID NO:4.. The vaccine can have a nucleic acid and antigenic peptide mass ratio from 5:1 and 1:5; and 1:1 and 2:1. The vaccine of the invention is DNA. The nucleic acid encodes for the RSV antigenic peptide SEQ ID NO: 4 (optimized amino acid RSV G amino acid sequence). The DNA can be present in a linear expression cassette of a circular plasmid. The plasmid can be selected from the group consisting of pVAX, pcDNA3.0, and proVAX. The plasmid can further comprise a promoter selected from the group consisting of CMV, SV40 early promoter, SV40 later promoter, metallothionein promoter, murine mammary tumor virus promoter, Rous sarcoma virus promoter, and polyhedrin promoter.

The present invention can further be directed to a vaccination kit comprising a vaccine administration device and the vaccine as described above. The vaccine administration device of the kit can be a vaccine gun, or an electroporation device. The kit can comprise minimally-invasive electroporation device.

The present invention can also be directed to a vaccine as described herein for use in a method for preventing or treating respiratory syncytial virus infection in a patient. The method can further protect the subject from airway hyper-responsiveness (AHR) after respiratory syncytial virus challenge.

The present invention is further directed to a vaccine as described herein for use in a method of inducing neutralizing antibody against respiratory syncytial virus infection and suppressing inflammatory T cells, wherein iTreg cells are induced and auto-reactive CD4+ and CD8+ T cells are suppressed. The vaccine can be for administration by electroporation using, for example, a minimally-invasive electroporation device, wherein the electroporation route is selected from the group consisting of intradermal and intramuscular.

The present invention is further directed to a vaccine as described herein for use in a method of treating or preventing vaccine-induced disease in a subject immunized against respiratory syncytial virus (RSV), wherein the subject has been immunized with formalin-inactivated RSV (FI-RSV) or RSV antigen prior to encounter with natural RSV infection. The vaccine can be for administration by electroporation using, for example, a minimally-invasive electroporation device, wherein the electroporation route can be intradermal or intramuscular.

### BRIEF DESCRIPTION OF THE DRAWINGS

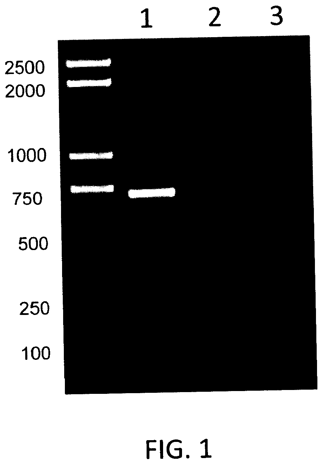

FIG. 1 shows the eukaryotic expression of the plasmids proVAX/G in NIH/3T3 cells. Total RNA was extracted from NIH/3T3 cells 48 h after transfection with proVAX/G. The templates used for RT-PCR were as follows: lane1, cDNA from the transfected NIH/3T3 cells with RTs; lane 2, proVAX vector control transfected cells; lane 3, RNA from non-transfected NIH/3T3 cells as a negative control.

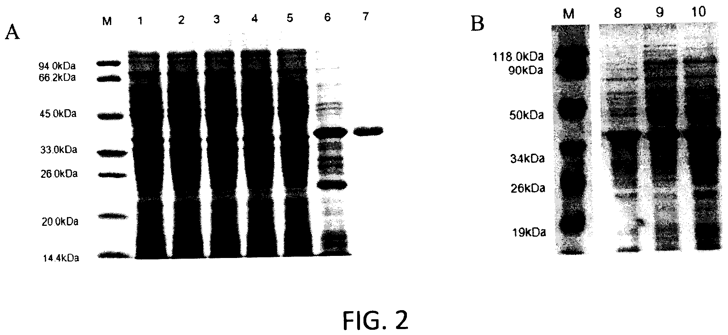

FIGS. 2A-2B show the Coomassie blue stains of SDS-PAGE of recombinant protein expression in *E. coli* BL21(DE3). FIGS. 2A-2B show in lane M, protein molecular markers; lane 1, *E. coli* BL21(DE3) not induced; lane 2, *E. coli* BL21(DE3) induced by IPTG under 0.5 mM; lane 3 *E. coli* BL21(DE3) pET28a(+) not induced; lane 4, *E. coli* BL21(DE3) pET28a induced by IPTG under 0.5 mM, lane 5, *E. coli* BL21(DE3) pET28a(+)/G not induced; lanes 6&10, *E. coli* BL21(DE3) pET28a(+)/G induced by IPTG under 0.5mM; lane 7, purified polyhistidine-tagged recombinant protein; lane 8, supernatants of *E. coli* BL21(DE3) lysate transformed by pET28a(+)/G with 0.5 mM IPTG induction; and lane 9, sediment of *E. coli* BL21(DE3) lysate transformed by pET28a(+)/G with 0.5 mM IPTG induction.

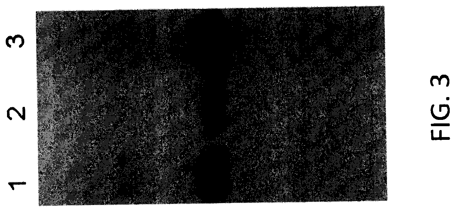

FIG. 3 shows the western blot analysis of polyhistidine-tagged recombinant G protein. *E. coli* BL21(DE3) lysates obtained from cells grown in LB broth and induced with 0.5 mM IPTG. Protein samples were separated on 12% SDS-PAGE and transferred to a nitrocellulose membrane. The membrane was reacted with goat anti-RSV antibodies and visualized by reacting conjugated secondary anti-goat antibodies. Lane 1, Supernatants of *E. coli* BL21(DE3) lysate transformed by pET28a(+)/G with 0.5mM IPTG induced; lane 2, insoluble fraction of *E. coli* BL21(DE3) lysate transformed by pET28a(+)/G; lane 3, nickel column purified recombinant RSV G protein.

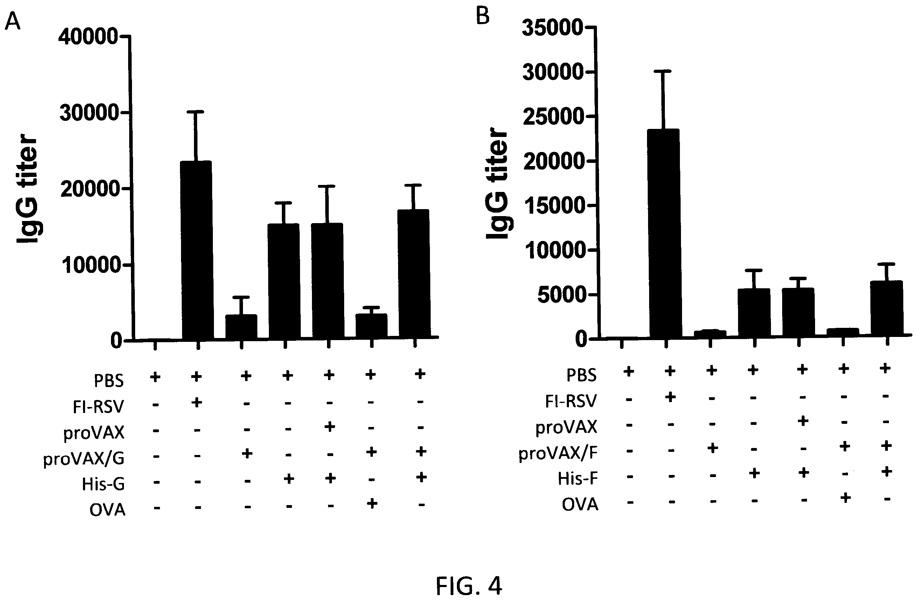

FIGS. 4A-4B show analysis of the humoral response. Serum samples were collected from Balb/c mice on day 7 after the last immunization. RSV G specific antibody (FIG. 4A) and RSV F specific antibody (FIG. 4B) were determined by ELISA using UV-inactivated RSV. Results are presented as an average of data from three groups in triplicate, error bars represent standard deviation. The data shown summarizes one of three experiments, all of which demonstrated similar results. The single asterisk indicates *p* < 0.05, double asterisk indicates *p* < 0.01.

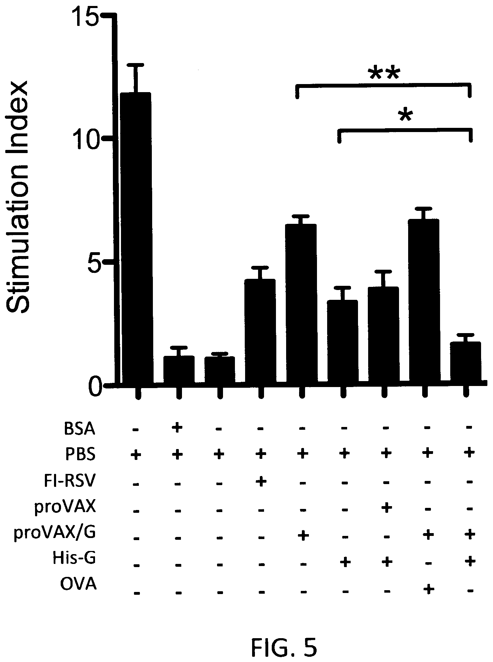

FIG. 5 shows that T cell response impairment is induced by co-immunization of DNA and protein vaccines. T cells were isolated from mice that had been immunized with PSB or proVAX/G or His-G or co-immunized with proVAX + His-G (antigen-mismatched) or proVAX/G + OVA (antigen-mismatched) or co-immunized with proVAX/G + His-G (antigen-matched). The T cells were re-stimulated *in vitro* using UV-irradiated RSV antigen as specific antigen (third data column from the left), BSA as a non-specific antigen (second data column from the left), or PMA + Ion as a positive control stimulant (first data column from the left). T cell proliferation was determined as described in Example 1. Data shown are representative from three independent experiments. The single asterisk indicates P *<* 0.05, double asterisk indicates *p* < 0.001.

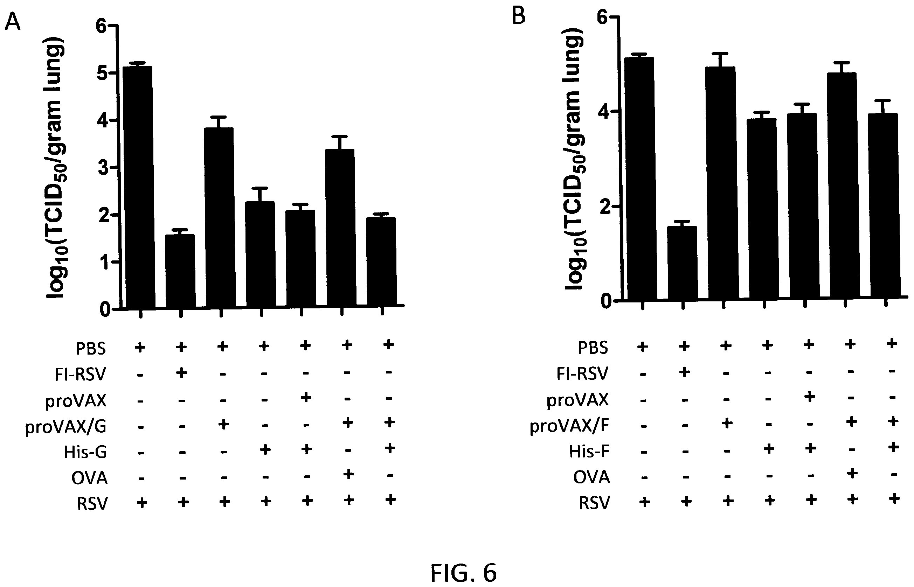

FIGS. 6A-6B show lung RSV titers of immunized mice.

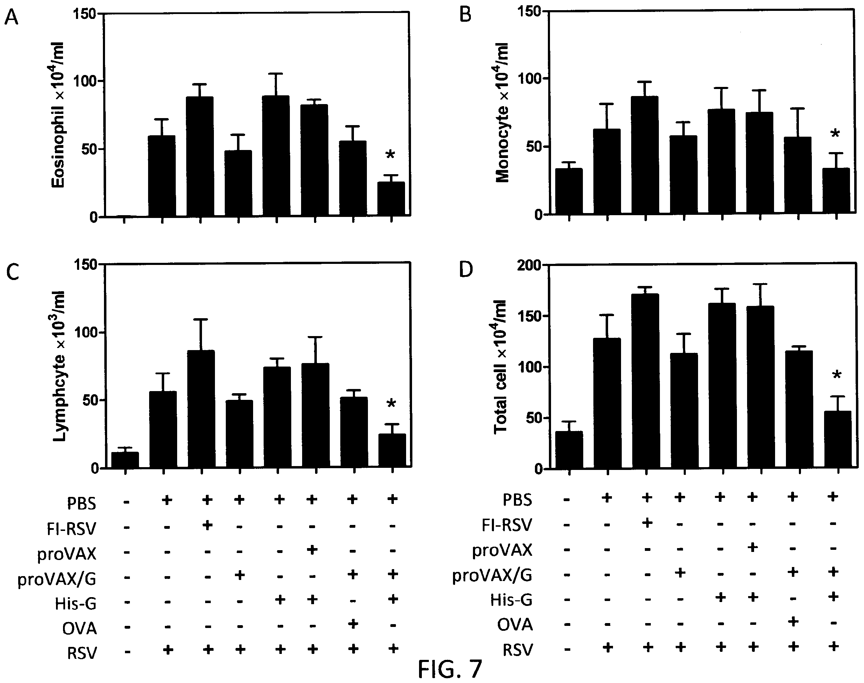

FIGS. 7A-7D show the amount of eosinophils, lymphocytes and monocytes present after RSV challenge compared with total cells. Mice were challenged intranasally with 10⁶ TCID₅₀ live RSV on day 14 after the last immunization. Mice were sacrificed 5 days after RSV challenge and BALs were analyzed. Mean ± SEM of total eosinophils (FIG. 7A), monocytes (FIG. 7B), lymphocytes (FIG. 7C) and total cells (FIG. 7D) are shown (n= 6 per group). The single asterisk indicates *p* < 0.05 in comparison with the PBS groups.

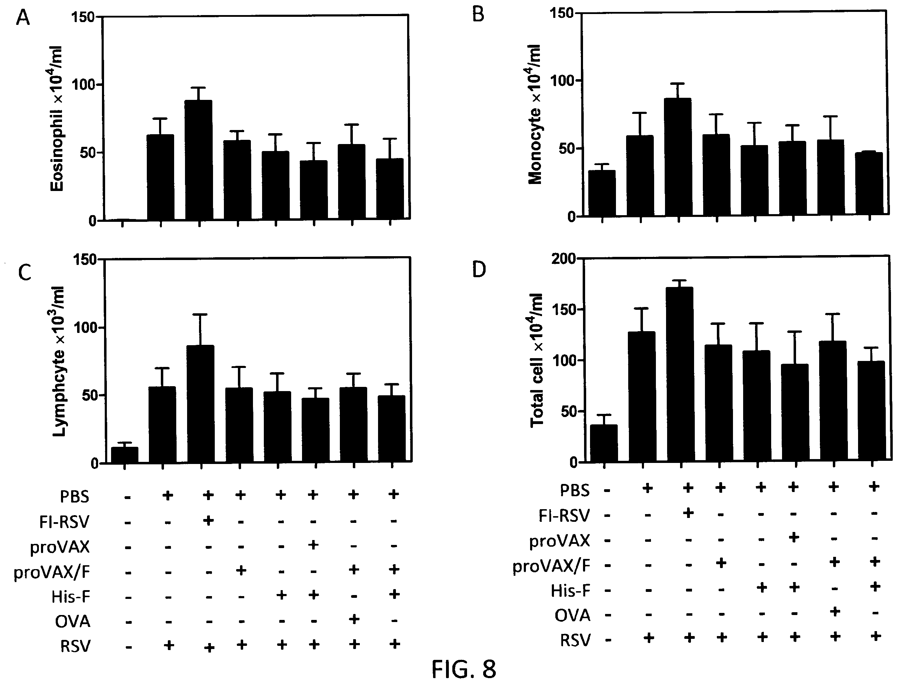

FIGS. 8A-8D show the amount of eosinophils, lymphocytes and monocytes present after RSV challenge compared with total cells. Mice were challenged intranasally with 10⁶ TCID₅₀ live RSV on day 14 after the last immunization. Mice were sacrificed 5 days after RSV challenge and BALs were analyzed. Mean ± SEM of total eosinophils (FIG. 8A), monocytes (FIG. 8B), lymphocytes (FIG. 8C) and total cells (FIG. 8D) are shown (n= 6 per group). The single asterisk indicates *p* < 0.05 in comparison with the PBS groups.

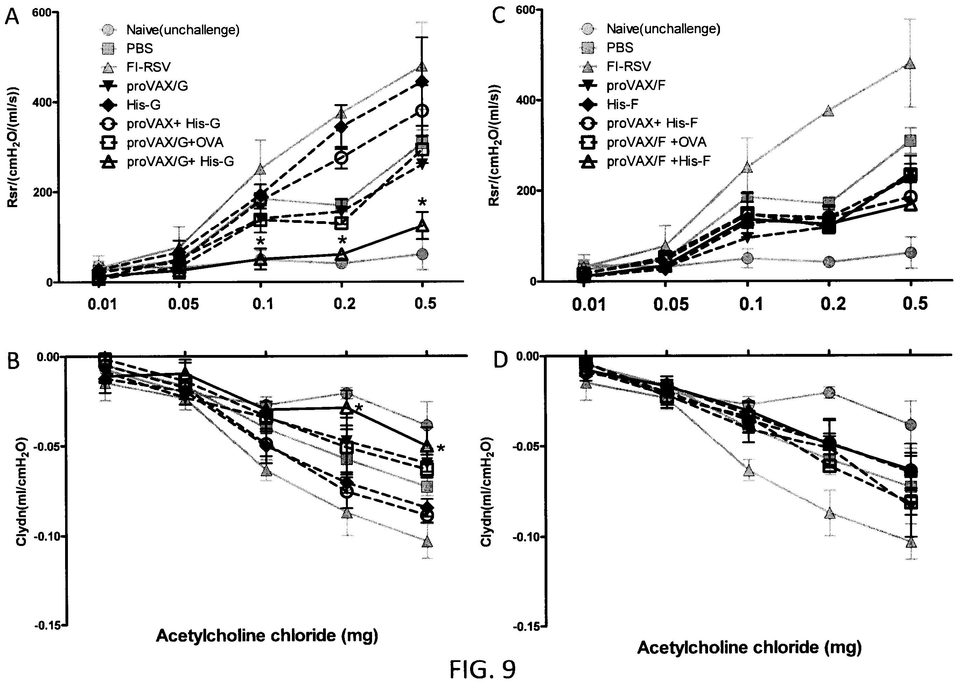

FIGS. 9A-9D show whole-body plethysmograpy in response to the intra-jugular administration of acetylcholine chloride. 5 days after RSV challenge, airway obstruction was measured by whole-body plethysmograpy in response to the intra-jugular administration of acetylcholine chloride at various doses (at the x-axis) and expressed as dynamic resistance (Rrs) (FIGS. 9A and 9C) and dynamic compliance (Cldyn) (FIGS. 9B and 9D) on the y-axis. The naive mice were without pre-treatment and challenge. The single asterisk indicates *p <* 0.05 in comparison with other groups.

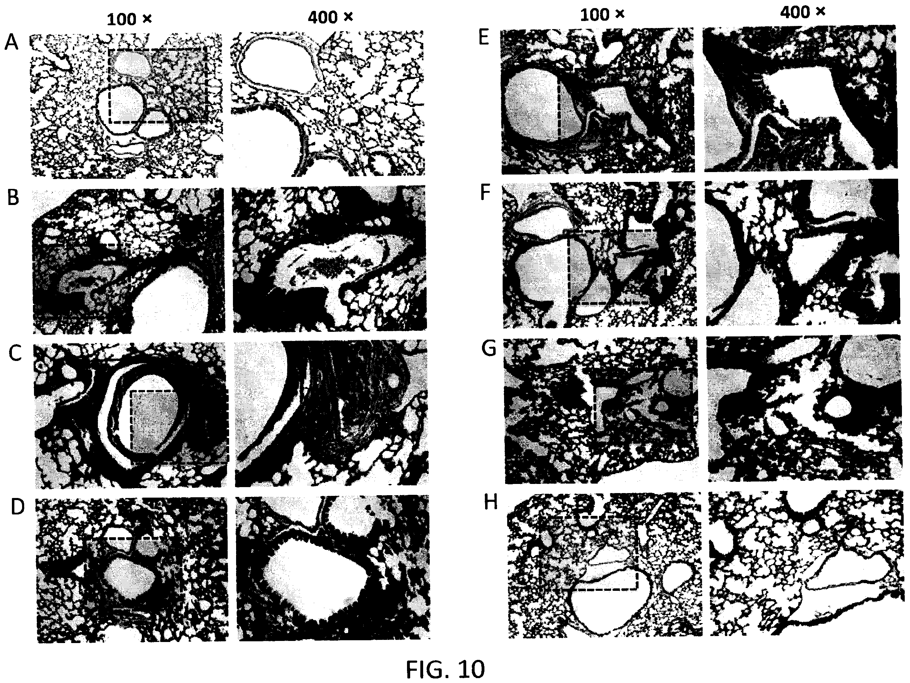

FIGS. 10A-10H show the histological examination of lung tissues after hematoxylin and eosin staining. 5 days after RSV challenge, mice were euthanized, and the lung tissues were removed and fixed in formalin. Thin sections of paraffin-embedded tissue were cut and stained with hematoxylin and eosin. A representative section (of 6 per group) is shown at each magnification (magnification, 100X or 400X) for tissues from naïve/unchallenged mice (FIG. 10A), PBS mice (FIG. 10B), FI-RSV mice (FIG. 10C), proVAX/G mice (FIG. 10D), His-G mice (FIG. 10E), proVA+His-G mice (FIG. 10F), proVAX/G+OVA mice (FIG. 10G) and proVAX/G+His-G mice (FIG. 10H).

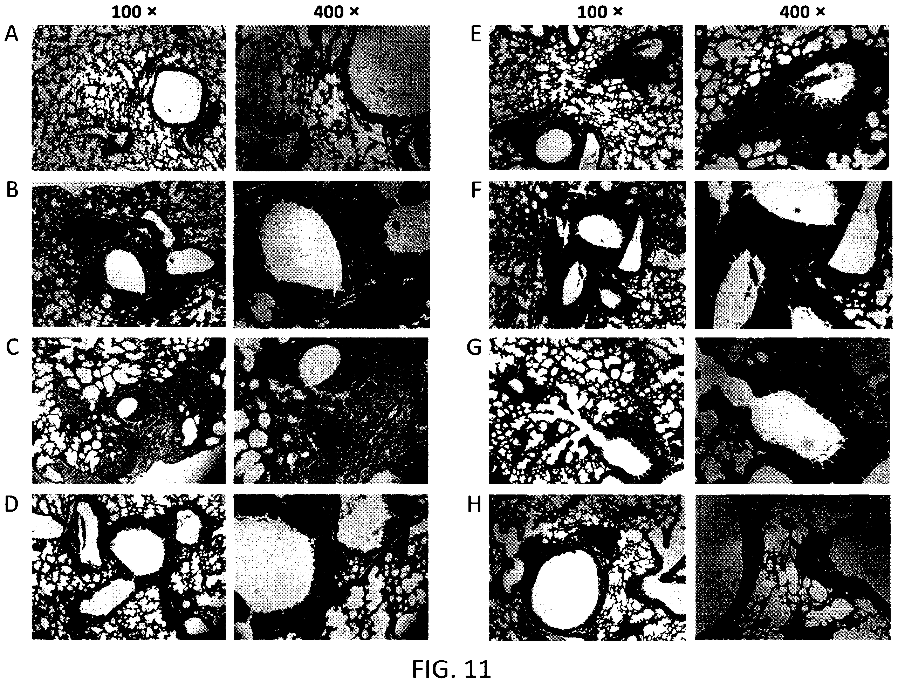

FIGS. 11A-11H show the histological examination of lung tissues after hematoxylin and eosin staining. 5 days after RSV challenge, mice were euthanized, and the lung tissues were removed and fixed in formalin. Thin sections of paraffin-embedded tissue were cut and stained with hematoxylin and eosin. A representative section (of 6 per group) is shown at each magnification (magnification, 100X or 400X) for tissues from naïve/unchallenged mice (FIG. 11A), PBS mice (FIG. 11B), FI-RSV mice (FIG. 11C), proVAX/G mice (FIG. 11D), His-G mice (FIG. 11E), proVA+His-G mice (FIG. 11F), proVAX/G+OVA mice (FIG. 11G) and proVAX/G+His-G mice (FIG. 11H).

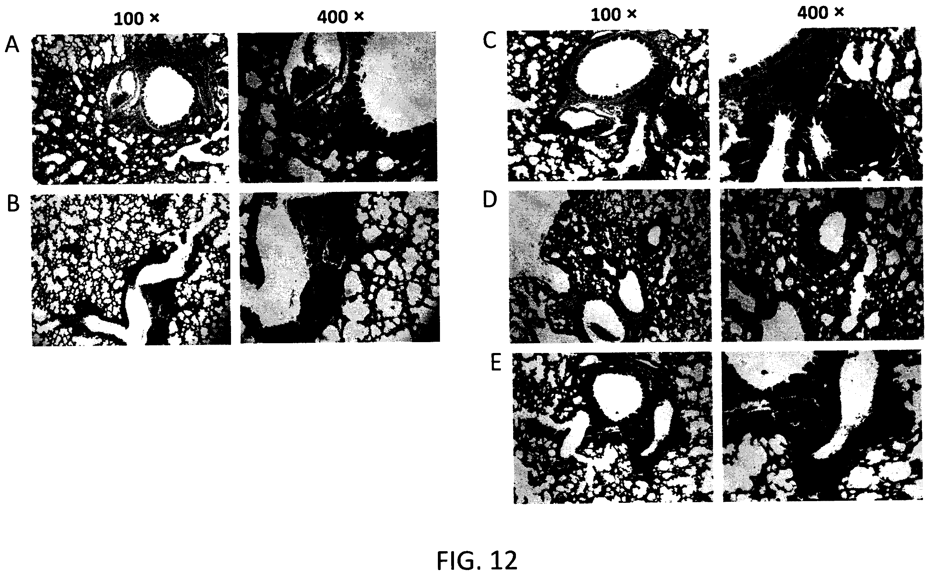

FIGS. 12A-12E show the histological examination of lung tissues after hematoxylin and eosin staining. 5 days after RSV challenge, mice were euthanized, and the lung tissues were removed and fixed in formalin. Thin sections of paraffin-embedded tissue were cut and stained with hematoxylin and eosin. A representative section (of 6 per group) is shown at each magnification (magnification, 100X or 400X) for tissues from proVAX/F mice (FIG. 12A), His-F mice (FIG. 12B), proVA+His-F mice (FIG. 12C), proVAX/F+OVA mice (FIG. 12D) and proVAX/F+His-F mice (FIG. 12E).

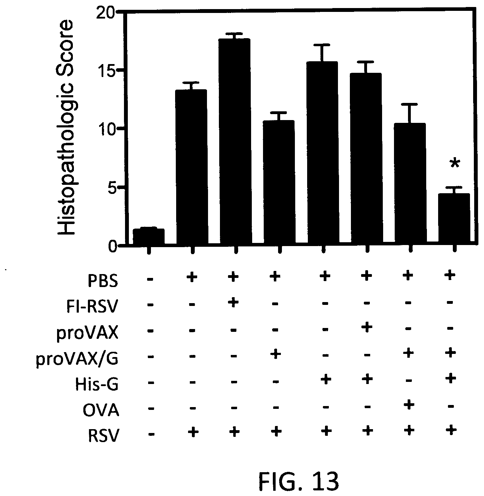

FIG. 13 shows the histopathologic scores of lung tissue described in FIG. 10. Histopathologic scores (HPS) were evaluated in hematoxylin and eosin stained tissue by a pathologist in a blinded fashion, as described in Example 1. The single asterisk indicates *p <* 0.05 in comparison with other groups.

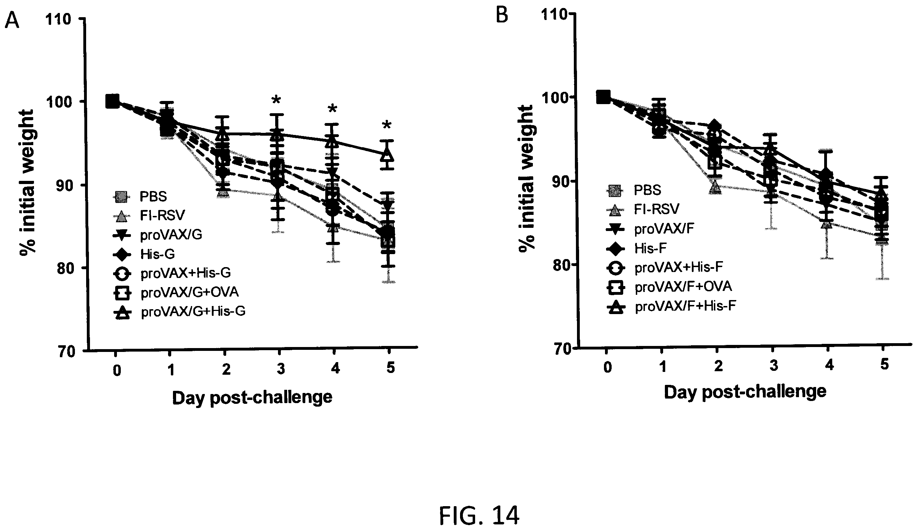

FIGS. 14A-14B show the weight loss in immunized mice following challenge with live RSV. Following RSV challenge, mice were weighed daily, and weights were normalized to the base weight at day 0. The single asterisk indicates *p* < 0.05 in comparison with other groups.

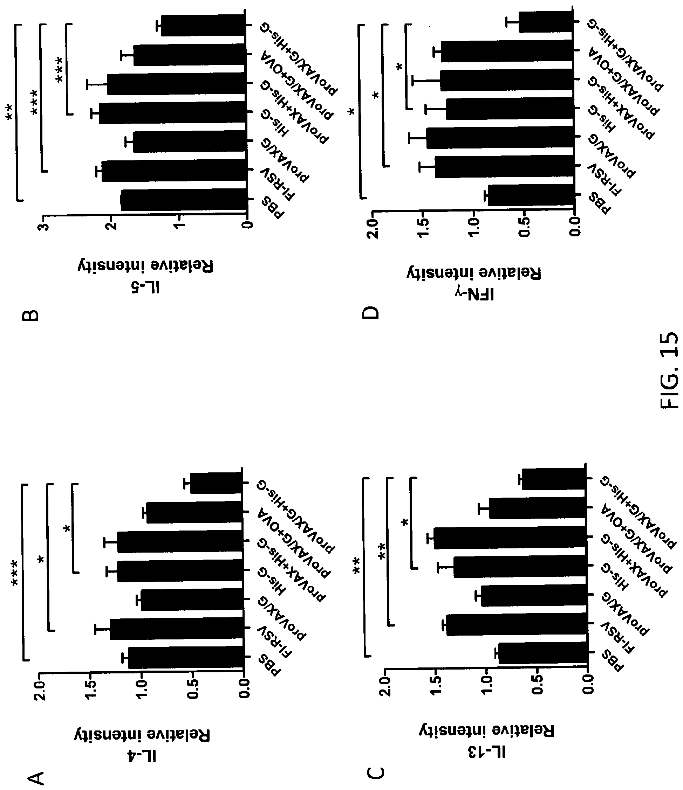

FIGS. 15A-15D show cytokine production in the lung tissues of mice on day 5 after RSV challenge examined by qPCR. Levels of IL-4 (FIG. 15A), IL-5 (FIG. 15B), IL-13 (FIG. 15C) and IFN-γ (FIG. 15D) were measured. The single asterisk indicates *p* < 0.05, double asterisk indicates *p* < 0.01 and triple asterisk indicates *p <* 0.001.

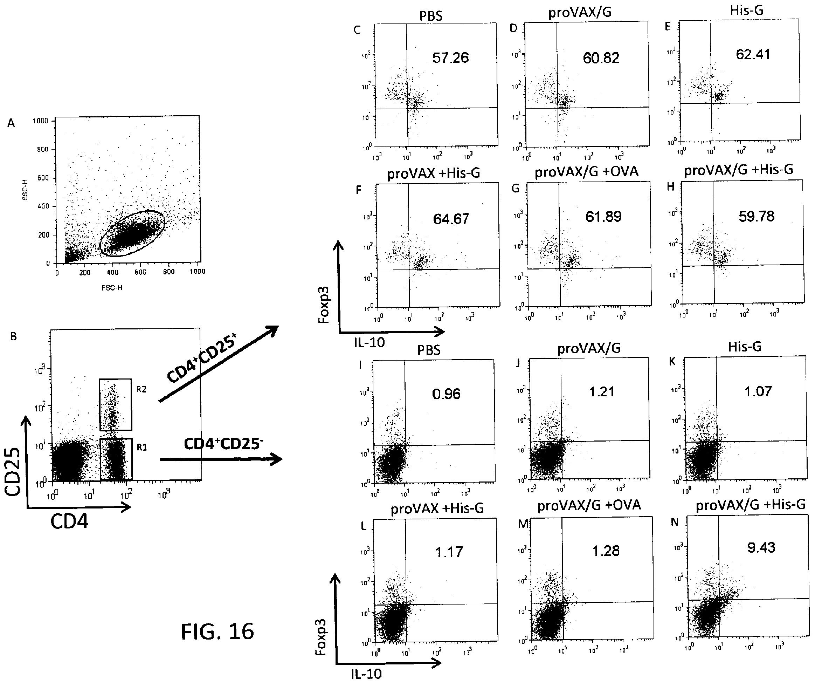

FIGS. 16A-16N show fluorescence-activated cell sorting (FACS) analysis of T cell phenotypes. Co-immunization induced iTreg cells can mediate antigen-specific T cell suppression *in vivo* and inhibit allo-mixed lymphocyte reaction *in vitro.* Lymphocytes were obtained from mice 7 days after the last immunization and stained with anti-CD4-FITC and anti-CD25⁻PE Cy5 mAb, and then intracellular stained with anti-IL-10- PE and anti-Foxp3-APC mAbs. FIG. 16A shows SSC-H versus FSC-H plot. FIG. 16B shows CD4 versus CD25 plot. FIGS. 16C-16N show IL-10 (x-axis) and Foxp3 (y-axis) co-expression. Both CD4⁺CD25⁻ (R1) (FIGS. 16I-16N) and CD4⁺CD25⁺ (FIGS. 16C-16H) (R2) T cells were gated for the co-expression of IL-10 and Foxp3. Results shown are representative of three experiments. Percentages represent percent of double-positive cells.

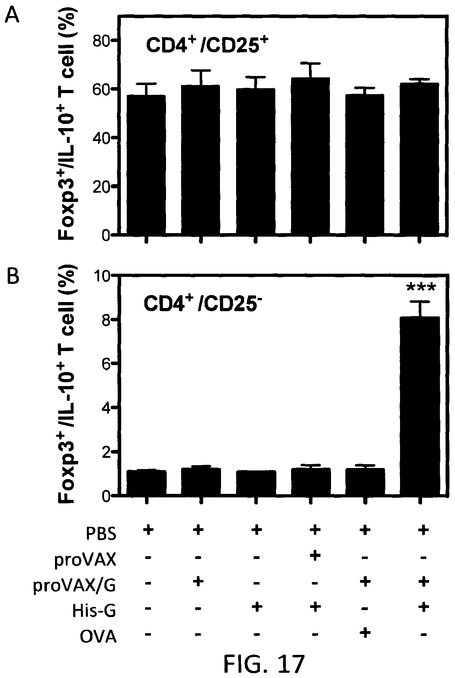

FIGS. 17A-17B show the percentage summaries of CD4⁺CD25⁻Foxp3⁺IL-10⁺ (FIG. 17B) or CD4⁺CD25⁺Foxp3⁺IL-10⁺ (FIG. 17A) T cells in total splenocytes. Triple asterisk indicates *p* < 0.001 when compared with other groups.

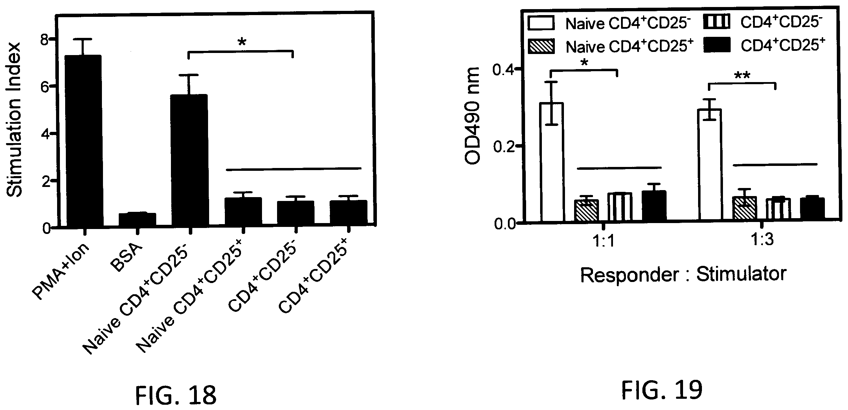

FIG. 18 shows suppression mediated by adoptively transferred CD4⁺CD25⁻ donor T cells. CD4⁺CD25⁻ or CD4⁺+CD25⁺ subsets of cells were prepared from Balb/c mice that had been co-immunized with proVAX/G + His-G or from naive mice, and adoptively transferred into naive Balb/c mice. The recipient mice were then immunized with His-G 24hr post-transfer and used to isolate splenocytes 7 days after the immunization. Splenocytes of recipients were restimulated *in vitro* using UV-irradiated RSV antigen as a specific antigen and the proliferative responses measured by the MTT method.
FIG. 19 shows suppression mediated by adoptively transferred CD4⁺CD25⁻ donor T cells. CD4⁺CD25⁻ or CD4⁺+CD25⁺ subsets of cells were prepared from Balb/c mice that had been co-immunized with proVAX/G + His-G or from naive mice, and adoptively transferred into naive Balb/c mice. The recipient mice were then immunized with His-G 24hr post-transfer and used to isolate splenocytes 7 days after the immunization. Splenocytes of recipient animals (as responder cells) were mixed with mitomycin C-treated splenocytes from naive C57BL/6 mice (as allogeneic stimulator cells) for MLR. The single asterisk indicates *p* < 0.05, double asterisk indicates *p* < 0.01.

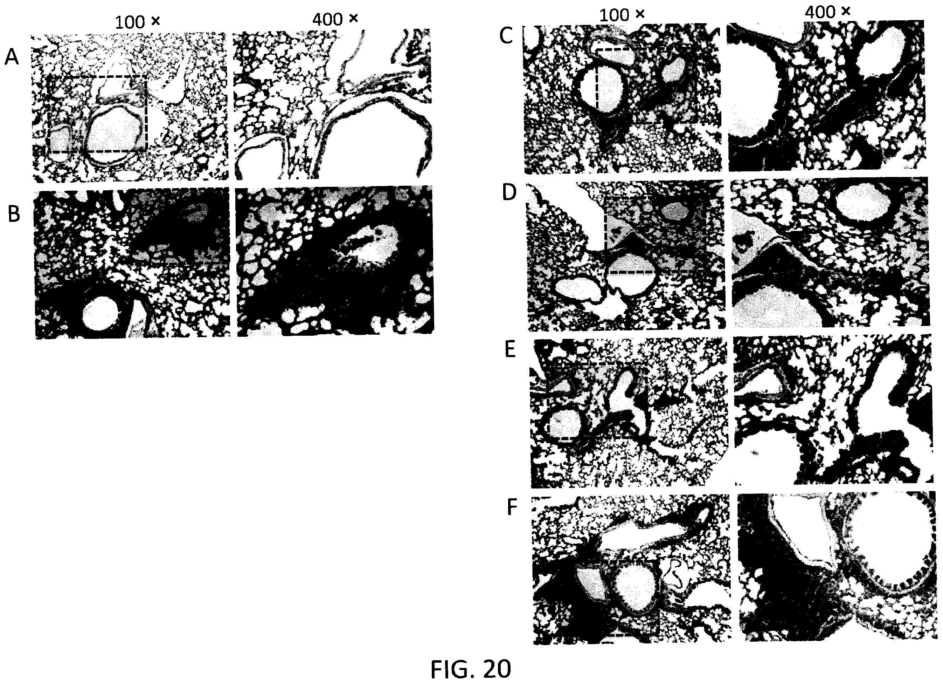

FIGS. 20A-20F show the amelioration of pulmonary inflammatory response by adoptive transfer CD4⁺CD25⁻ iTreg cells from the co-immunized mice. CD4⁺CD25⁺ and CD4⁺CD25⁻ T cells were purified from mice co-immunized with proVAX/G + His-G (antigen-matched) or proVAX/G + OVA (antigen-mismatched) on day 7 after the last immunization and these were adoptively transferred intravenously into mice that were previously immunized with His-G, then followed with RSV challenge. 5 days after RSV challenge, the mice were euthanized, and the lung tissues were removed and fixed in formalin. Thin sections of paraffin-embedded tissue were cut and stained with hematoxylin and eosin. A representative section (of 6 per group) is shown at each magnification (magnification, 100X or 400X) for tissues from naive mice (FIG. 20A), His-G mice (FIG. 20B), "CD4⁺CD25⁺ from proVAX/G+His-G" mice (FIG. 20C), "CD4⁺CD25⁻ from proVAX/G+His-G" mice (FIG. 20D), "CD4⁺CD25⁺ from proVAX/G+OVA" mice (FIG. 20E), and "CD4⁺CD25⁻ from proVAX/G+OVA" mice (FIG. 20F).

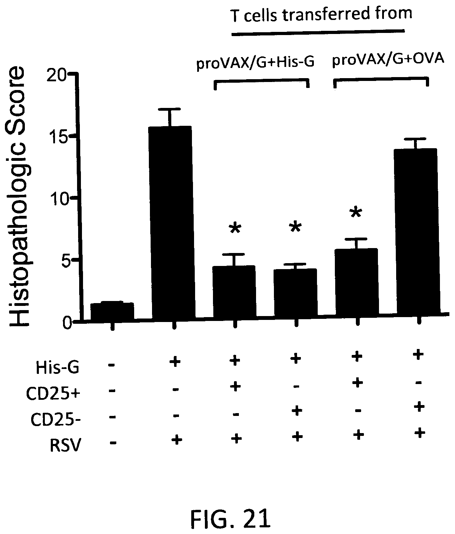

FIG. 21 shows the histopathologic scores of the lung tissue described in FIG. 20. Histopathologic scores (HPS) were evaluated in hematoxylin and eosin stained tissue by a pathologist in a blinded fashion, as described in Example 1. The single asterisk indicates *p <* 0.05.

### DETAILED DESCRIPTION

The present invention relates to vaccines for treating and protecting subjects from RSV infection while suppressing vaccine-induced disease due to previous RSV-antigen immunization.

Vaccine induced disease (VID) is due to the predisposition of naive individuals to exacerbate inflammatory responses, including massive lymphocytes infiltrations, pulmonary eosinophilia and type 2 cytokine productions, when they are immunized either with RSV antigen (such as F/G proteins) prior to encounter with the natural RSV infection. However, antibodies have a major role in protection. Passive transfer of neutralizing antibodies can protect immune-deficient individuals or children against RSV infection and the mouse model showed that treatment with anti-RSV neutralizing monoclonal antibody markedly decreased RSV replication and was associated with significant reduction of inflammatory and clinical markers of disease severity. Because of the high costs of passive immunotherapy in children, a vaccine aiming to induce a high level of neutralizing antibodies against RSV is of great interest.

The vaccine of the present invention comprises RSV protein-encoding nucleic acid and its cognate-recombinant RSV protein to protect against RSV infection and to also ameliorate RSV-induced pulmonary inflammatory disorders. The vaccine provides marked reduction of virus replication in the lungs by inducing neutralizing antibody against RSV infection as high as formulin-inactivated-RSV (FI-RSV) infection, but suppressed inflammatory T cells by inducing iTreg (CD4⁺, CD25⁻, FoxP3⁺, IL10⁺) cells. Antigen specific iTreg cells were induced by co-immunization with DNA and protein together. iTreg stimulated cells generated high levels of IL-10, which suggests stimulation of B cells for the neutralizing antibody production. This strategy induces high levels of neutralizing antibody as well as antigen-specific iTreg cells that suppress inflammatory T cells and reduce pathology. Thus, this strategy can protect against RSV infection effectively with minimal, if any, VID.

The use of co-immunization with protein and nucleic acid expressing cognate antigen to induce iTreg *in vivo* has several advantages, 1) it is comparatively easy, since it only requires administration of a defined protein antigen with its expressing plasmid; 2) it induces a potent iTreg to suppress T cells in an antigen specific manner; 3) it also induces a high level of antibodies. This approach can surmount the main obstacle to an RSV vaccine development since a vaccine with the dual functions, induction of neutralizing antibody and iTreg cells as disclosed, would likely be effective without the VID side-effect.

### 1. Definitions

The terminology used herein is for the purpose of describing particular embodiments only and is not intended to be limiting. As used in the specification and the appended claims, the singular forms "a," "and" and "the" include plural references unless the context clearly dictates otherwise.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

"Airway hyper-responsiveness (AHR)" as used herein refers to an abnormality of the airways that allows them to narrow too easily and/or too much in response to a stimulus capable of inducing airflow limitation. AHR can be a functional alteration of the respiratory system resulting from inflammation in the airways or airway remodeling (e.g., such as by collagen deposition). Airflow limitation refers to narrowing of airways that can be irreversible or reversible. Airflow limitation or airway hyperresponsiveness can be caused by collagen deposition, bronchospasm, airway smooth muscle hypertrophy, airway smooth muscle contraction, mucous secretion, cellular deposits, epithelial destruction, alteration to epithelial permeability, alterations to smooth muscle function or sensitivity, abnormalities of the lung parenchyma and infiltrative diseases in and around the airways. Many of these causative factors can be associated with inflammation.

"Consensus" or "Consensus Sequence" or "Optimized sequence" as used herein can mean a synthetic nucleic acid sequence, or corresponding polypeptide sequence, constructed based on analysis of an alignment of multiple subtypes of a particular antigen. The sequence can be used to induce broad immunity against multiple subtypes or sertypes of a particular antigen. Synthetic antigens, such as fusion proteins, can be manipulated to consensus sequences (or consensus antigens).

"Fragment" or "functional fragment" as used herein with respect to nucleic acid sequences means a nucleic acid sequence or a portion thereof, that encodes a polypeptide capable of eliciting an immune response in a mammal that cross reacts with a full length wild type RSV antigen. The fragments can be DNA fragments selected from at least one of the various nucleotide sequences that encode protein fragments set forth herein. The fragment can be a nucleic acid sequence encoding a portion of an RSV antigen wherein the portion encodes epitopes that can elicit an immune response in a mammal that cross reacts with a full length wild type RSV antigen.

"Fragment" or "functional fragment" as used herein with respect to polypeptide sequences means a polypeptide capable of eliciting an immune response in a mammal that cross reacts with a full length wild type RSV antigen. Fragments of consensus proteins may comprise at least 10%, at least 20%, at least 30%, at least 40%, at least 50%, at least 60%, at least 70%, at least 80%, at least 90% or at least 95% of a consensus protein. Fragment can be peptide sequence that is portion of the full length polypeptide antigen of a particular RSV protein such as the G glycoprotein or the F glycoprotein and encodes epitopes that can elicit an immune response in a mammal that cross reacts with a full length wild type RSV polypeptide antigen sequence. Fragments of an RSV protein may comprise at least 10%, at least 20%, at least 30%, at least 40%, at least 50%, at least 60%, at least 70%, at least 80%, at least 90% or at least 95% of a full length RSV protein and are capable of generating an immune response.

"nTreg cells" as used herein are T cells (CD4⁺, CD25⁺, Foxp3⁺) whose major mechanism is peripheral tolerance by inhibiting the proliferative responses of convention CD4 and CD8 T cells and can suppress autoimmune and allergic diseases. The transcription factor Foxp3 is necessary and sufficient for immune suppressive activity of the nTreg cells. nTreg cells proliferate readily in vivo in response to antigenic challenge or homeostatic expansion and exhibit higher rates of homeostatic division than convention CD4 T cells. nTreg cells have a high affinity T cell receptors for self antigens allowing these cells to be efficiently activated by self-antigens to suppress autoreactive T cells.

A "peptide" or "polypeptide" as used herein can mean a linked sequence of amino acids and can be natural, synthetic, or a modification or combination of natural and synthetic.

"Treatment" or "treating," as used herein can mean protecting of an animal from a disease through means of preventing, suppressing, repressing, or completely eliminating the disease. Preventing the disease involves administering a composition of the present invention to an animal prior to onset of the disease. Suppressing the disease involves administering a composition of the present invention to an animal after induction of the disease but before its clinical appearance. Repressing the disease involves administering a composition of the present invention to an animal after clinical appearance of the disease.

"Subject" as used herein can mean a mammal that wants to or is in need of being immunized with the RSV vaccine. The can be a human, chimpanzee, dog, cat, horse, cow, mouse, or rat.

"Superantigen" as used herein can mean a class of antigens which cause non-specific activation of T-cells resulting in polyclonal T cell activation and massive cytokine release. Compared to a normal antigen-induced T-cell response where 0.001-0.0001% of the body's T-cells are activated, superantigens are capable of activating up to 20% of the body's T-cells. The large number of activated T-cells generates a massive immune response which is not specific to any particular epitope on the superantigen thus undermining one of the fundamental strengths of the adaptive immune system, that is, its ability to target antigens with high specificity. More importantly, the large number of activated T-cells secrete large amounts of cytokines, the most important of which is Interferon gamma (IFN-γ). This excess amount of IFN-γ in turn activates the macrophages. The activated macrophages, in turn, overproduce proinflammatory cytokines such as IL-1, IL-6 and TNF-α. TNF-α is particularly important as a part of the body's inflammatory response. In normal circumstances it is released locally in low levels and helps the immune system defeat pathogens. However when it is systemically released in the blood and in high levels (due to mass T-cell activation resulting from the superantigen binding), it can cause severe and life-threatening symptoms, including shock and multiple organ failure.

"Substantially identical" as used herein can mean that a first and second amino acid sequence are at least 60%, 65%, 70%, 75%, 80%, 85%, 90%, 95%, 96%, 97%, 98%,or 99% over a region of 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 200, 300, 400, 500, 600, 700, 800, 900, 1000, 1100 amino acids.

A "variant" as used here can mean a peptide or polypeptide that differs in amino acid sequence by the insertion, deletion, or conservative substitution of amino acids, but retain at least one biological activity. Representative examples of "biological activity" include the ability to be bound by a specific antibody or to promote an immune response. Variant can also mean a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids, as understood in the art. Kyte et al., J. Mol. Biol. 157:105-132 (1982). The hydropathic index of an amino acid is based on a consideration of its hydrophobicity and charge. It is known in the art that amino acids of similar hydropathic indexes can be substituted and still retain protein function. In one aspect, amino acids having hydropathic indexes of ±2 are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity. Substitution of amino acids having similar hydrophilicity values can result in peptides retaining biological activity, for example immunogenicity, as is understood in the art. Substitutions can be performed with amino acids having hydrophilicity values within ±2 of each other. Both the hydrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties.

### 2. Vaccine

The present invention is directed to a vaccine comprising a respiratory syncytial virus (RSV) antigen and a nucleic acid encoding the same RSV antigen. The vaccine acts to coimmunize a subject in need thereof with both the virus RSV antigen and nucleic acid encoding the RSV antigen as described below. The vaccine provides the subject in need thereof an antigen-specific immune response against subsequent RSV challenge. The antigen-specific immune response generates high levels of neutralizing antibody comparable to the levels generated by formulin-inactivated-RSV (FI-RSV) vaccine. The vaccine, however, is different from the FI-RSV based vaccine in that iTreg cells (CD4⁺, CD25⁻, FoxP3⁺, IL10⁺) are induced, which results in a significant reduction of pulmonary abnormality in comparison to subjects immunized with FI-RSV vaccines. iTreg stimulated cells generated high levels of IL-10, which suggests stimulation of B cells for the neutralizing antibody production. The iTreg stimulation further results in less inflammation overall and infiltration of T cells into the lungs, characteristic of vaccine-induced disease upon reintroduction of the subject to RSV infection. The vaccine can further prevent and treat respiratory syncytial virus infection in a patient by protecting the subject from airway hyper-responsiveness (AHR) or airway obstruction after RSV challenge. The vaccine can further ameliorate pulmonary histopathogenesis of RSV infection. The vaccine can further reduce the severity of illness after live RSV challenge including combating weight loss.

FI-RSV induces VID due to activation of Th2 (CD4+) type response, which results in the release of β-cell activator and other related cytokines such as IL-3, IL-4, IL-5, CD40 ligands, IL-10, IL-13 granulocyte-macrophage colony-stimulating factor (GM-CSF), and eotaxin. RSV G glycoprotein (G) vaccines induce VID following RSV challenge as both Th1 and Th2 type response are induced. Th1 release macrophage activating effector and other related cytokines such as interferon-gamma (IFN-γ), GM-CSF, tumor necrosis factor alpha (TNF-α) IL-3, TNF-β and IL-2. The vaccine suppresses both Th1 and Th2 responses in an antigen dependent manner yet generate neutralizing antibody titers similar to those generated by Th2 helper cells thereby inducing neutralization and reducing the level of RSV infection.

The vaccine eliminates T cell (Th1, Th2, Th17) responses from the vaccination. The vaccine co-immunizing activity induces iTreg (CD4⁺, CD25⁻, Foxp3⁺) cells. iTreg cells play a role in both regulating the adaptive and innate immune responses to acute infection and in resolving inflammation following viral clearance. iTreg stimulated cells can generate high levels of IL-10, which stimulates B cells for neutralizing antibody production. The vaccine does not induce nTreg cells. RSV infection can actually lead to depletion of CD4+CD25+ nTregs in RSV infected subjects resulting in enhanced disease severity including increased weight loss, recruitment of innate cells to the bronchoalveolar lavage (BAL) fluid and lung, and increased levels of CD4+ and CD8+ T cells producing IFN-γ.

Co-immunization with the vaccine not only protected a subject from RSV challenge, but also suppressed the exacerbated pulmonary inflammation that leads to VID. The prevention of RSV infection and the reduced VID can be due to induction of both high levels of antigen specific neutralizing antibody and of iTreg cells that could suppress T-cell recalled proliferation in an antigen-specific manner. Such co-immunization up-regulated the level of the anti-inflammatory cytokine IL-10, while down regulating the RSV-induced inflammatory cytokines, such as IL-4, IL-5, IL-13 and IFN-γ.

The vaccine can have an RSV encoding nucleic acid to RSV antigen at a mass ratio of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10. The vaccine can have an RSV encoding nucleic acid to RSV antigen mass ratio range of 10:1 to 1:10, 9:1 to 1:9, 8:1 to 8:1, 7:1 to 1:7, 6:1 to 1:6, 5:1 to 1:5, 4:1 to 1:4, 3:1 to 1:3, or 2:1 to 1:2.

### a. Antigen

The vaccine of the invention comprises RSV antigen of SEQ ID NO:4. The RSV antigen is encoded by a nucleic acid wherein the nucleic acid is DNA. The RSV antigen can be a peptide or protein that causes an immune response. The antigen can trigger the production of an antibody by the immune system. The antibody generated by the immune response can then kill or neutralize the antigen that is recognized as a foreign and potentially harmful invader. The RSV antigen can be any molecule or molecular fragment that can be bound by a major histocompatibility complex (MHC) and presented to a T-cell receptor. The RSV antigen can be an immunogen, which is a molecule that is able to provoke an adaptive immune response.

According to the disclosure, the RSV antigen can be derived from any subtype, such as subtype A or subtype B, or any strain of naturally occurring or recombinant RSV, preferably from human RSV strains. Examples of RSV strains include, but not limited to, strains of subtype A, such as Long (ATCC® VR-26), A2 (ATCC® VR-1540), RSB1734, RSB5857, RSB6190, RSB6256, RSB642, and RSB6614, strains of subtype B, such as B1, 18537, 8/60, and 9320, S2, RSS-2, RSP112/Sweden/02-03, RSP120/Sweden/02-03, RSP121/Sweden/02-03, RSP122/Sweden/02-03, RSP13/Sweden/02-03), RSP140/Sweden/02-03, RSP16/Sweden/02-03, RSP171/Sweden/02-03, RSP183/Sweden/02-03, RSP191/Sweden/02-03, RSP199/Sweden/02-03, RSP212/Sweden/02-03, RSP41/Sweden/02-03, RSP45/Sweden/02-03, RSP56/Sweden/02-03, RSP58/Sweden/02-03, RSP67/Sweden/02-03 and RSP94/Sweden/02-03.

This disclosure demonstrates the protective efficacy obtained by co-immunization with DNA vaccine encoding RSV F antigen together with its F protein or by co-immunization with DNA vaccine encoding RSV G antigen together with its G protein, which gave protection against RSV challenge that was comparable to that obtained with FI-RSV. Other RSV antigens can have similar efficacy. The protection can be ascribed to induction of high levels of neutralizing antibody, similar to FI-RSV, however, the co-immunization protection is associated with the induction of iTreg and resulted in less inflammation and infiltration of T cells into lungs, i.e., significant reduction of pulmonary abnormality.

The vaccine includes an RSV antigenic peptide and DNA encoding the RSV antigenic peptide. In some embodiments, the RSV antigenic peptide is selected from the group consisting of human RSV F and G proteins.

Also provided herein is a DNA that encodes the antigen. The DNA can include an encoding sequence that encodes the antigen. The DNA can also include additional sequences that encode linker or tag sequences that are linked to the antigen by a peptide bond.

### (1) RSV F and G antigens

The RSV antigen of the disclosure can be a human RSV fusion protein (also referred to herein as "RSV F", "RSV F protein" and "F protein"), or fragment or variant thereof. The human RSV fusion protein is conserved between RSV subtypes A and B. The RSV antigen can be a
RSV F protein, or fragment or variant thereof, from the RSV Long strain (GenBank AAX23994.1; SEQ ID NO: 1) encoded by the nucleotide sequence of SEQ ID NO: 26, which corresponds to position 5660-7384 of GenBank AY911262.1 (SEQ ID NO: 6). The RSV antigen can be a RSV F protein from the RSV A2 strain (GenBank AAB59858.1), or a fragment or variant thereof. The RSV antigen can be a monomer, a dimer or trimer of the RSV F protein, or a fragment or variant thereof. The RSV antigen can be an optimized amino acid RSV F amino acid sequence, or fragment or variant thereof. For example, the RSV antigen can be amino acid 412-524 of RSV F or an optimized amino acid sequence thereof. The RSV antigen can be a fusion protein of a dimer of amino acid 412-524 of RSV F or an optimized amino acid sequence thereof, such as SEQ ID NO: 25. The RSV antigen can be an RSV F protein encoded by SEQ ID NO: 23, 24 or 26.

The postfusion form of RSV F elicits high titer neutralizing antibodies in immunized animals and protects the animals from RSV challenge. The present invention utilizes this immunoresponse in the claimed vaccines. According to the disclosure, the RSV F protein can be in a prefusion form or a postfusion form.

The RSV antigen of the invention is human RSV attachment glycoprotein (also referred to herein as "RSV G", "RSV G protein" and "G protein. The antigen is SEQ ID NO:4, an optimized amino acid sequence of RSV G protein The human RSV G protein differs between RSV subtypes A and B. The antigen RSV G protein, or fragment or variant thereof, from the RSV Long strain (GenBank AAX23993; SEQ ID NO: 2), encoded by the nucleotide sequence of SEQ ID NO: 19, which corresponds to position 4687-5583 of GenBank AY911262.1 (SEQ ID NO: 6) is also disclosed herein. The RSV antigen of the disclosure can be RSV G protein from: the RSV subtype B isolate H5601 (SEQ ID NO: 46), the RSV subtype B isolate H1068 (SEQ ID NO: 48), the RSV subtype B isolate H5598 (SEQ ID NO: 50), the RSV subtype B isolate H1123 (SEQ ID NO: 52), or a fragment or variant thereof. The RSV antigen of the disclosure can be an optimized amino acid RSV G amino acid sequence, or fragment or variant thereof. For example, the antigen can be amino acid 67-298 of RSV G protein. The RSV antigen can be an RSV G protein encoded by SEQ ID NOs: 3, 5, 19, 20, 21, 22, 45, 47, 49, or 51.

### (2) Other RSV antigens of the disclosure

The RSV antigen can be human RSV non-structural protein 1 ("NS1 protein"), or fragment or variant thereof. For example, the RSV antigen can be RSV NS1 protein, or fragment or variant thereof, from the RSV Long strain (GenBank AAX23987.1; SEQ ID NO: 28) encoded by the nucleotide sequence of SEQ ID NO: 27, which corresponds to position 99-518 of GenBank AY911262.1 (SEQ ID NO: 6).

The RSV antigen can be human RSV non-structural protein 2 ("NS2 protein"), or fragment or variant thereof. For example, the RSV antigen can be RSV NS2 protein, or fragment or variant thereof, from the RSV Long strain (GenBank AAX23988.1; SEQ ID NO: 30) encoded by the nucleotide sequence of SEQ ID NO: 29, which corresponds to position 628-1002 of GenBank AY911262.1 (SEQ ID NO: 6).

The RSV antigen can be human RSV nucleocapsid ("N") protein, or fragment or variant thereof. For example, the RSV antigen can be RSV N protein, or fragment or variant thereof, from the RSV Long strain (GenBank AAX23989.1; SEQ ID NO: 32) encoded by the nucleotide sequence of SEQ ID NO: 31, which corresponds to position 1140-2315 of GenBank AY911262.1 (SEQ ID NO: 6).

The RSV antigen can be human RSV Phosphoprotein ("P") protein, or fragment or variant thereof. For example, the RSV antigen can be RSV P protein, or fragment or variant thereof, from the RSV Long strain (GenBank AAX23990.1; SEQ ID NO: 34) encoded by the nucleotide sequence of SEQ ID NO: 33, which corresponds to position 2346-3071 of GenBank AY911262.1 (SEQ ID NO: 6).

The RSV antigen can be human RSV Matrix protein ("M") protein, or fragment or variant thereof. For example, the RSV antigen can be RSV M protein, or fragment or variant thereof, from the RSV Long strain (GenBank AAX23991.1; SEQ ID NO: 36) encoded by the nucleotide sequence of SEQ ID NO: 35, which corresponds to position 3261-4031 of GenBank AY911262.1 (SEQ ID NO: 6).

The RSV antigen can be human RSV small hydrophobic ("SH") protein, or fragment or variant thereof. For example, the RSV antigen can be RSV SH protein, or fragment or variant thereof, from the RSV Long strain (GenBank AAX23992.1; SEQ ID NO: 38) encoded by the nucleotide sequence of SEQ ID NO: 37, which corresponds to position 4302-4496 of GenBank AY911262.1 (SEQ ID NO: 6).

The RSV antigen can be human RSV Matrix protein2-1 ("M2-1") protein, or fragment or variant thereof. For example, the RSV antigen can be RSV M2-1 protein, or fragment or variant thereof, from the RSV Long strain (GenBank AAX23995.1; SEQ ID NO: 40) encoded by the nucleotide sequence of SEQ ID NO: 39, which corresponds to position 7605-8189 of GenBank AY911262.1 (SEQ ID NO: 6).

The RSV antigen can be human RSV Matrix protein 2-2 ("M2-2") protein, or fragment or variant thereof. For example, the RSV antigen can be RSV M2-2 protein, or fragment or variant thereof, from the RSV Long strain (GenBank AAX23997.1; SEQ ID NO: 42) encoded by the nucleotide sequence of SEQ ID NO: 41, which corresponds to position 8158-8430 of GenBank AY911262.1 (SEQ ID NO: 6).

The RSV antigen can be human RSV Polymerase L ("L") protein, or fragment or variant thereof. For example, the RSV antigen can be RSV L protein, or fragment or variant thereof, from the RSV Long strain (GenBank AAX23996.1; SEQ ID NO: 44) encoded by the nucleotide sequence of SEQ ID NO: 43, which corresponds to position 8497-14994 of GenBank AY911262.1 (SEQ ID NO: 6).

The RSV antigen can be an optimized amino acid sequence of NS1, NS2, N, P, M, SH, M2-1, M2-2, or L protein.

### (3) Summarization of RSV herein disclosed

The RSV antigen can be a human RSV protein or recombinant antigen, such as any one of the proteins encoded by the human RSV genome (SEQ ID NO: 6). The antigen can be a human RSV or recombinant protein or consensus thereof, a fragment thereof, or a variant thereof, of SEQ ID NOs: 2, 4, 25, 28, 30, 32, 34, 36, 38, 40, 42, 44, 46, 48, 50, or 52. The RSV antigen can be a human RSV or recombinant protein or consensus thereof, a fragment thereof, or a variant thereof, encoded by SEQ ID NO: 3, 5, 19, 20, 21, 22, 23, 24, 26, 27, 29, 31, 33, 35, 37, 39, 41, 43, 45, 47, 49, or 51.

SEQ ID NOs: 1 and 26 represent the amino acid and nucleotide sequences for the RSV F protein from the RSV Long strain.

SEQ ID NOs: 2 and 19 represent the amino acid and nucleotide sequences for the RSV G protein from the RSV Long strain.

SEQ ID NOs: 3, 5, 20, 21, and 22 represent nucleotide sequences encoding the optimized amino acid RSV G amino acid sequence of SEQ ID NO: 4 and belong to the invention. SEQ ID NO: 20 encodes the amino acid sequence of SEQ ID NO: 4. SEQ ID NOs: 3 and 21 represent prokaryotic (*E. coli*) codon-optimized sequences encoding the amino acid sequence of SEQ ID NO: 4 and SEQ ID NOs: 5 and 22 represent eukaryotic (mouse) codon-optimized sequences encoding the amino acid sequence of SEQ ID NO: 4.

SEQ ID NO: 6 represents the nucleotide sequence of the human RSV genome of the RSV Long strain.

SEQ ID NOs: 23 and 24 represent nucleotide sequences encoding the optimized amino acid RSV F amino acid sequence of SEQ ID NO: 25. SEQ ID NO: 23 encodes the amino acid sequence of SEQ ID NO: 25. SEQ ID NO: 24 represents a prokaryotic (*E. coli*) codon-optimized sequence encoding the amino acid sequence of SEQ ID NO: 25.

SEQ ID NOs: 27 and 28 represent the amino acid and nucleotide sequences for the RSV NS1 protein from the RSV Long strain.

SEQ ID NOs: 29 and 30 represent the amino acid and nucleotide sequences for the RSV NS2 protein from the RSV Long strain.

SEQ ID NOs: 31 and 32 represent the amino acid and nucleotide sequences for the RSV N protein from the RSV Long strain.

SEQ ID NOs: 33 and 34 represent the amino acid and nucleotide sequences for the RSV P protein from the RSV Long strain.

SEQ ID NOs: 35 and 36 represent the amino acid and nucleotide sequences for the RSV M protein from the RSV Long strain.

SEQ ID NOs: 37 and 38 represent the amino acid and nucleotide sequences for the RSV SH protein from the RSV Long strain.

SEQ ID NOs: 39 and 40 represent the amino acid and nucleotide sequences for the RSV M2-1 protein from the RSV Long strain.

SEQ ID NOs: 41 and 42 represent the amino acid and nucleotide sequences for the RSV M2-2 protein from the RSV Long strain.

SEQ ID NOs: 43 and 44 represent the amino acid and nucleotide sequences for the RSV L protein from the RSV Long strain.

SEQ ID NOs: 45 and 46 represent the amino acid and nucleotide sequences for the RSV G protein from the RSV subtype B isolate H5601.

SEQ ID NOs: 47 and 48 represent the amino acid and nucleotide sequences for the RSV G protein from the RSV subtype B isolate H1068.

SEQ ID NOs: 49 and 50 represent the amino acid and nucleotide sequences for the RSV G protein from the RSV subtype B isolate H5598.

SEQ ID NOs: 51 and 52 represent the amino acid and nucleotide sequences for the RSV G protein from the RSV subtype B isolate H1123.

### b. Vectors

The vaccine can comprise the RSV nucleic acid encoding the antigen and this nucleic acid can be located in a vector. The vector therefore can include the nucleic acid encoding the antigen described above. The vector can be capable of expressing the antigen. The vector can be an expression construct, which is generally a plasmid that is used to introduce a specific gene into a target cell. Once the expression vector is inside the cell, the protein that is encoded by the gene is produced by the cellular-transcription and translation machinery ribosomal complexes. The plasmid is frequently engineered to contain regulatory sequences that act as enhancer and promoter regions and lead to efficient transcription of the gene carried on the expression vector. The vectors of the present invention express large amounts of stable messenger RNA, and therefore proteins.

A particular DNA vector comprising the DNA encoding the antigen is proVAX/G (SEQ ID NO: 22), which corresponds to the coding region for 67-298 amino acid of RSV G glycoprotein (full length nucleotide sequence corresponds to position 4687-5583 of GenBank: AY911262.1 (RSV genome; SEQ ID NO: 6; full length nucleotide sequence (SEQ ID NO: 19)). Another particular DNA vector comprising the DNA encoding the antigen is proVAX/F (SEQ ID NO: 23), which corresponds to a dimer of the coding region of 412-524 amino acid of RSV F fusion protein (full length nucleotide sequence corresponds to position 5660-7384 of GenBank: AY911262.1 (SEQ ID NO: 6); full length F fusion protein nucleotide sequence (SEQ ID NO: 26)). The coding region can be codon optimized, i.e., codons which are employed more frequently in one organism relative to another organism, a distantly related organism, as well as modifications to add or modify Kozak sequences and/or introns, and /or to remove undesirable sequences, for instance, potential transcription factor binding sites.

The vectors can have expression signals such as a strong promoter, a strong termination codon, adjustment of the distance between the promoter and the cloned gene, and the insertion of a transcription termination sequence and a PTIS (portable translation initiation sequence).

### (1) Expression Vectors

The vector can be a circular plasmid or a linear nucleic acid. The circular plasmid and linear nucleic acid are capable of directing expression of a particular nucleotide sequence in an appropriate subject cell. The vector can have a promoter operably linked to the antigen-encoding nucleotide sequence, which can be operably linked to termination signals. The vector can also contain sequences required for proper translation of the nucleotide sequence. The vector comprising the nucleotide sequence of interest can be chimeric, meaning that at least one of its components is heterologous with respect to at least one of its other components. The expression of the nucleotide sequence in the expression cassette can be under the control of a constitutive promoter or of an inducible promoter which initiates transcription only when the host cell is exposed to some particular external stimulus. In the case of a multicellular organism, the promoter can also be specific to a particular tissue or organ or stage of development.

### (2) Circular or Linear Vectors

The vector can be circular plasmid, which can transform a target cell by integration into the cellular genome or exist extrachromosomally (e.g. autonomous replicating plasmid with an origin of replication).

The vector can be pVAX, pcDNA3.0, or proVAX, or any other expression vector capable of expressing the DNA and enabling a cell to translate the sequence to the antigen that is recognized by the immune system. Also provided herein is a linear nucleic acid vaccine, or linear expression cassette ("LEC"), that is capable of being efficiently delivered to a subject via electroporation and expressing one or more desired antigens. The LEC can be any linear DNA devoid of any phosphate backbone. The DNA can encode one or more antigens. The LEC can contain a promoter, an intron, a stop codon, a polyadenylation signal. The expression of the antigen can be controlled by the promoter. The LEC can not contain any antibiotic resistance genes and/or a phosphate backbone. The LEC can not contain other nucleic acid sequences unrelated to the desired antigen gene expression.

The LEC can be derived from any plasmid capable of being linearized. The plasmid can be capable of expressing the antigen. The plasmid can be pNP (Puerto Rico/34) or pM2 (New Caledonia/99). The plasmid can be pVAX, pcDNA3.0, or proVAX, or any other expression vector capable of expressing the DNA and enabling a cell to translate the sequence to the antigen that is recognized by the immune system.

The LEC can be pcrM2. The LEC can be pcrNP. pcrNP and pcrMR can be derived from pNP (Puerto Rico/34) and pM2 (New Caledonia/99), respectively. The LEC can be combined with antigen at a mass ratio of between 5:1 and 1:5, or of between 1:1 to 2:1.

### (3) Promoter, Intron, Stop codon, and Polyadenylation signal

The vector can have a promoter. A promoter can be any promoter that is capable of driving gene expression and regulating expression of the isolated nucleic acid. Such a promoter is a cis-acting sequence element required for transcription via a DNA dependent RNA polymerase, which transcribes the antigen sequence described herein. Selection of the promoter used to direct expression of a heterologous nucleic acid depends on the particular application. The promoter can be positioned about the same distance from the transcription start in the vector as it is from the transcription start site in its natural setting. However, variation in this distance can be accommodated without loss of promoter function.

The promoter can be operably linked to the nucleic acid sequence encoding the antigen and signals required for efficient polyadenylation of the transcript, ribosome binding sites, and translation termination. The promoter can be a CMV promoter, SV40 early promoter, SV40 later promoter, metallothionein promoter, murine mammary tumor virus promoter, Rous sarcoma virus promoter, polyhedrin promoter, or another promoter shown effective for expression in eukaryotic cells.

The vector can include an enhancer and an intron with functional splice donor and acceptor sites. The vector can contain a transcription termination region downstream of the structural gene to provide for efficient termination. The termination region can be obtained from the same gene as the promoter sequence or can be obtained from different genes.

### c. Excipients and other components of the Vaccine

The vaccine can further comprise other components such as a transfection facilitating agent, a pharmaceutically acceptable excipient, an adjuvant. The pharmaceutically acceptable excipient can be functional molecules as vehicles, adjuvants, carriers, or diluents. The pharmaceutically acceptable excipient can be a transfection facilitating agent, which can include surface active agents, such as immune-stimulating complexes (ISCOMS), Freunds incomplete adjuvant, LPS analog including monophosphoryl lipid A, muramyl peptides, quinone analogs, vesicles such as squalene and squalene, hyaluronic acid, lipids, liposomes, calcium ions, viral proteins, polyanions, polycations, or nanoparticles, or other known transfection facilitating agents.

The transfection facilitating agent can be a polyanion, polycation, including poly-L-glutamate (LGS), or lipid. The transfection facilitating agent can be poly-L-glutamate. The poly-L-glutamate can be present in the vaccine at a concentration less than 6 mg/ml. The transfection facilitating agent can also include surface active agents such as immune-stimulating complexes (ISCOMS), Freunds incomplete adjuvant, LPS analog including monophosphoryl lipid A, muramyl peptides, quinone analogs and vesicles such as squalene and squalene, and hyaluronic acid can also be used administered in conjunction with the genetic construct. In some embodiments, the DNA plasmid vaccines can also include a transfection facilitating agent such as lipids, liposomes, including lecithin liposomes or other liposomes known in the art, as a DNA-liposome mixture (see for example WO9324640), calcium ions, viral proteins, polyanions, polycations, or nanoparticles, or other known transfection facilitating agents. The transfection facilitating agent is a polyanion, polycation, including poly-L-glutamate (LGS), or lipid. Concentration of the transfection agent in the vaccine is less than 4 mg/ml, less than 2 mg/ml, less than 1 mg/ml, less than 0.750 mg/ml, less than 0.500 mg/ml, less than 0.250 mg/ml, less than 0.100 mg/ml, less than 0.050 mg/ml, or less than 0.010 mg/ml.

The pharmaceutically acceptable excipient can be an adjuvant. The adjuvant can be other genes that are expressed in alternative plasmid or are delivered as proteins in combination with the plasmid above in the vaccine. The adjuvant can be selected from the group consisting of: α-interferon(IFN-α), β-interferon (IFN-β), γ-interferon, platelet derived growth factor (PDGF), TNFα, TNFβ, GM-CSF, epidermal growth factor (EGF), cutaneous T cell-attracting chemokine (CTACK), epithelial thymus-expressed chemokine (TECK), mucosae-associated epithelial chemokine (MEC), IL-12, IL-15, MHC, CD80,CD86 including IL-15 having the signal sequence deleted and optionally including the signal peptide from IgE. The adjuvant can be IL-12, IL-15, IL-28, CTACK, TECK, platelet derived growth factor (PDGF), TNFα, TNFβ, GM-CSF, epidermal growth factor (EGF), IL-1, IL-2, IL-4, IL-5, IL-6, IL-10, IL-12, IL-18, or a combination thereof.

Other genes that can be useful adjuvants include those encoding: MCP-1, MIP-la, MIP-1p, IL-8, RANTES, L-selectin, P-selectin, E-selectin, CD34, GlyCAM-1, MadCAM-1, LFA-1, VLA-1, Mac-1, p150.95, PECAM, ICAM-1, ICAM-2, ICAM-3, CD2, LFA-3, M-CSF, G-CSF, IL-4, mutant forms of IL-18, CD40, CD40L, vascular growth factor, fibroblast growth factor, IL-7, nerve growth factor, vascular endothelial growth factor, Fas, TNF receptor, Flt, Apo-1, p55, WSL-1, DR3, TRAMP, Apo-3, AIR, LARD, NGRF, DR4, DR5, KILLER, TRAIL-R2, TRICK2, DR6, Caspase ICE, Fos, c-jun, Sp-1, Ap-1, Ap-2, p38, p65Rel, MyD88, IRAK, TRAF6, IkB, Inactive NIK, SAP K, SAP-1, JNK, interferon response genes, NFkB, Bax, TRAIL, TRAILrec, TRAILrecDRC5, TRAIL-R3, TRAIL-R4, RANK, RANK LIGAND, Ox40, Ox40 LIGAND, NKG2D, MICA, MICB, NKG2A, NKG2B, NKG2C, NKG2E, NKG2F, TAP1, TAP2 and functional fragments thereof.

The vaccine can further comprise a genetic vaccine facilitator agent as described in U.S. Serial No. 021,579 filed April 1994.

The vaccine can be formulated according to the mode of administration to be used. An injectable vaccine pharmaceutical composition can be sterile, pyrogen free and particulate free. An isotonic formulation or solution can be used. Additives for isotonicity can include sodium chloride, dextrose, mannitol, sorbitol, and lactose. The vaccine can comprise a vasoconstriction agent. The isotonic solutions can include phosphate buffered saline. Vaccine can further comprise stabilizers including gelatin and albumin. The stabilizers can allow the formulation to be stable at room or ambient temperature for extended periods of time, including LGS or polycations or polyanions.

### 3. Vaccination to Treat or Prevent

The present invention is also directed to the vaccine as described herein for use in methods for preventing and treating respiratory syncytial virus (RSV) infection in a patient. The vaccine includes an RSV antigenic peptide and a nucleic acid encoding the RSV antigenic peptide. The co-immunization vaccine can stimulate iTreg cells, including but not limited to, CD4⁺, CD25⁻, FoxP3⁺, IL-10⁺.

The co-immunization can be used to treat or ameliorate vaccine-induced disease (VID) which is due to the predisposition of naive individuals to exacerbation of inflammatory responses, including massive lymphocytes infiltrations, pulmonary eosinophilia and type 2 cytokine productions, when they are immunized either with FI-RSV or its G antigen or its F antigen prior to encounter with the natural RSV infection. VID amelioration can be indicated by reduced histomorphological changes in lung following virus challenge, and or the association with significant inhibition of the proliferation and infiltration of lymphocytes and eosinophils. For example, amelioration of VID can be caused by the induction of G antigen specific iTreg cells exhibiting a CD4⁺CD25⁻FoxP3⁺IL-10⁺ phenotype. iTreg stimulated cells can generate high levels of IL-10, which stimulates B cells for neutralizing antibody production.

The co-immunization can induce the up-regulation of anti-inflammatory cytokine Il-10 levels, while down regulating the RSV-induced inflammatory cytokines, such as IL-4, Il-5, IL-13 and IFN-γ.

The vaccine dose can be between 1 µg to 10 mg active component/kg body weight/time, and can be 20 µg to 10 mg component/kg body weight/time. The vaccine can be administered every 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days. The number of vaccine doses for effective treatment can be 1, 2, 3, 4, 5, 6, 7, 8, 9, or 10.

### a. Administration

The vaccine can be formulated in accordance with standard techniques well known to those skilled in the pharmaceutical art. Such compositions can be administered in dosages and by techniques well known to those skilled in the medical arts taking into consideration such factors as the age, sex, weight, and condition of the particular subject, and the route of administration. The subject can be a mammal, such as a human, a horse, a cow, a pig, a sheep, a cat, a dog, a rat, or a mouse.

The vaccine can be for prophylactic or therapeutic administration. In prophylactic administration, the vaccines can be administered in an amount sufficient to induce iTreg responses. In therapeutic applications, the vaccines are administered to a subject in need thereof in an amount sufficient to elicit a therapeutic effect. An amount adequate to accomplish this is defined as "therapeutically effective dose." Amounts effective for this use will depend on, e.g., the particular composition of the vaccine regimen administered, the manner of administration, the stage and severity of the disease, the general state of health of the patient, and the judgment of the prescribing physician.

The vaccine can be administered by methods well known in the art as described in Donnelly et al. (Ann. Rev. Immunol. 15:617-648 (1997)); Felgner et al. (U.S. Pat. No. 5,580,859, issued Dec. 3, 1996); Felgner (U.S. Pat. No. 5,703,055, issued Dec. 30, 1997); and Carson et al. (U.S. Pat. No. 5,679,647, issued Oct. 21, 1997). The DNA of the vaccine can be complexed to particles or beads that can be administered to an individual, for example, using a vaccine gun. One skilled in the art would know that the choice of a pharmaceutically acceptable carrier, including a physiologically acceptable compound, depends, for example, on the route of administration of the expression vector.

The vaccines can be delivered via a variety of routes. Typical delivery routes include parenteral administration, e.g., intradermal, intramuscular or subcutaneous delivery. Other routes include oral administration, intranasal, and intravaginal routes. For the DNA of the vaccine in particular, the vaccine can be delivered to the interstitial spaces of tissues of an individual (Felgner et al., U.S. Pat. Nos. 5,580,859 and 5,703,055). The vaccine can also be administered to muscle, or can be administered via intradermal or subcutaneous injections, or transdermally, such as by iontophoresis. Epidermal administration of the vaccine can also be employed. Epidermal administration can involve mechanically or chemically irritating the outermost layer of epidermis to stimulate an immune response to the irritant (Carson et al., U.S. Pat. No. 5,679,647).

The vaccine can also be formulated for administration via the nasal passages. Formulations suitable for nasal administration, wherein the carrier is a solid, can include a coarse powder having a particle size, for example, in the range of about 10 to about 500 microns which is administered in the manner in which snuff is taken, i.e., by rapid inhalation through the nasal passage from a container of the powder held close up to the nose. The formulation can be a nasal spray, nasal drops, or by aerosol administration by nebulizer. The formulation can include aqueous or oily solutions of the vaccine.

The vaccine can be a liquid preparation such as a suspension, syrup or elixir. The vaccine can also be a preparation for parenteral, subcutaneous, intradermal, intramuscular or intravenous administration (e.g., injectable administration), such as a sterile suspension or emulsion.

The vaccine can be incorporated into liposomes, microspheres or other polymer matrices (Felgner et al., U.S. Pat. No. 5,703,055; Gregoriadis, Liposome Technology, Vols. Ito III (2nd ed. 1993). Liposomes can consist of phospholipids or other lipids, and can be nontoxic, physiologically acceptable and metabolizable carriers that are relatively simple to make and administer.

The vaccine is for administration via electroporation, such as by a method described in U.S. Patent No. 7,664,545. The electroporation can be by a method and/or apparatus described in U.S. Patent Nos. 6,302,874; 5,676,646; 6,241,701; 6,233,482; 6,216,034; 6,208,893; 6,192,270; 6,181,964; 6,150,148; 6,120,493; 6,096,020; 6,068,650; and 5,702,359,. The electroporation can be carried out via a minimally invasive device.

The minimally invasive electroporation device ("MID") can be an apparatus for injecting the vaccine described above and associated fluid into body tissue. The device can comprise a hollow needle, DNA cassette, and fluid delivery means, wherein the device is adapted to actuate the fluid delivery means in use so as to concurrently (for example, automatically) inject DNA into body tissue during insertion of the needle into the said body tissue. This has the advantage that the ability to inject the DNA and associated fluid gradually while the needle is being inserted leads to a more even distribution of the fluid through the body tissue. The pain experienced during injection can be reduced due to the distribution of the DNA being injected over a larger area.

The MID can inject the vaccine into tissue without the use of a needle. The MID can inject the vaccine as a small stream or jet with such force that the vaccine pierces the surface of the tissue and enters the underlying tissue and/or muscle. The force behind the small stream or jet can be provided by expansion of a compressed gas, such as carbon dioxide through a micro-orifice within a fraction of a second. Examples of minimally invasive electroporation devices, and methods of using them, are described in published U.S. Patent Application No. 20080234655; U.S. Patent No. 6,520,950; U.S. Patent No. 7,171,264; U.S. Patent No. 6,208,893; U.S. Patent NO. 6,009,347; U.S. Patent No. 6,120,493; U.S. Patent No. 7,245,963; U.S. Patent No. 7,328,064; and U.S. Patent No. 6,763,264.

The MID can comprise an injector that creates a high-speed jet of liquid that painlessly pierces the tissue. Such needle-free injectors are commercially available. Examples of needle-free injectors that can be utilized herein include those described in U.S. Patent Nos. 3,805,783; 4,447,223; 5,505,697; and 4,342,310.

A desired vaccine in a form suitable for direct or indirect electrotransport can be introduced (e.g., injected) using a needle-free injector into the tissue to be treated, usually by contacting the tissue surface with the injector so as to actuate delivery of a jet of the agent, with sufficient force to cause penetration of the vaccine into the tissue. For example, if the tissue to be treated is mucosa, skin or muscle, the agent is projected towards the mucosal or skin surface with sufficient force to cause the agent to penetrate through the stratum corneum and into dermal layers, or into underlying tissue and muscle, respectively.

Needle-free injectors are well suited to deliver vaccines to all types of tissues, particularly to skin and mucosa. In some embodiments, a needle-free injector can be used to propel a liquid that contains the vaccine to the surface and into the subject's skin or mucosa. Representative examples of the various types of tissues that can be treated using the invention methods include pancreas, larynx, nasopharynx, hypopharynx, oropharynx, lip, throat, lung, heart, kidney, muscle, breast, colon, prostate, thymus, testis, skin, mucosal tissue, ovary, blood vessels, or any combination thereof.

The MID can have needle electrodes that electroporate the tissue. By pulsing between multiple pairs of electrodes in a multiple electrode array, for example, set up in rectangular or square patterns, provides improved results over that of pulsing between a pair of electrodes. Disclosed, for example, in U.S. Patent No. 5,702,359 entitled "Needle Electrodes for Mediated Delivery of Drugs and Genes" is an array of needles wherein a plurality of pairs of needles can be pulsed during the therapeutic treatment. In that application, needles were disposed in a circular array, but have connectors and switching apparatus enabling a pulsing between opposing pairs of needle electrodes. A pair of needle electrodes for delivering recombinant expression vectors to cells can be used. Such a device and system is described in U.S. Patent No. 6,763,264. Alternatively, a single needle device can be used that allows injection of the DNA and electroporation with a single needle resembling a normal injection needle and applies pulses of lower voltage than those delivered by presently used devices, thus reducing the electrical sensation experienced by the patient.

The MID can comprise one or more electrode arrays. The arrays can comprise two or more needles of the same diameter or different diameters. The needles can be evenly or unevenly spaced apart. The needles can be between 0.005 inches and 0.03 inches, between 0.01 inches and 0.025 inches; or between 0.015 inches and 0.020 inches. The needle can be 0.0175 inches in diameter. The needles can be 0.5 mm, 1.0 mm, 1.5 mm, 2.0 mm, 2.5 mm, 3.0 mm, 3.5 mm, 4.0 mm, or more spaced apart.

The MID can consist of a pulse generator and a two or more-needle vaccine injectors that deliver the vaccine and electroporation pulses in a single step. The pulse generator can allow for flexible programming of pulse and injection parameters via a flash card operated personal computer, as well as comprehensive recording and storage of electroporation and patient data. The pulse generator can deliver a variety of volt pulses during short periods of time. For example, the pulse generator can deliver three 15 volt pulses of 100 ms in duration. An example of such a MID is the Elgen 1000 system by Inovio Pharmaceuticals, which is described in U.S. Patent No. 7,328,064,.

The MID can be a CELLECTRA (Inovio Pharmaceuticals) device and system, which is a modular electrode system, that facilitates the introduction of a macromolecule, such as a DNA, into cells of a selected tissue in a body or plant. The modular electrode system can comprise a plurality of needle electrodes; a hypodermic needle; an electrical connector that provides a conductive link from a programmable constant-current pulse controller to the plurality of needle electrodes; and a power source. An operator can grasp the plurality of needle electrodes that are mounted on a support structure and firmly insert them into the selected tissue in a body or plant. The macromolecules are then delivered via the hypodermic needle into the selected tissue. The programmable constant-current pulse controller is activated and constant-current electrical pulse is applied to the plurality of needle electrodes. The applied constant-current electrical pulse facilitates the introduction of the macromolecule into the cell between the plurality of electrodes. Cell death due to overheating of cells is minimized by limiting the power dissipation in the tissue by virtue of constant-current pulses. The Cellectra device and system is described in U.S. Patent No. 7,245,963.

The Elgen 1000 system can comprise device that provides a hollow needle; and fluid delivery means, wherein the apparatus is adapted to actuate the fluid delivery means in use so as to concurrently (for example, automatically) inject fluid, the described vaccine herein, into body tissue during insertion of the needle into the said body tissue. The advantage is the ability to inject the fluid gradually while the needle is being inserted leads to a more even distribution of the fluid through the body tissue. It is also believed that the pain experienced during injection is reduced due to the distribution of the volume of fluid being injected over a larger area.

In addition, the automatic injection of fluid facilitates automatic monitoring and registration of an actual dose of fluid injected. This data can be stored by a control unit for documentation purposes if desired.

It will be appreciated that the rate of injection could be either linear or non-linear and that the injection can be carried out after the needles have been inserted through the skin of the subject to be treated and while they are inserted further into the body tissue.

Suitable tissues into which fluid can be injected by the apparatus of the present disclosure include tumor tissue, skin or liver tissue but can be muscle tissue.

The apparatus can further comprise a needle insertion means for guiding insertion of the needle into the body tissue. The rate of fluid injection is controlled by the rate of needle insertion. This has the advantage that both the needle insertion and injection of fluid can be controlled such that the rate of insertion can be matched to the rate of injection as desired. It also makes the apparatus easier for a user to operate. If desired means for automatically inserting the needle into body tissue could be provided.

A user could choose when to commence injection of fluid. Ideally however, injection is commenced when the tip of the needle has reached muscle tissue and the apparatus can include means for sensing when the needle has been inserted to a sufficient depth for injection of the fluid to commence. This means that injection of fluid can be prompted to commence automatically when the needle has reached a desired depth (which will normally be the depth at which muscle tissue begins). The depth at which muscle tissue begins could for example be taken to be a preset needle insertion depth such as a value of 4 mm which would be deemed sufficient for the needle to get through the skin layer.

The sensing means can comprise an ultrasound probe. The sensing means can comprise a means for sensing a change in impedance or resistance. In this case, the means can not as such record the depth of the needle in the body tissue but will rather be adapted to sense a change in impedance or resistance as the needle moves from a different type of body tissue into muscle. Either of these alternatives provides a relatively accurate and simple to operate means of sensing that injection can commence. The depth of insertion of the needle can further be recorded if desired and could be used to control injection of fluid such that the volume of fluid to be injected is determined as the depth of needle insertion is being recorded.

The apparatus can further comprise: a base for supporting the needle; and a housing for receiving the base therein, wherein the base is moveable relative to the housing such that the needle is retracted within the housing when the base is in a first rearward position relative to the housing and the needle extends out of the housing when the base is in a second forward position within the housing. This is advantageous for a user as the housing can be lined up on the skin of a patient, and the needles can then be inserted into the patient's skin by moving the housing relative to the base.

As stated above, it is desirable to achieve a controlled rate of fluid injection such that the fluid is evenly distributed over the length of the needle as it is inserted into the skin. The fluid delivery means comprise piston driving means adapted to inject fluid at a controlled rate. The piston driving means could for example be activated by a servo motor. The piston driving means can be actuated by the base being moved in the axial direction relative to the housing. It will be appreciated that alternative means for fluid delivery could be provided. Thus, for example, a closed container which can be squeezed for fluid delivery at a controlled or non-controlled rate could be provided in the place of a syringe and piston system.

The apparatus described above could be used for any type of injection. It is however envisaged to be particularly useful in the field of electroporation and so it can further comprise a means for applying a voltage to the needle. This allows the needle to be used not only for injection but also as an electrode during, electroporation. This is particularly advantageous as it means that the electric field is applied to the same area as the injected fluid. There has traditionally been a problem with electroporation in that it is very difficult to accurately align an electrode with previously injected fluid and so user's have tended to inject a larger volume of fluid than is required over a larger area and to apply an electric field over a higher area to attempt to guarantee an overlap between the injected substance and the electric field. Using the present invention, both the volume of fluid injected and the size of electric field applied can be reduced while achieving a good fit between the electric field and the fluid.

### 4. Vaccinating Against RSV Infection

The present invention is also directed to vaccines as described herein for use in methods for vaccinating a subject against RSV infection. The vaccine can include an RSV antigenic peptide and a nucleic acid encoding the RSV antigenic peptide. The vaccine can stimulate iTreg cells, including but not limited to CD4⁺, CD25⁻, FoxP3⁺, IL-10⁺, which in turn generate an antigen-specific response. The iTreg stimulated cells can generate high levels of IL-10, which stimulates B cells for neutralizing antibody production

### 5. Inducing Neutralizing Antibody Against RSV Infection and Suppressing Inflammatory T cells

The present invention is also directed to vaccines as described herein for use in methods for inducing neutralizing antibody against RSV infection and suppressing inflammatory T cells in a subject. The vaccine can include an RSV antigenic peptide and a nucleic acid encoding the RSV antigenic peptide. The vaccine can stimulate iTreg cells, including but not limited to CD4⁺, CD25⁻, FoxP3⁺, IL-10⁺. iTreg stimulated cells can generate high levels of IL-10, which stimulates B cells for neutralizing antibody production. Suppressing inflammatory T cell includes inducing iTreg cells.

The method takes advantage of the antigen-specific immune response generated by coimmunizing a subject with both an RSV antigen and a nucleic acid encoding the same RSV antigen, as discussed above.

### 6. Suppressing Auto-Reactive CD4+ T cell Induction after RSV Challenge

The present invention is also directed to vaccines as described herein for use in methods for suppressing auto-reactive CD4+ T cell induction after RSV challenge in a subject. The vaccine can include an RSV antigenic peptide and a nucleic acid encoding the RSV antigenic peptide. The vaccine can stimulate iTreg cells, including but not limited to CD4⁺, CD25⁻, FoxP3⁺, IL-10⁺. iTreg stimulated cells can generate high levels of IL-10, which stimulates B cells for neutralizing antibody production. Suppressing inflammatory T cell includes suppressing auto-reactive CD4+ and CD8+ T cells.

### 7. Ameliorating Vaccine-Induced Disease (VID)

The present invention is also directed to vaccines as described herein for use in methods of ameliorating VID. The vaccine can include an RSV antigenic peptide and a nucleic acid encoding the RSV antigenic peptide. The vaccine can stimulate iTreg cells, including but not limited to CD4⁺, CD25⁻, FoxP3⁺, IL-10⁺. iTreg stimulated cells can generate high levels of IL-10, which stimulates B cells for neutralizing antibody production. The subject can be immunized with formalin-inactivated RSV (FI-RSV) or RSV antigen prior to encounter with natural RSV infection.

### 8. Protecting a Subject from Airway Hyper-responsiveness (AHR) after RSV Challenge

The present invention is also directed to vaccines as described herein for use in methods for protecting a subject from airway hyper-responsiveness (AHR) after RSV challenge. The vaccine can include an RSV antigenic peptide and a nucleic acid encoding the RSV antigenic peptide. The vaccine can stimulate iTreg cells, including but not limited to CD4⁺, CD25⁻, FoxP3⁺, IL-10⁺. iTreg stimulated cells can generate high levels of IL-10, which stimulates B cells for neutralizing antibody production. The subject can be immunized with formalin-inactivated RSV (FI-RSV) or RSV antigen prior to encounter with natural RSV infection. The method takes advantage of the antigen-specific immune response generated by coimmunizing a subject with both an RSV antigen and a nucleic acid encoding the same RSV antigen, as discussed above.

RSV can trigger AHR in a subject previously sensitized to RSV. Sensitization to RSV refers to being previously exposed one or more times to RSV such that an immune response is developed against RSV. Responses associated with an allergic reaction (e.g., histamine release, rhinitis, edema, vasodilation, bronchial constriction, airway inflammation), typically do not occur when a naive individual is exposed to RSV for the first time, but once a cellular and humoral immune response is produced against RSV, the individual is "sensitized" to RSV. AHR or airway obstruction can occur when the sensitized subject is re-exposed to RSV. Once a subject is sensitized to RSV, the allergic reactions can become worse with each subsequent exposure to RSV, because each re-exposure not only produces allergic symptoms, but further increases the level of antibody produced against RSV and the level of T cell response against RSV.

A subject that is at risk of developing airway hyperresponsiveness is a subject that has been exposed to, or is at risk of being exposed to, RSV that is sufficient to trigger AHR, but does not yet display a measurable or detectable characteristic or symptom of AHR. A mammal that is at risk of developing RSV-induced AHR is a mammal that has been previously sensitized to RSV, and that has been exposed to, or is at risk of being exposed to, an amount of RSV that is sufficient to trigger AHR (i.e., a triggering, or challenge dose of RSV), but does not yet display a measurable or detectable characteristic or symptom of AHR.

Inflammation is typically characterized by the release of inflammatory mediators (e.g., cytokines or chemokines) which recruit cells involved in inflammation to a tissue. AHR after RSV challenge involves the elicitation of one type of immune response (e.g., a Th2-type immune response) against RSV, which can result in the release of inflammatory mediators that recruit cells involved in inflammation in a mammal, the presence of which can lead to tissue damage and sometimes death. A Th2-type immune response is characterized in part by the release of cytokines which include IL-4, IL-5 and IL-13.

### 9. Combined Therapies

As used herein, the term "in combination" in the context of the administration of other therapies refers to the use of more than one therapy. The use of the term "in combination" does not restrict the order in which therapies are administered to a subject with an infection. A first therapy can be administered before (e.g., 1 minute, 45 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks), concurrently, or after (e.g., 1 minute, 45 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks) the administration of a second therapy to a subject which had, has, or is susceptible to a RSV infection, otitis media or a respiratory condition related thereto. Any additional therapy can be administered in any order with the other additional therapies. In certain embodiments, the vaccines of the invention can be administered in combination with one or more therapies (e.g., therapies that are not the vaccines of the invention that are currently administered to prevent, treat, manage, and/or ameliorate a RSV infection (e.g., acute RSV disease or a RSV URI and/or LRI, otitis media, and/or a symptom or respiratory condition or other symptom related thereto).

Non-limiting examples of therapies that can be administered in combination with a vaccine of the invention include at least one of any suitable and effective amount of a composition or pharmaceutical composition comprising at least one RSV antibody to a cell, tissue, organ, animal or patient in need of such modulation, treatment or therapy, optionally further comprising at least one selected from at least one TNF antagonist (e.g., but not limited to a TNF antibody or fragment, a soluble TNF receptor or fragment, fusion proteins thereof, or a small molecule TNF antagonist), an antirheumatic (e.g., methotrexate, auranofin, aurothioglucose, azathioprine, etanercept, gold sodium thiomalate, hydroxychloroquine sulfate, leflunomide, sulfasalzine), a muscle relaxant, a narcotic, a non-steroid inflammatory drug (NSAID), an analgesic, an anesthetic, a sedative, a local anesthetic, a neuromuscular blocker, an antimicrobial (e.g., aminoglycoside, an antifungal, an antiparasitic, an antiviral, a carbapenem, cephalosporin, a flurorquinolone, a macrolide, a penicillin, a sulfonamide, a tetracycline, another antimicrobial), an antipsoriatic, a corticosteriod, an anabolic steroid, a diabetes related agent, a mineral, a nutritional, a thyroid agent, a vitamin, a calcium related hormone, an antidiarrheal, an antitussive, an antiemetic, an antiulcer, a laxative, an anticoagulant, an erythropoeitin (e.g., epoetin alpha), a filgrastim (e.g., G-CSF, Neupogen), a sargramostim (GM-CSF, Leukine), an immunization, an immunoglobulin, an immunosuppressive (e.g., basiliximab, cyclosporine, daclizumab), a growth hormone, a hormone replacement drug, an estrogen receptor modulator, a mydriatic, a cycloplegic, an alkylating agent, an antimetabolite, a mitotic inhibitor, a radiopharmaceutical, an antidepressant, antimanic agent, an antipsychotic, an anxiolytic, a hypnotic, a sympathomimetic, a stimulant, donepezil, tacrine, an asthma medication, a beta agonist, an inhaled steroid, a leukotriene inhibitor, a methylxanthine, a cromolyn, an epinephrine or analog, dornase alpha (Pulmozyme), a cytokine or a cytokine antagonist, or any other agent listed in the U.S. Pharmacopoeia and/or Physician's Desk Reference. Non-limiting examples of such cytokines include, but are not limited to, any of IL-1 to IL-23. Suitable dosages are well known in the art. See, e.g., Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000).

### 10. Kit

Provided herein is a kit, which can be used for vaccinating a subject. The kit can comprise a vaccine, which includes an antigenic peptide and a DNA encoding the antigenic peptide, and a MID. The kit can further comprise instructions for using the kit and conducting the analysis, monitoring, or treatment.

The kit can also comprise one or more containers, such as vials or bottles, with each container containing a separate reagent. The kit can further comprise written instructions, which can describe how to perform or interpret an analysis, monitoring, treatment, or method described herein. The kit can further comprise a vaccine administration device. The vaccine administration device can be a vaccine gun, a needle for administering the vaccine, or a electroporation device linked to a conduit for delivering the vaccine.

The present invention has multiple aspects, illustrated by the following non-limiting examples.

### EXAMPLES

### EXAMPLE 1

### MATERIALS AND METHODS

The following is a description of the materials and methods used in the below-identified Examples 2-8.

With respect to the animals and cells, female Balb/c mice aged 6-8 weeks were purchased from the Animal Institute of the Chinese Medical Academy (Beijing, China), cared for under a 12-hour light cycle, and fed with pathogen-free food and water. All animal protocols were approved by the Animal Welfare Committee of China Agricultural University. NIH/3T3 cell line (American Type Culture Collection (ATCC), Rockville, MD, USA CCL-1658) and HEp-2 (ATCC, CCL-23) cells were maintained in Dulbecco's modified Eagle's medium (DMEM) (Gibco/BRL, NY, USA) supplemented with 10% fetal calf serum (FCS) and penicillin/streptomycin (Gibco/BRL) in a humidified incubator set at 37°C and 5% CO₂.

With respect to the bacterial strains and plasmids, *E. coli* TOP10 (TIANGEN Biotech LTD, Beijing, China) was used as the host strain for manipulation and *E. coli* BL21 (DE3) (TIANGEN) was used as the expression strain. Eukaryotic expression vector proVAX was previously constructed in this laboratory with a cytomegalovirus (CMV) promoter and an hCG-β leader sequence (Du et al. The Journal of Gene Medicine 9: 136-146 (2007)). Prokaryotic expression vector pET28a(+) (Novagen Inc, WI, USA) contains the T7 promoter and allows expression of recombinant protein fused to a polyhistidine-tag. All bacterial cultures were carried out in shake-flasks using Luria-Bertani medium (Sangon Biological Engineering Technology & Services Co., Ltd., Shanghai, China) supplemented with kanamycin where appropriate. Protein expression was induced by Isopropyl β-D-1-thiogalactopyranoside (IPTG) purchased from Invitrogen (CA, USA).

With respect to plasmid construction and eukaryotic expression, a gene encoding an optimized version of amino acids 67-298 of RSV G glycoprotein was used (full length RSV G nucleotide sequence corresponds to position 4687-5583 of GenBank: AY911262.1 (RSV genome; SEQ ID NO: 6); RSV G glycoprotein amino acid sequence (GenBank AAX23993; SEQ ID NO: 2); RSV G glycoprotein nucleotide sequence (SEQ ID NO: 19)). The nucleotide sequence (SEQ ID NO: 20) of optimized amino acid RSV G amino acid sequence was mouse-codon optimized (SEQ ID NO: 22) and synthesized by outsourcing (Sangon, Shanghai, China). Codon optimization not only eliminated the rare codons of the G fragment, but also removed the 66-amino acid transmembrane domain of the N-terminus (Ghildyal et al. Journal of General Virology 86: 1879-1884 (2005)). The codon-optimized gene was subcloned into a eukaryotic expression vector, proVAX, at *EcoR* I and *Xho* I restriction sites and designated as proVAX/G. Its correctness was confirmed by restriction digestions and sequencing. Purified plasmid was transfected into NIH/3T3 cells with Lipofectamine according to the manufacturer's instructions (Invitrogen, CA, USA). The transfectants were harvested after 48 h and total cellular RNA was extracted according to a previously described protocol (Jin et al. Vaccine 22: 2925-2935 (2004)). The expression of the gene of interest was detected by reverse-transcription polymerase chain reaction (RT-PCR) with specific primer sets: forward primer, 5'-ATGCATAAGGTGACTCCT-3' (SEQ ID NO: 7), reverse primer, 5'-TTACTGCCGTGGGGTGTT-3' (SEQ ID NO: 8).

A gene encoding an optimized version of amino acids 412-524 of RSV F fusion protein was used (full length RSV F fusion protein nucleotide sequence corresponds to 5660-7384 of GenBank: AY911262.1 (SEQ ID NO: 6) RSV F fusion protein amino acid sequence (GenBank AAX23994.1; SEQ ID NO: 1); RSV F fusion protein nucleotide sequence (SEQ ID NO: 26)). The nucleotide sequence (SEQ ID NO: 23) of the optimized amino acid RSV F amino acid sequence was codon optimized for prokaryotic (*i.e., E. coli*) expression (SEQ ID NO: 24).

With respect to plasmid construction and prokaryotic expression, a prokaryotic codon-optimized gene encoding amino acids at 67-298 of RSV G glycoprotein (SEQ ID NO: 21) was subcloned into the prokaryotic expressing vector by a similar procedure and designated as pET28a(+)/G. The prokaryotic codon-optimized gene encoding amino acids at 412-524 of RSV F (SEQ ID NO: 24) was subcloned into the prokaryotic expressing vector by a similar procedure and designated as pET28a(+)/F. Its correctness was confirmed by restriction digestions and sequencing. *E. coli* BL21 (DE3) competent cells were transformed with pET28a(+)/G by a standard method (Molecular cloning: A laboratory manual, 2nd edn : by J. Sambrook, E. F. Fritsch and T. Maniatis, Cold Spring Harbor Laboratory Press, 1989) and protein expression was induced with 0.5 mmol/L IPTG at 37°C. After 4 h, cells were collected, washed and resuspended in PBS. The soluble fraction and the insoluble fraction containing inclusion bodies were isolated from the cells after sonication. Recombinant protein was purified by passing through a Ni-NTA Superflow column (QIAGEN GmbH, Hilden, Germany) on a Biologic DuoFlowTM Chromatography System (Bio-Rad, CA, USA) according to the manufacturer's manual. The purified protein was analyzed by 12% SDS-PAGE.

With respect to western blot analysis, protein samples were subjected to electrophoresis on 12% SDS-PAGE gel and transferred onto nitrocellulose membrane using a Bio-Rad transblot apparatus (Bio-Rad, CA, USA). After overnight blocking in PBST mixed with 2% BSA, the membrane was incubated with goat anti-RSV antiserum (Meridian, Me, USA) at a dilution of 1:2000 for 2 h at 4°C. The membrane was washed three times with 150 mM PBST and then incubated with bovine anti-goat IgG-HRP conjugate (Santa Cruz, CA, USA) in blocking buffer (PBST mixed with 2% BSA) at a dilution of 1:1000 for 1 h at RT. Immune complexes were detected by the ECL method according to the manufacturer's instruction (GE Healthcare Europe, Uppsala, Sweden).

With respect to RSV stock preparation, the RSV Long strain was obtained from ATCC (catalog no. VR-26) and propagated in HEp-2 cells at 37°C in a humidified atmosphere with 5% CO₂. The viruses were added to the cells in DMEM at a multiplicity of infection (m.o.i.) of 4:1. After 1 h, DMEM with 2% FCS was added to the flask and infection of cells was monitored for 3-4 days. RSV was harvested by centrifugation at 3,000 × g at 4°C to remove cellular debris, aliquoted, and stored at -80°C until use.

Formalin-inactivated RSV (FI-RSV) was prepared as described by Kim et al. American Journal of Epidemiology 89: 422-434 (1969). Briefly, 1 part formalin (approximately 37% - 40%) was incubated with 4,000 parts clarified virus lysate for 3 days at 37°C and pelleted by centrifugation for 1 h at 50,000 × g. The virus was diluted 1:25 in minimum essential medium (MEM) and subsequently precipitated with aluminum hydroxide (4 mg/ml), resuspended in 1/100 of the original volume in serum-free MEM, and stored at 4°C.

With respect to immunization and challenge of mice, plasmid DNA for immunization was isolated using the EndoFree Plasmid Giga kit (QIAGEN, Hilden, Germany) following the manufacturer's recommendations. Both plasmids and recombinant proteins were dissolved in PBS. For vaccination, the mice were randomly divided into groups of 9 animals per group as listed in Table 1. Mice were immunized intramuscularly with one of FI-RSV, plasmid, plasmid plus protein or saline solution on days 0, 14 and 28. The mice were bled before and after immunization at 2-week intervals. Mice were challenged intranasally with RSV (10⁶ TCID₅₀ in 50 µl) on day 14 after the last immunization. Mice were weighed daily following the challenge. The data were expressed as the percentages of the base weights at day 0 of challenge. Mice were sacrificed 5 days later and assayed for lung virus titers and leukocyte infiltration in bronchoalveolar fluids.

**Table 1. Immunization groups**

| Group | Vaccine |
|---|---|
| 1 | Naïve |
| 2 | FI-RSV |
| 3 | 100 µl saline solution |
| 4 | 100 µg proVAX/G |
| 5 | 100 µg His-G |
| 6 | 100 µg proVAX +100 µg His-G |
| 7 | 100 µg proVAX/G +100 µg OVA |
| 8 | 100 µg proVAX/G +100 µg His-G |
| 9 | 100 µg proVAX/F |
| 10 | 100 µg His-F |
| 11 | 100 µg proVAX +100 µg His-F |
| 12 | 100 µg proVAX/F +100 µg OVA |
| 13 | 100 µg proVAX/F +100 µg His-F |

With respect to the assay of antibodies, antibodies against virus were assayed by enzyme-linked immunosorbent assay (ELISA). The 96-well microtiter plates were coated overnight with UV- inactivated RSV in 0.05 M bicarbonate buffer (pH 9.6), 100 µl per well at 4°C. The plates were blocked with 5% BSA-PBST at 37°C for 1 h, washed, and incubated with serially diluted serum for 1 h at 37°C. The secondary goat anti-mouse IgG conjugated with horseradish peroxidase (Bio-Rad Laboratories, Hercules, CA, USA) was diluted 1:1000, added to each well and incubated at 37°C for 1 h. Ten milligrams of TMB tablet was dissolved in 0.025 M phosphate-citrate buffer and added to each well for color development. After addition of 2 M H₂SO₄ the plate was read with a plate reader (Magellan; Tecan Group, Maennedorf, Switzerland) at 450 nm. Antibody titers were expressed as an absolute ratio of post/naive serum at a cutoff of 2.1.

With respect to the virus neutralization assay, the virus neutralization assay was performed as previously described with modifications (Singh et al. Vaccine 25: 6211-6223 (2007); Anderson et al. J Clin Microbiol 22: 1050-1052 (1985)). In brief, sera were serially diluted two-fold in a total of 100 µl PBS, heat inactivated at 56°C for 30 min and incubated with 3 × 10³ TCID₅₀ of virus for 2 h at 4°C. Approximately, 5×10⁴ HEp-2 cells in 100 µl DMEM supplemented with 2% FCS were added to each well of a 96-well microtiter plate. The virus-serum mixture was added to the appropriate wells and incubated for 3 days in a 5% CO₂ incubator at 37°C. Plates were then washed three times with 0.05% Tween-20 in PBS and fixed with 80% cold acetone in PBS followed by blocking with 3% blocking buffer. Goat anti-RSV antibody (Meridian, ME, USA) was added to the appropriate wells and incubated for 60 min at 37°C. After three washings, bovine anti-goat IgG-HRP (Santa Cruz, CA, USA) was added, the enzymatic reaction was developed from the average ODs were read at 450 nm/630 nm. The neutralization titer was calculated from the average OD of the wells by extrapolating the inverse of the serum dilution that resulted in 50% reduction of RSV activity.

With respect to virus quantification, lungs were harvested from immunized mice on day 5 post-RSV challenge, homogenized and then centrifuged at 2000 rpm for 10 min. Cell-free supernatants from these samples were snap-frozen in liquid nitrogen and virus was titrated in thawed samples. Briefly, serial log₁₀ dilutions of each test sample in 2% FCS-DMEM were added to microtiter plates containing 3 × 10³ HEp-2 cells/well and incubated at 37°C with 5% CO₂. On Day 5, the wells were scored by visual inspection for the formation of syncytia. Endpoints were calculated by Karber's method. The amount of virus present in each suspension was expressed as the geometric mean virus titer (GMT; log₁₀ TCID₅₀/gram).

With respect to flow cytometric analysis, T cells were isolated and stained with phycoerythrin-(PE-), fluorescein isothiocyanate- (FITC-) or allophycocyanin- (APC-) conjugated mAbs in PBS for 30 min at 4°C. For intracellular staining, T cells were stimulated *in vitro* with G protein (10 µg/ml) for 8 h and subsequently treated with monensin (100 µg/ml) for 2 h. The cells were blocked with Fc-Block (BD Pharmingen, San Diego, USA) in PBS for 30 min at 4°C before being fixed with 4% paraformaldehyde and permeabilized with saponin. The cells were intracellularly stained for 30 min at 4°C with the appropriate concentrations of antibodies, including APC-labeled anti-FoxP3 or PECy5-labeled anti-CD25 antibody, FITC-labeled anti-CD4 antibody or PE-labeled anti-IL-10 antibody (BD Pharmingen, San Diego, USA). The cells were washed and analyzed with a FACScalibur using the Cell QuestPro Software (BD Bioscience, San Jose, USA).

With respect to the T cell proliferation assay, spleens were removed from immunized mice on day 7 after the last immunization and used to prepare single T cell suspensions (Tim. Journal of Immunological Methods 65: 55-63 (1983)). Single lymphocyte suspensions were incubated in triplicate in 96-well plates at 5 × 10⁴ cells/well, in RPMI-1640 plus 5% FCS at 37°C in a 5% CO₂ incubator and stimulated for 48 h with 0.1 µg/ml of phorbol myristate acetate (PMA) plus 1 ng/ml Ionomycin (Ion) as positive control, 10 µg/ml UV-irradiated RSV antigen as specific antigen, 2 µg/ml bovine serum albumin (BSA) as irrelevant antigen, or no antigen as negative control. T cell proliferation was evaluated using the MTT method and OD values of the plates were read at 570 nm by a plate reader (Magellan; Tecan Austria GmbH) after 4 h of incubation with MTT (Wang et al. Vaccine 18: 1227-1235 (2000)). Data were expressed as the stimulation index (SI), calculated as the mean reading of triplicate wells stimulated with an antigen, divided by the mean reading of triplicate wells stimulated with medium.

With respect to cell isolation and adoptive transfer, single splenocyte suspensions were prepared from mouse spleen. CD4⁺CD25⁺ or CD4⁺CD25⁻ T cells were isolated and purified by using the MagCellect Mouse CD4⁺CD25⁺+ T Cell Isolation Kit according to the manufacturer's protocol (R&D Systems, Inc., Minneapolis, MN, USA). The resulting, CD4⁺CD25⁺, and CD4⁺CD25⁻ T cell suspensions were >90% pure as determined by flow cytometry (FACSCalibur, BD Bioscience, San Jose, USA). The CD4⁺CD25⁻ at 4 × 10⁵/mouse (about 6 × 10⁴ iTreg cells) or CD4⁺CD25⁺ at 2 × 10⁵/mouse (about 1.8 × 10⁵ nTreg cells) were adoptively transferred intravenously into mice.

With respect to the mixed lymphocyte reaction, the CD4⁺CD25⁺ or CD4⁺CD25⁻ T cell populations were purified from Balb/c mice as described under cell isolation above and used as the responder cells. Stimulator cells were isolated from the spleen of naive C57BL/6 mice by panning using anti-CD3 monoclonal antibody (eBioscience, CA, USA) to delete T cells and were further treated with mitomycin C before use. The responder and stimulator cells were mixed at various ratios, from 1:1, 1:3 to 1:10 (Balb/c responder : C57BL/6 stimulator), seeded at 2 × 10⁵ total cells per well in triplicate wells, and cultured for 48 h. The response was measured by the MTS/PMS colorimetric assay (Kang et al. Vaccine 23: 5543-5550 (2005)) using OD values read at 490 nm on a plate reader (Magellan; Tecan Group, Maennedorf, Switzerland).

With respect to real-time PCR, total mRNA was extracted from lungs samples that were isolated from immunized mice on day 5 post-RSV challenge. cDNA was synthesized by reverse-transcription PCR and SYBR Green incorporation during quantitative Real-time PCR was measured using a Fast Start SYBR Green mix (Roche) in the ABI7400 Sequence Detection System (Applied Biosystems). The primers used are listed in Table 2.

**Table 2. Real-time PCR primers (5'-3')**

| Target genes | Forward | Reverse |
|---|---|---|
| IL-4 | ACAGGAGAAGGGACGCCAT (SEQ ID NO: 9) | GAAGCCCTACAGACGAGCTCA (SEQ ID NO: 10) |
| IL-5 | AGCACAGTGGTGAAAGAGACCTT (SEQ ID NO: 11) | TCCAATGCATAGCTGGTGATTT (SEQ ID NO: 12) |
| IL-13 | GGAGCTGAGCAACATCACACA (SEQ ID NO: 13) | GGTCCTGTAGATGGCATTGCA (SEQ ID NO: 14) |
| IFN-γ | TCAAGTGGCATAGATGTGGAAGAA (SEQ ID NO: 15) | TGGCTCTGCAGGATTTTCATG (SEQ ID NO: 16) |
| HPRT | CTGGTGAAAAGGACCTCTCG (SEQ ID NO: 17) | TGAAGTACTCATTATAGTCAAGGGCA (SEQ ID NO: 18) |

With respect to plethysmography, measurements of respiratory system dynamic resistance (Rrs) and compliance (Cldyn) were measured by placing mice in a whole-body plethysmograph (model AniRes2005; Beijing Bestlab Technology Co. Beijing, China) according to the manufacturer's manual. In brief, mice were anaesthetized 5 days after RSV challenge. Tracheotomy was performed and the trachea connected to the ventilator. Mechanical ventilation was carried out, dynamic airway pressure (*ΔP*) and volume of chamber (*ΔV*) were recorded, and peak resistance and compliance were automatically measured as Rrs = *ΔP*/ (*ΔV*/*ΔT*) and Clydn = *ΔV*/*ΔP* after each 200 µl intrajugular administration of various doses of acetylcholine chloride (mg) (Jin et al. European Journal of Immunology 38: 2451-2463 (2008)). With respect to the bronchoalveolar lavage (BAL) collection, five days after challenge, the mice were euthanized, a tracheotomy was performed, and the large airways were washed with 1 ml PBS containing 0.1% bovine serum albumin. The bronchoalveolar lavage (BAL) wash was centrifuged and the supernatant was removed. The BAL pellet was resuspended and the total cell count obtained by FACS. Cells were stained with Wright's Giemsa (Lichen Biotechnology Co., Ltd., Shanghai, China), and cell types were identified by morphological criteria. At least two hundred total cells were examined per slide for a differential count.

With respect to histopathology, lung tissues were fixed in buffered formalin, and transverse sections (thickness, 5 µm) were stained with hematoxylin and eosin. The histopathologic score (HPS) was based on grading of five different parameters: (i) peribronchiolar and bronchial infiltrates, (ii) bronchiolar and bronchial luminal exudates, (iii) perivascular infiltrates, (iv) amount of monocytes, and (v) parenchymal pneumonia. The HPS system assigned values from 0 to 21; the higher the score, the greater the inflammatory changes in the lung (Jafri et al. Journal of Infectious Diseases 189: 1856-1865 (2004); Mejías et al. Antimicrobial Agents and Chemotherapy 48: 1811-1822 (2004)). The HPS was determined by a pathologist who was unaware of the infection status of the animals from which specimens were taken.

With respect to statistical analysis, results are presented as means ± standard error of the mean (S.E.M.). Student's *t* and non-parametric test analysis were used for data analysis. A value of *p* < 0.05 was considered to be statistically significant.

### EXAMPLE 2

### Expression of DNA and protein vaccines

The eukaryotic expression constructs proVAX/G and proVAX/F were separately transfected into NIH/3T3 cells to confirm expression. Assay of G-specific mRNA by RT-PCR confirmed efficient expression (FIG. 1). The prokaryotic expression constructs pET28a(+)/G and pET28a(+)/F were transformed into *E. coli* BL21 (DE3) and the resultant recombinant protein was purified by Ni-NTA Superflow on a Biologic DuoFlowTM Chromatography System (pET28a(+)/G shown in FIGS. 2A-2B). The identity of the G protein and F protein produced was confirmed by Western blot using specific goat anti-RSV polyclonal antisera (the identity of the G protein shown in FIG. 3).

### EXAMPLE 3

### Antibody responses

Groups of seven Balb/c mice were immunized intramuscularly on days 0, 14, and 28 and the level of IgG antibody against virus was determined by ELISA 7 days after the last immunization. proVAX/G and His-G given separately were compared with proVAX/G plus His-G protein and with FI-RSV as a positive control. In addition, proVAX/F and His-F given separately were compared with proVAX/F plus His-F protein and with FI-RSV as a positive control. Additional control mice received proVAX (empty vector) plus His-G, proVAX (empty vector) plus His-F, proVAX/G plus an irrelevant protein (ovalbumin; OVA), proVAX/F plus an irrelevant protein (ovalbumin; OVA) or PBS. All immunized groups produced detectable IgG antibodies (positive: ODExp/ODPre > 2.1) except mice immunized with PBS. Although the highest level was achieved with FI-RSV immunized mice, groups immunized with proVAX/G + His-G, His-G alone or His-G + proVAX produced similar levels of antibody response. Groups immunized with proVAX/F + His-F, His-F alone or His-F + proVAX also produced an antibody response. The lowest levels were produced by proVAX/G, proVAX/G + OVA, proVAX/F or proVAX/F + OVA groups (FIGS. 4A-4B).

Studies in animals and humans had demonstrated that a higher level of neutralizing antibody correlated with a higher level of protection against RSV infection. Therefore the capability of the serum antibodies to neutralize RSV was assessed. The serum neutralization titer was determined as follows: pooled sera collected from immunized mice on day 7 after the last immunization were used to determine the RSV neutralization titer as described in Example 1. The lung RSV titer was determined as follows: lung samples from mice immunized with the indicated antigens and subsequently challenged with RSV (10⁶ TCID₅₀) were used to collect RSV. Viral titer was determined as described in Example 1 and discussed in Example 5. Data are presented from at least six replicates with standard error (Table 3). As shown in Table 3, the positive control, FI-RSV vaccination, provided the highest neutralizing antibody level; whereas antisera from His-G, proVAX+His-G, proVAX/G+His-G, His-F, proVAX+His-F, and proVAX/F+His-F immunized groups exhibited significant higher and similar levels of neutralizing antibodies. Thus, the level of binding antibody was approximately paralleled by the neutralizing activity.

**Table 3. Serum neutralization titer and lung RSV titer (log₁₀)**

| **Antigen** | **Serum neutralization titer** | **Virus titer (log₁₀TCID₅₀/gram of lung tissue)** |
|---|---|---|
| PBS | 0.418 ± 0.052 | 5.107 ± 0.0994 |
| FI-RSV | 3.710 ± 0.036 | 1.543 ± 0.1233 |
| proVAX/G | 1.867 ± 0.089 | 3.790 ± 0.2479 |
| proVAX/F | 1.667 ± 0.032 | 4.893 ± 0.3034 |
| His-G | 3.354 ± 0.026 | 2.217 ± 0.3005 |
| His-F | 2.911 ± 0.023 | 3.783 ± 0.1590 |
| proVAX/G+ His-G | 3.463 ± 0.042 | 1.850 ± 0.1041 |
| proVAX/F+ His-F | 3.089 ± 0.030 | 3.857 ± 0.2942 |
| proVAX+ His-G | 3.454 ± 0.038 | 2.023 ± 0.1468 |
| proVAX+ His-F | 3.085 ± 0.050 | 3.883 ± 0.2205 |
| proVAX/G+OVA | 1.935 ± 0.046 | 3.300 ± 0.2887 |
| proVAX/F+OVA | 1.634 ± 0.032 | 4.733 ± 0.2333 |

### EXAMPLE 4

### Suppression of auto-reactive CD4⁺ T cell by co-immunization with antigen-matched DNA and protein vaccines

Since severe VID upon RSV challenge is mediated by induction of autoreactive CD4⁺ T cells and co-immunization with antigen-matched DNA and protein vaccines has been shown to impair antigen-specific T cell and not antibody responses, T cell proliferative responses to UV-irradiated RSV antigen *in vitro* 7 days after the last immunizations were examined. The lowest level of proliferative response was observed in T cells isolated from mice co-immunized with proVAX/G+His-G, whereas strong proliferative responses were observed in the T cells isolated from the other immunized groups (FIG. 5) except for the PBS or BSA negative controls. The strong proliferative responses were observed in the T cells isolated from proVAX/G immunized group, showed that the cellular immune response was well elicited in the group. Co-immunizations with either proVAX/G+OVA, or His-G+proVAX vector as antigen-mismatched controls did not reveal any unrelated vector or protein influences on the response. This result shows that co-immunization with the DNA vaccine plus protein, proVAX/G+His-G, was unique in preferentially suppressing the T cell proliferative response.

### EXAMPLE 5

### Protection measured as reduction of viral load after RSV challenge

Two weeks following the final immunization, mice were challenged with RSV intranasally with 10⁶ TCID₅₀ (50 µl) per animal (*see* Table 3 and FIGS. 6A-6B). Virus titers were quantified in lung samples and in BALs collected 5 days after the challenge when the viral load peaked in the lungs. RSV replicated to significant titers at 5.107 ± 0.0994 log₁₀ TCID₅₀/gram of lung tissue in PBS control mice. Mice immunized with FI-RSV, His-G, proVAX+His-G, or proVAX/G+His-G exhibited dramatic reductions of viral titer compared to the PBS, proVAX/G and proVAX/G+OVA immunized groups (Table 3 and FIG. 6A). Mice immunized with His-F, proVAX+His-F, or proVAX/F+His-F also exhibited reductions of viral titer compared to the PBS, proVAX/F and proVAX/F+OVA immunized groups (Table 3 and FIG. 6B). The protection was apparently correlated with neutralizing antibody titers (Table 3).

### EXAMPLE 6

### Elimination of VID from the co-immunized mice

The ability of the co-immunization strategy to protect animals from airway hyper-responsiveness (AHR) after RSV challenge was examined. Cells were isolated from BALs, stained, and analyzed by microscopy to measure the amount of eosinophils, lymphocytes and monocytes present after RSV challenge. As shown in FIGS. 7A-7D, the infiltration of inflammatory cells was significantly less in the proVAX/G + His-G co-immunized group compared with the other immunized groups, indicating that co-immunization with RSV G antigens protects mice from RSV infection and ameliorates pulmonary inflammatory response. Similarly, the infiltration of inflammatory cells was less in proVAX/F+His-F co-immunized group compared with the other immununized group. (FIGS. 8A-8D). The lungs of groups immunized with FI-RSV or His-G or proVAX+His-G exhibited massive infiltrations after the RSV challenge.

Due to the infiltration, RSV infected mice developed significant airway obstruction. To assess this degree of airway obstructions among the groups, whole-body plethysmograpy was performed during stimulation with various doses of acetylcholine chloride by intra-jugular administration five days after RSV challenge. As shown in FIGS. 9A-9B, the least values of Rrs and Cldyn in response to the acetylcholine stimulation were seen in the proVAX/G + His-G co-immunized group; whereas, Rrs and Cldyn values were significantly increased in the mice immunized with FI-RSV or His-G or proVAX+His-G compared with PBS controls. This result showed that airway obstruction was ameliorated by the proVAX/G+His-G co-immunizations. Similar results were seen for proVAX/F+His-F co-immunized group (FIGS. 9C-9D).

The amelioration was further assessed via the histopathological analysis of lung sections. The PBS control group displayed histopathological features of RSV infection 5 days post-challenge (FIGS. 10A-10H, FIGS. 11A-11H, and FIGS. 12A-12E). RSV replication resulted in marked lung inflammation with a dense lymphocytic infiltrate. The intense lymphocytic infiltration was noted in both the perivascular and peribronchial areas. Compared with mice immunized with proVAX/G, proVAX/G+OVA or PBS, mice immunized with FI-RSV or His-G or proVAX+His-G gave significantly higher histopathologic scores (HPS) (FIG. 13), with dense peribronchial and perivascular circumferential infiltrates and severe pneumonia (FIGS. 10A-10H). However, mice immunized with proVAX/G+His-G showed significantly less pulmonary inflammatory response and lung HPS values were significantly lower than in the other groups. These results further demonstrated that co-immunization with proVAX/G+His-G or proVAX/F+His-F can ameliorate the pulmonary histopathogensis of RSV infection.

Besides impairing respiratory function, RSV infection can greatly affect body weight. After challenge with live RSV, the mice were weighed daily. All immunization groups had an early onset (day 2) of weight loss relative to the first day after challenge, however the proVAX/G+His-G co-immunized groups exhibited statistically less weight loss than other groups at days 5 to 7 postchallenge (FIG. 14A, *p* < 0.05). In contrast, at days 8 to 9 after challenge, mice immunized with FI-RSV or His-G or proVAX+His-G had greater weight loss than other groups. Hence, co-immunization with proVAX/G+His-G can substantially reduce the severity of illness after live RSV challenge. Similar results were seen for proVAX/F+His-F co-immunized groups (FIG. 14B).

FI-RSV-induced VID was previously demonstrated to be associated with activation of a Th2 type response. To examine if cytokine profiles were affected by the co-immunizing regimens, qPCR was used to assay the levels of cytokines in lung tissues on day 5 after RSV challenge. As shown in FIGS. 15A-15D, the levels of nearly all cytokines tested in the His-G or proVAX+His-G-immunized groups were similar to those in the FI-RSV immunized group; whereas, mice co-immunized with proVAX/G+His-G produced the lowest levels of these cytokines. This shows that both Th1 and Th2 responses were suppressed by the co-immunization. Mice immunized with proVAX/G or proVAX/G+OVA showed higher levels of IFN-γ expression, suggesting that the DNA vaccination could elicit immunity polarized towards Th 1.

### EXAMPLE 7

### RSV-G specific iTreg cells impair T cell responses

It was previously demonstrated that the impaired T cell response induced by co-immunization with DNA and protein vaccines was due to induction of CD4⁺CD25⁻IL-10⁺FoxP3⁺ iTreg cells. To determine if the impaired T cell responses and skewed cytokine expressions observed here in the co- immunized group were due to the induction of iTreg cells, T cell phenotypes were analyzed by FACS 7 days after the last co-immunization. The results showed that the population of CD4⁺CD25⁻IL-10⁺FoxP3⁺ iTreg cells in spleen was indeed induced at a significantly higher level (p<0.001) in the mice co-immunized with proVAX/G+His-G compared with the other groups (FIGS. 16A-16N and FIGS. 17A-17B). In contrast, as a nTreg control, CD4⁺CD25⁺ T cells from each group highly expressed both FoxP3 and IL-10, but there was no significant difference in their concentration between the groups.

CD4⁺CD25⁻ iTreg cells were adoptively transferred to test their role in the amelioration of VID observed *in vivo.* Naïve Balb/c mice received CD4⁺CD25⁻ iTreg cells obtained from co-immunized mice. Controls received CD4⁺CD25⁺ cells as a nTreg population prepared from naive mice. The recipient mice were then immunized against His-G antigen and their responses to this immunization were assessed in T cell proliferation assays and MLR in vitro. As shown in FIG. 18, splenocytes from mice that had received CD4⁺CD25⁻ iTreg cells isolated from Balb/c mice that had been co-immunized with proVAX/G+His-G significantly inhibited T responder cell-recalled immune responses. The same splenocytes also inhibited proliferation in response to allogeneic APCs (FIG. 19), while the corresponding CD4⁺CD25⁻ cells from naive donors did not inhibit proliferation. Splenocytes from mice that had received CD4⁺CD25⁺ nTreg cells from immunized mice, and cells from those mice that had received CD4⁺CD25⁺ nTreg cells from naïve mice could suppress T cell proliferation equally well, suggesting a nonspecific suppression by the transferred nTreg cells. These results indicated that co- immunization with proVAX/G+His-G could induce the CD4⁺CD25⁻ IL-10⁺FoxP3⁺ iTreg cells that impair T cell response in an antigen dependent manner.

### EXAMPLE 8

### Adoptive transfer of CD4⁺CD25⁻ T cells arrests Ag-specific inflammation

CD4⁺CD25⁻ iTreg and CD4⁺CD25⁺ nTreg cells were purified from mice co-immunized with proVAX/G+His-G or proVAX/G+OVA (antigen-mismatched control) and adoptively transferred intravenously into mice that had been previously immunized with His-G. The mice were challenged with RSV infection 7 days later. Lymphocytic infiltration was significantly decreased in mice receiving the CD4⁺CD25⁻ iTreg cells from the mice co-immunized with proVAX/G+His-G, but not in those receiving such cells from the proVAX/G+OVA immunized mice (FIGS. 20A-20F). Histological analysis revealed less inflammatory cell infiltrations in the groups received antigen specific CD4⁺CD25⁻ iTreg cells (FIGS. 20A-20F and FIG. 21). Although similar improvements of histopathological outcome were also observed in groups that received either CD4⁺CD25⁺ nTreg cells, the improvement from nTreg did not exhibit any antigen specificity.

CD4+CD25- cells from mice co-immunized with a DNA vaccine encoding G antigen and a G protein vaccine dramatically reduce the VID seen after live RSV challenge (FIGS. 20A-20F and FIG. 21). It is noted that CD4+CD25- cells transferred from mismatched vaccination regimens into His-G immunized mice resulted in a greater severity of clinical onset upon infection, suggesting that these cells from antigen-mismatched controls might suffer from a lack of iTreg cells and contain effector T cells (FIGS. 20A-20F and FIG. 21).

Co-immunization with an RSV G antigen-expressing DNA vaccine and its G protein together (proVAX/G+His-G) not only protected from RSV challenge in mice, but also suppressed the exacerbated pulmonary inflammation that leads to VID. The prevention of RSV infection and the reduced VID were apparently due to the induction of both high levels of antigen specific neutralizing antibody and of iTreg cells that could suppress T-cell recalled proliferation in an antigen-specific manner. Such co-immunization up-regulated the level of the anti-inflammatory cytokine IL-10, while down regulating the RSV-induced inflammatory cytokines, such as IL-4, IL-5, IL-13 and IFN-γ.

Although CD4+CD25+ cells (nTreg) from either co-immunized mice or mismatch controls were able to reduce inflammatory cell infiltrations in recipients after transfer (FIGS. 20A-20F and FIG. 21), they provided only a global nonspecific suppression. Presumably their suppression of inflammation is limited *in vivo* because nTreg is not an inducible population and has a plasticity dependent on immune macroenvironment. For example, it is difficult to induce substantial numbers of nTreg cells *in vivo* or *ex vivo.* Furthermore, a capacity for global immunosuppression might increase the risk of developing cancer and create an opportunity for infections

## Claims

1. A matched antigenic peptide and DNA vaccine against respiratory syncytial virus (RSV) infection comprising an RSV antigenic peptide and a nucleic acid encoding the RSV antigenic peptide, wherein the nucleic acid is DNA and the DNA encodes for the RSV antigenic peptide of SEQ ID NO:4, and wherein the vaccine stimulates iTreg cells which are CD4⁺, CD25⁻, FoxP3⁺, IL10⁺.

2. The vaccine of claim 1, wherein the DNA is present in a linear expression cassette of a circular plasmid.

3. The vaccine of claim 1, wherein the plasmid is selected from the group consisting of pVAX™, pcDNA3.0™, and proVAX™.

4. The vaccine of claim 1, wherein the plasmid further comprises a promoter selected from the group consisting of CMV, SV40 early promoter, SV40 late promoter, metallothionein promoter, murine mammary tumor virus promoter, Rous sarcoma virus promoter, and polyhedrin promoter.

5. The vaccine of claim 1, wherein the nucleic acid and antigenic peptide are at a mass ratio selected from the group consisting of 5:1 and 1:5; and 1:1 and 2:1.

6. The vaccine of claim 1, wherein the vaccine is capable of being electroporated into a subject in need thereof.

7. A vaccination kit comprising a vaccine administration device and the vaccine of claim 1, optionally wherein the vaccine administration device is selected from the group consisting of vaccine gun, needle, and an electroporation device.

8. The vaccine of claim 1 for use in preventing or treating respiratory syncytial virus infection in a patient.

9. The vaccine for use as claimed in claim 8, wherein the subject is further protected from airway hyper-responsiveness (AHR) after respiratory syncytial virus challenge.

10. The vaccine of claim 1 for use in inducing neutralizing antibody against respiratory syncytial virus infection and suppressing inflammatory T cells.

11. The vaccine for use as claimed in claim 10, wherein suppressing inflammatory T cell comprises inducing iTreg cells, and suppressing auto-reactive CD4+ and CD8+ T cells.

12. The vaccine of claim 1 for use in treating or preventing vaccine-induced disease in a subject immunized against respiratory syncytial virus (RSV).

13. The vaccine for use as claimed in claim 12, wherein the subject is immunized with formalin-inactivated RSV (FI-RSV) or RSV antigen prior to encounter with natural RSV infection.

14. The vaccine for use as claimed in claim 8 or 12, wherein the vaccine is for administration via electroporation.

15. The vaccine for use as claimed in claim 14, wherein the electroporation route is selected from the group consisting of intradermal and intramuscular.

16. The vaccine for use according to claim 14, wherein the vaccine is for electroporation with a minimally-invasive electroporation device or the vaccination kit of claim 5 wherein the vaccine administration device is a minimally-invasive electroporation device.

## Patentansprüche

1. Abgestimmter Antigenpeptid- und DNA-Impfstoff gegen eine Infektion mit dem respiratorischen Synzytial-Virus (RSV), umfassend ein RSV-Antigenpeptid und eine Nukleinsäure, die das RSV-Antigenpeptid codiert, wobei die Nukleinsäure DNA ist und die DNA das RSV-Antigenpeptid der SEQ ID NO: 4 codiert, und wobei der Impfstoff iTreg-Zellen, die CD4⁺, CD25⁻, FoxP3⁺, IL-10⁺ sind, stimuliert.

2. Impfstoff nach Anspruch 1, wobei die DNA in einer linearen Expressionskassette eines zirkulären Plasmids vorliegt.

3. Impfstoff nach Anspruch 1, wobei das Plasmid aus der Gruppe, bestehend aus pVAX™, pcDNA3.0™ und proVAX™, ausgewählt ist.

4. Impfstoff nach Anspruch 1, wobei das Plasmid weiterhin einen Promotor umfasst, ausgewählt aus der Gruppe, bestehend aus CMV-, SV40-Early-Promotor, SV40-Late-Promotor, Metallothionein-Promotor, Maus-Mammary-Tumor-Virus-Promotor, Rous-Sarkom-Virus-Promotor und Polyhedrin-Promotor.

5. Impfstoff nach Anspruch 1, wobei die Nukleinsäure und das Antigenpeptid in einem Massenverhältnis, ausgewählt aus der Gruppe, bestehend aus 5:1 und 1:5 und 1:1 und 2:1, vorliegen.

6. Impfstoff nach Anspruch 1, wobei der Impfstoff in ein Individuum, das dessen bedarf, elektroporiert werden kann.

7. Impfkit, umfassend eine Impfstoffverabreichungsvorrichtung und den Impfstoff nach Anspruch 1, wobei gegebenenfalls die Impfstoffverabreichungsvorrichtung aus der Gruppe, bestehend aus Impfpistole, -nadel und einer Elektroporationsvorrichtung, ausgewählt ist.

8. Impfstoff nach Anspruch 1 für die Verwendung zur Vorbeugung oder Behandlung einer Infektion mit dem respiratorischen Synzytial-Virus bei einem Patienten.

9. Impfstoff für die Verwendung nach Anspruch 8, wobei das Individuum weiterhin vor Atemwegshyperreagibilität (AHR) nach einer Provokation mit dem respiratorischen Synzytialvirus geschützt ist.

10. Impfstoff nach Anspruch 1 für die Verwendung zum Induzieren eines neutralisierenden Antikörpers gegen eine Infektion mit dem respiratorischen Synzytialvirus und zum Unterdrücken inflammatorischer T-Zellen.

11. Impfstoff für die Verwendung nach Anspruch 10, wobei das Unterdrücken inflammatorischer T-Zellen das Induzieren von iTreg-Zellen und das Unterdrücken autoreaktiver CD4⁺- und CD8⁺-T-Zellen umfasst.

12. Impfstoff nach Anspruch 1 für die Verwendung bei der Behandlung oder Vorbeugung einer Impfstoff-induzierten Erkrankung bei einem gegen das respiratorische Synzytialvirus (RSV) geimpftem Individuum.

13. Impfstoff für die Verwendung nach Anspruch 12, wobei das Individuum vor dem Ausgesetztsein gegenüber einer natürlichen RSV-Infektion mit Formalin-inaktiviertem RSV (FI-RSV) oder RSV-Antigen geimpft wird.

14. Impfstoff für die Verwendung nach Anspruch 8 oder 12, wobei der Impfstoff für die Verabreichung über Elektroporation bestimmt ist.

15. Impfstoff für die Verwendung nach Anspruch 14, wobei der Elektroporationsweg aus der Gruppe, bestehend aus intradermal und intramuskulär, ausgewählt ist.

16. Impfstoff für die Verwendung nach Anspruch 14, wobei der Impfstoff für die Elektroporation mit einer minimalinvasiven Elektroporationsvorrichtung bestimmt ist, oder Impfkit nach Anspruch 5, wobei die Impfstoffverabreichungsvorrichtung eine minimal-invasive Elektroporationsvorrichtung ist.

## Revendications

1. Vaccin à peptide antigénique et ADN appariés contre une infection par le virus respiratoire syncytial (VRS) comprenant un peptide antigénique de VRS et un acide nucléique codant pour le peptide antigénique de VRS, dans lequel l'acide nucléique est un ADN et l'ADN code pour le peptide antigénique de VRS de SEQ ID NO: 4, et le vaccin stimulant des cellules iTreg qui sont CD4⁺, CD25⁻, FoxP3⁺, IL10⁺.

2. Vaccin selon la revendication 1, dans lequel l'ADN est présent dans une cassette d'expression linéaire d'un plasmide circulaire.

3. Vaccin selon la revendication 1, dans lequel le plasmide est choisi dans le groupe constitué de pVAX™, pcDNA3.0™ et proVAX™.

4. Vaccin selon la revendication 1, dans lequel le plasmide comprend en outre un promoteur choisi dans le groupe constitué du promoteur de CMV, du promoteur précoce de SV40, du promoteur tardif de SV40, du promoteur de métallothionéine, du promoteur du virus de tumeur mammaire murine, du promoteur du virus du sarcome de Rous, et du promoteur de polyhédrine.

5. Vaccin selon la revendication 1, dans lequel l'acide nucléique et le peptide antigénique sont à un rapport en masse choisi dans le groupe constitué de 5:1 et 1:5 ; et 1:1 et 2:1.

6. Vaccin selon la revendication 1, le vaccin pouvant être soumis à électroporation chez un sujet en ayant besoin.

7. Trousse de vaccination comprenant un dispositif d'administration de vaccin et le vaccin selon la revendication 1, facultativement dans laquelle le dispositif d'administration de vaccin est choisi dans le groupe constitué d'un pistolet à vaccin, d'une aiguille, et d'un dispositif d'électroporation.

8. Vaccin selon la revendication 1 pour utilisation dans la prévention ou le traitement d'une infection par le virus respiratoire syncytial chez un patient.

9. Vaccin pour utilisation selon la revendication 8, le sujet étant en outre protégé contre une hyperréactivité bronchique (HRB) après exposition au virus respiratoire syncytial.

10. Vaccin selon la revendication 1 pour utilisation dans l'induction d'un anticorps neutralisant contre une infection par le virus respiratoire syncytial et la suppression des lymphocytes T inflammatoires.

11. Vaccin pour utilisation selon la revendication 10, la suppression des lymphocytes T inflammatoires comprenant l'induction de cellules iTreg, et la suppression de lymphocytes T CD4+ et CD8+ autoréactifs.

12. Vaccin selon la revendication 1 pour utilisation dans le traitement ou la prévention d'une maladie induite par un vaccin chez un sujet immunisé contre le virus respiratoire syncytial (VRS).

13. Vaccin pour utilisation selon la revendication 12, le sujet étant immunisé avec VRS inactivé par le formol (VRS-IF) ou un antigène de VRS avant d'être exposé à une infection par VRS naturel.

14. Vaccin pour utilisation selon la revendication 8 ou 12, le vaccin étant pour administration par électroporation.

15. Vaccin pour utilisation selon la revendication 14, la voie d'électroporation étant choisie dans le groupe constitué de la voie intradermique et intramusculaire.

16. Vaccin pour utilisation selon la revendication 14, le vaccin étant pour électroporation avec un dispositif d'électroporation mini-invasif ou trousse de vaccination selon la revendication 5, le dispositif d'administration de vaccin étant un dispositif d'électroporation mini-invasif.
